# EUROPEAN PATENT SPECIFICATION

(11) **EP 2 102 653 B1**
(45) Date of publication and mention of the grant of the patent: **13.08.2014**
(21) Application number: 08700136.8
(22) Date of filing: 07.01.2008
(51) Int. Cl.: G01N 33/50

(54) **METHOD AND KIT FOR TESTING WHETHER A SPECIFIC PLANT HAS BEEN EXPOSED TO A SPECIFIC PESTICIDE**
VERFAHREN UND KIT ZUR PRÜFUNG OB EINE BESTIMMTE PFLANZE ZU EINEM BESTIMMTEN PESTIZIDEN IS AUSGESETZT
MÉTHODE ET KIT POUR TESTER SI UNE CERTAINE PLANTE EST EXPOSÉE A UN CERTAIN PESTICIDE

(30) Priority: 07.01.2007 DK 200700022
(43) Date of publication of application: 23.09.2009
(73) Proprietor: Aarhus Universitet, 8000 Aarhus C (DK)
(72) Inventor: RAVN, Helle Weber, DK-8600 Silkeborg (DK)
(74) Representative: Høiberg A/S
(86) International application number: PCT/DK2008/050003
(87) International publication number: WO 2008/080409

(56) References cited:
- WO-A-01/92879
- WO-A-02/22859
- WO-A-03/027324
- WO-A-03/031937
- H.W. RAVN: "Two new methods for early detection of the effects of herbicides in plants using biomarkers.", JOURNAL OF PLANAR CHROMATOGRAPHY, vol. 22, no. 1, 1 January 2009 (2009-01-01), pages 65-71, Budapest
- H.W. RAVN: 'HWR-TEST S. Herbicide Weed Response test for Sulfonylurea-herbicides', [Online] 12 June 2008, AARHUS, Aarhus University Retrieved from the Internet: <URL:http://www.hptlc.com/pdf/2008helsinki/ 20080612/20080612_01.pdf> [retrieved on 2013-05-27]

## Description

### Field of invention

The present invention relates to a method and a kit for detection of pesticide effects in plants before any visual signs i.e. morphological signs are detectable on the plant. More specifically the invention relates to a simple and fast method to detect chemical compounds in living plants exposed to pesticides including herbicides. The kit and method correlate phytochemical compounds which have not been separated with exposure of a plant to pesticides or herbicides. The kit provides a simple and fast method to detect effects of pesticides and herbicides in the field.

### Background of invention

Plants and animals are exposed to stress continuously or temporarily throughout their life time. It is known that different types of stress, different exposure times and different amounts of a single stress type can influence differently depending on the species of plants or animals.

Phytochemical compounds have been used as biomarkers to obtain a biomarker pattern (WO 01/92879). A biomarker pattern in plants is defined as the changes in the composition and content of phytochemical compounds detected in plants after exposure to herbicides.

The detection and/or prediction of the final pesticide effects in plants is of special interest to the farming industry, mainly to reduce and control the use of chemicals such as pesticides. A reduction and/or control of the amount of pesticides used can be performed when the pesticide effects can be detected and/or predicted short time after exposure to the pesticide.

The present invention discloses a simple, fast and highly sensitive method of testing the final effects of chemicals in plants short time after exposure to chemicals such as pesticides. The method takes advantage of a change in the composition of chemical compounds and these compounds can be used as biomarkers in the material from a plant when exposed to stress. Particularly, the present invention relates to a method of testing the non-visual as well as the visual effects on plants exposed to chemical stress caused by pesticides.

### Summary of invention

The invention relates to a simple and fast method of testing the present composition of chemical compounds from a living organism. The chemical compounds are visualised e.g. as a colour reaction (or detection in UV-light) on a solid support e.g. on a stick or disk short time after exposure to chemical stress caused by pesticides. The purpose of the test is to estimate the final effect on the living organism of the stress as reduced growth or death of a living plant.

An aspect of the invention relates to a method to determine whether a weed plant has been exposed to chemical stress caused by pesticides, said method comprising
- obtaining plant material from a living weed plant, which may or may not have been exposed to an amount of chemical stress caused by pesticides or obtaining plant material from a non-living weed plant which when being alive may or may not have been exposed to an amount of chemical stress caused by pesticides,
- providing a liquid suspension of at least a part of said weed plant material, said liquid suspension of weed plant material comprising at least one chemical compound, wherein said chemical compound is a substance or at least part of a substance selected from the groups of amino acids, amines, sugars, flavanoids, phenolic
whether material from a specific plant has been exposed to a specific pesticide, the method comprising the steps of:
a) obtaining material from a specific plant,
b) preparing a liquid suspension of the specific plant of a) wherein the liquid suspension of the specific plant comprises at least one phytochemical compound,
c) detecting the phytochemical compounds in the liquid suspension of b) by their visual and/or UV-light colour, wherein the detection is performed without separation of said phytochemical compounds,
d) correlating said colour of the detected phytochemical compounds with a standard scale of unique visual and/or UV-light colours of unseparated phytochemical compounds for the specific plant and specific pesticide,
e) assessing whether the specific plant has been exposed to the specific pesticide.

Another aspect of the invention relates to a method of preparing a standard scale for assessing if a specific living plant has been exposed to a specific pesticide, the method comprising the steps of:
a) subjecting a specific living plant to a specific pesticide,
b) obtaining material from the exposed plant of a),
c) determining chemical responses of the specific plant by a visual and/or UV light colour detection without performing a separation of the phytochemical com pounds of the plant,
d) obtaining a standard scale of unique visual and/or UV-light colours of unseparated phytochemical compounds for the specific plant and specific pesticide combination.

Another aspect of the invention relates to an assay kit for use in the method according to any one of claims 1 to 4, said kit comprising:
- at least one filter paper,
- at least one solvent,
- at least one squeezing means,
- at least one standard visualising scale of unique visual and/or UV-light colours of unseparated phytochemical compounds for detection and/or prediction of exposure to a specific pesticide,
- at least one container.

### Description of Drawings

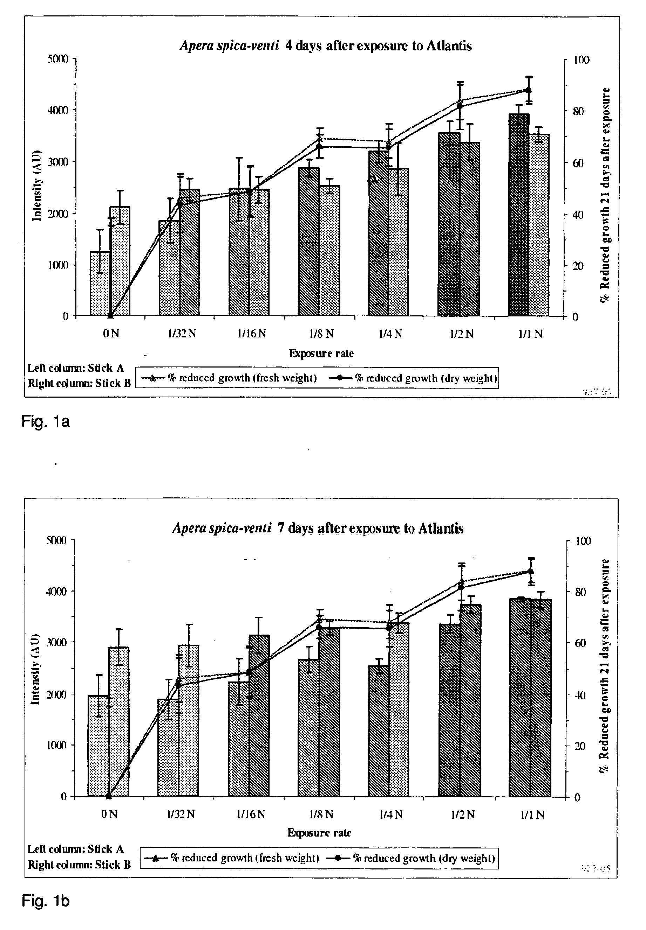

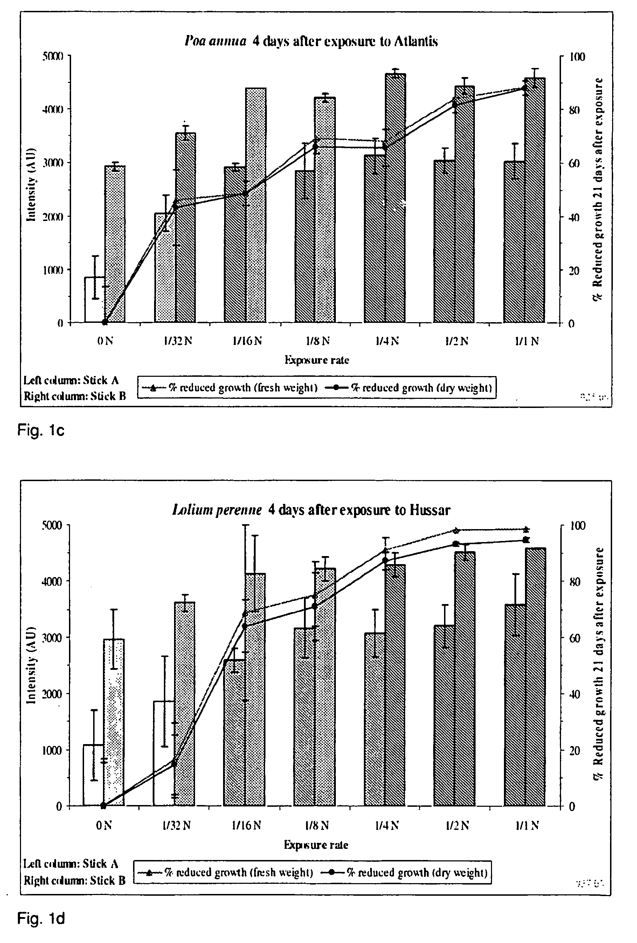

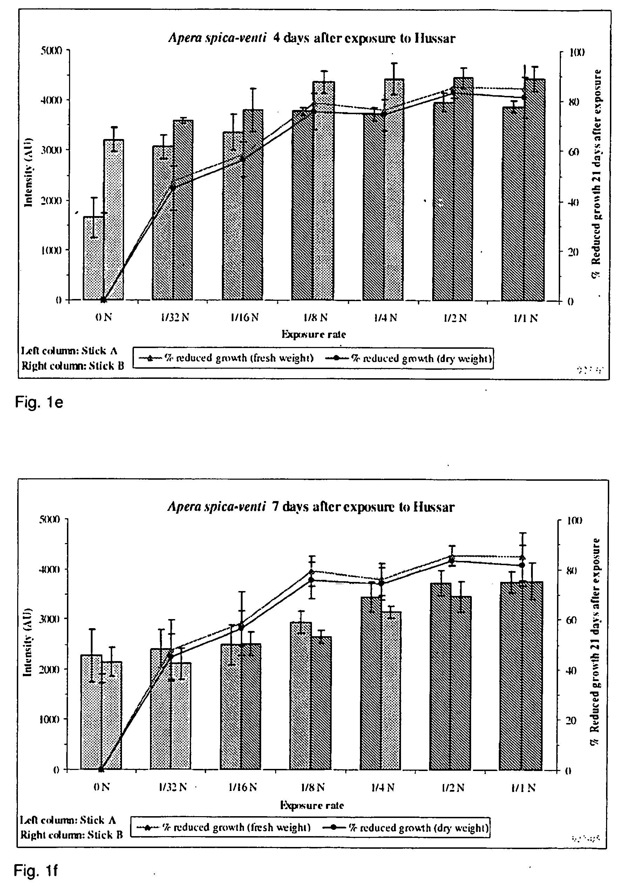

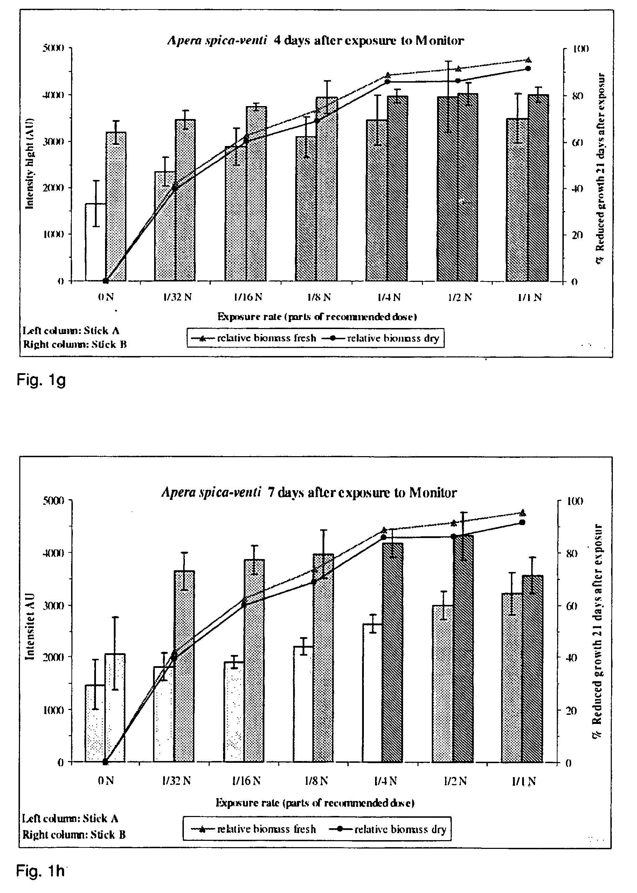

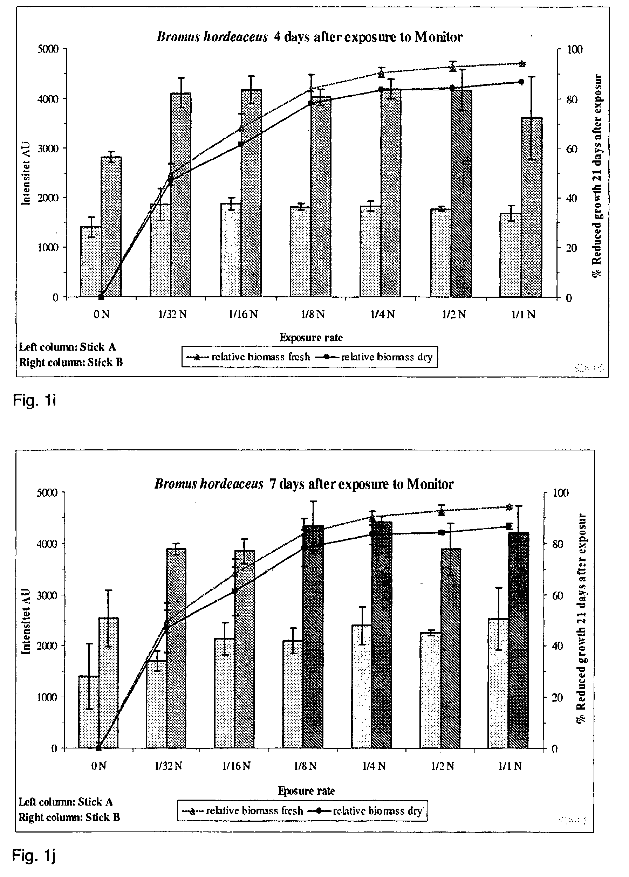

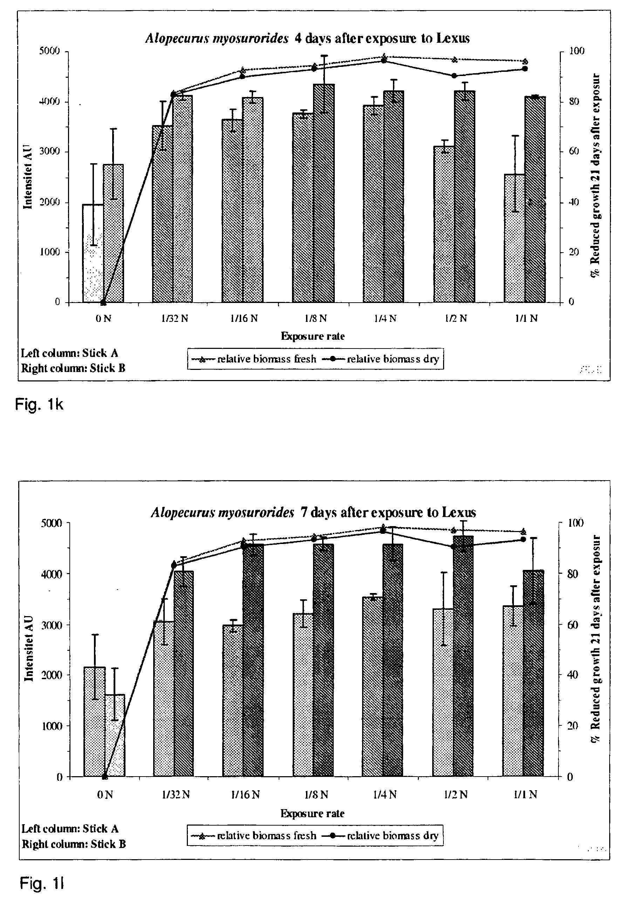

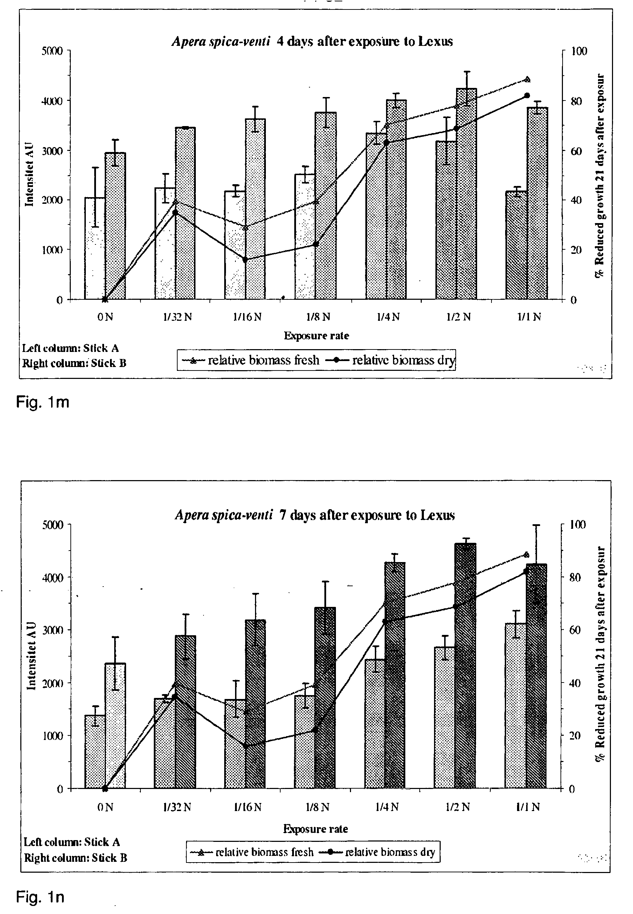

**Figure 1****.** Colour intensity of two different types of sticks (Stick A and B) of plant material in relation to the exposure rate to different herbicides and correlated with reduced growth of the plants (fresh and dry weight). The PANTONE®-colours (indicated in the columns), the intensity (AU) (indicated by the height of the columns) calculated on the CAMAG equipment and the reduced growth (fresh- and dry weight, (indicated by the graphs)) as 100%-the relative weight calculated 21 days after exposure: *Apera spica venti* 4 and 7 days after exposure to the herbicides Hussar (Fig 1e and Fig 1f), Atlantis (Fig 1 a and Fig 1b), Monitor (Fig 1g and Fig 1h) and Lexus (Fig 1m and Fig 1n); *Lolium perenne* 4 days after exposure to the herbicide Hussar (Fig 1 and Fig 1); *Poa annua* 4 days after exposure to the herbicide Atlantis (Fig 1c and Fig 1 d); *Alopecurus myosurorides* 4 and 7 days after exposure to the herbicide Lexus (Fig 1k and Fig 1I) and *Bromus hordeaceus* 4 and 7 days after exposure to the herbicide Monitor (Fig 1i and Fig 1j). The description of the colours of the sticks and thus of the columns of the figure can be seen from Table 4 and 5 where also the colour number according to the Pantone colour scale is indicated. The intensity of the colour indicates the level of the compounds tested by each test, and the darker the colour the more is the plant material affected by the herbicide treatment.

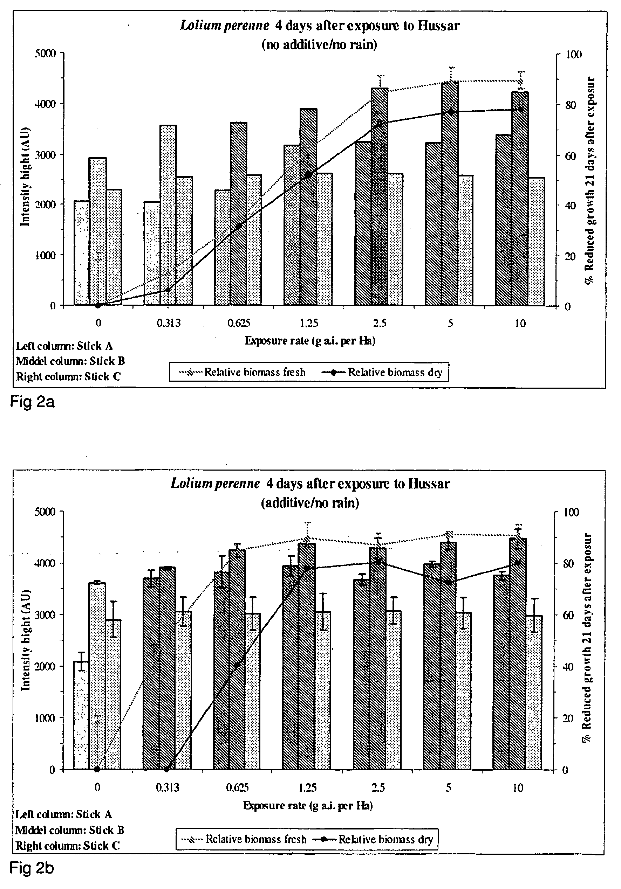

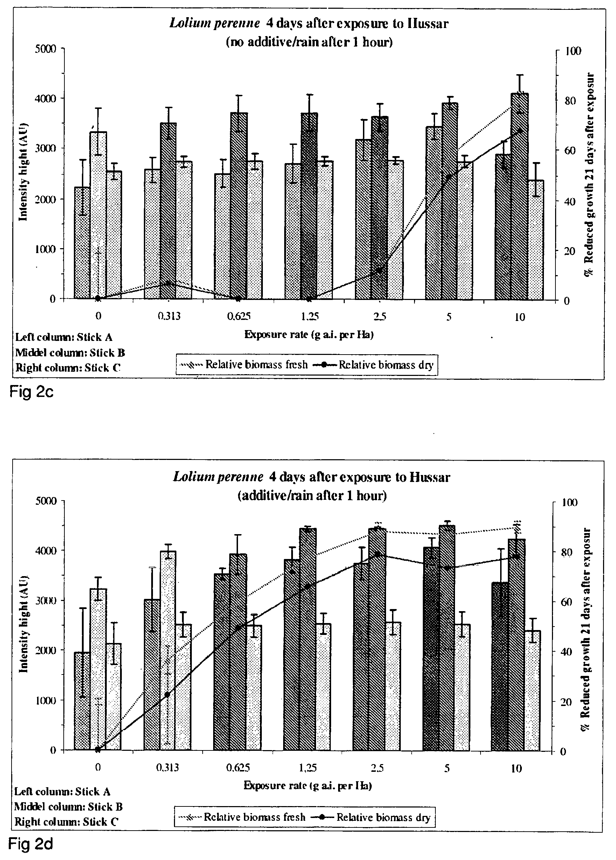

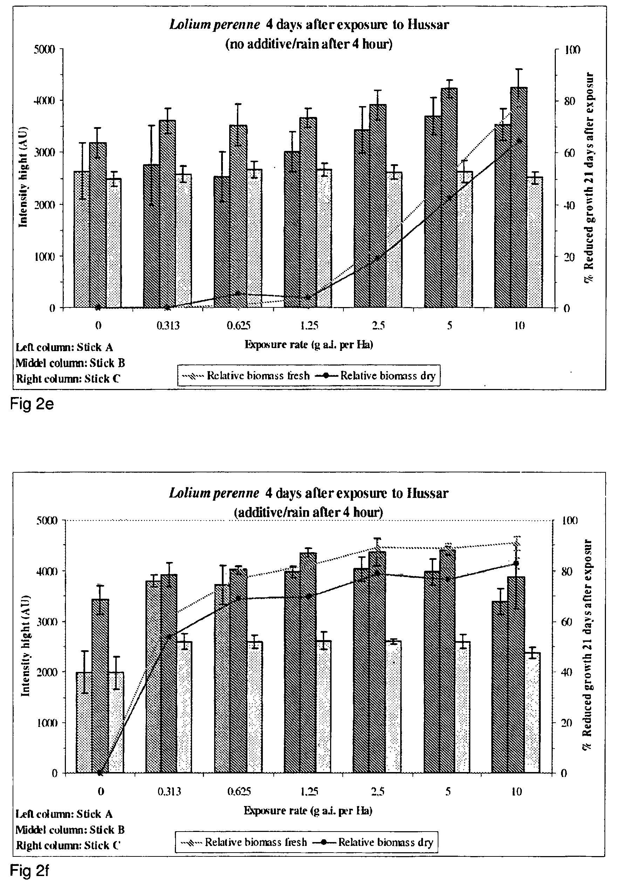

Figure 2. Results for study no. 972/04. Perennial ryegrass *(Lolium perenne)* exposed to Hussar (1 N= 200 g/ha) with rain treatment 0, 1 and 4 hours after application. ▲ = % effect (fresh weight), • = % effect (dry weight). Fig 2a: *Lolium perenne* 4 days after exposure to the herbicide Hussar with no additive and no rain; Fig 2b: *Lolium perenne* 4 days after exposure to the herbicide Hussar with additive and no rain; Fig 2c: *Lolium perenne* 4 days after exposure to the herbicide Hussar with no additive and rain after 1 hour; Fig 2d: *Lolium perenne* 4 days after exposure to the herbicide Hussar with additive and rain after 1 hour; Fig 2e: *Lolium perenne* 4 days after exposure to the herbicide Hussar with no additive and rain after 4 hours; Fig 2f: *Lolium perenne* 4 days after exposure to the herbicide Hussar with additive and rain after 4 hours. Further information is given in the Examples. For a description of the columns and graphs see the legend to Figure 1.

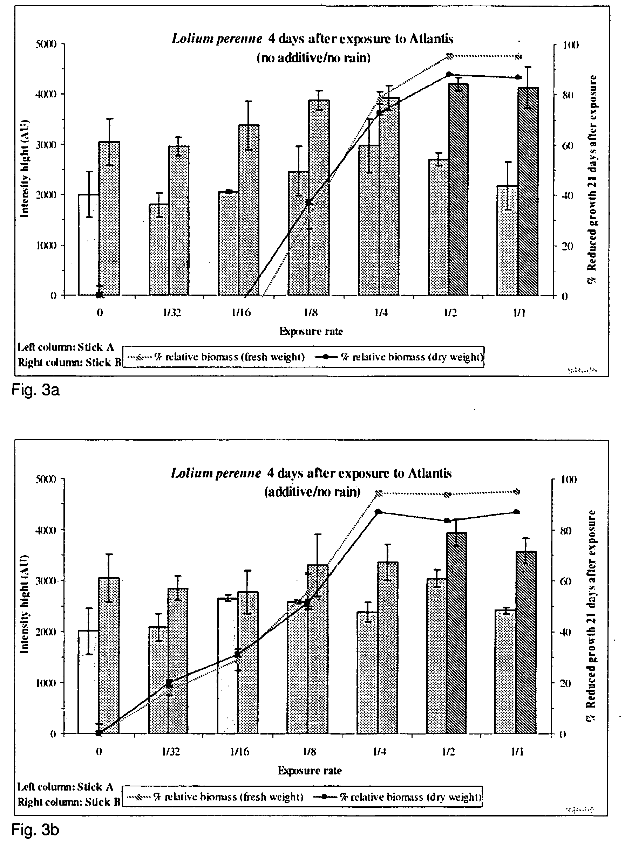

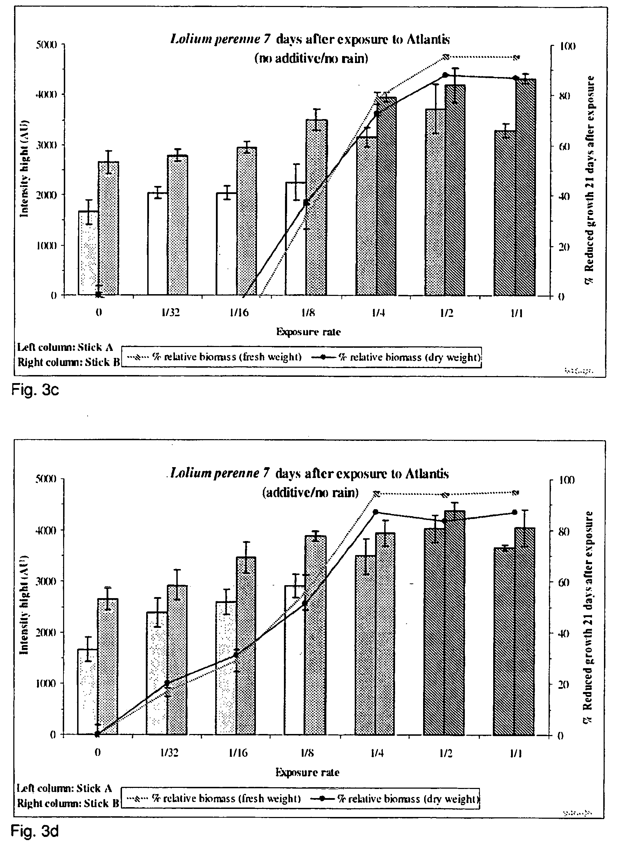

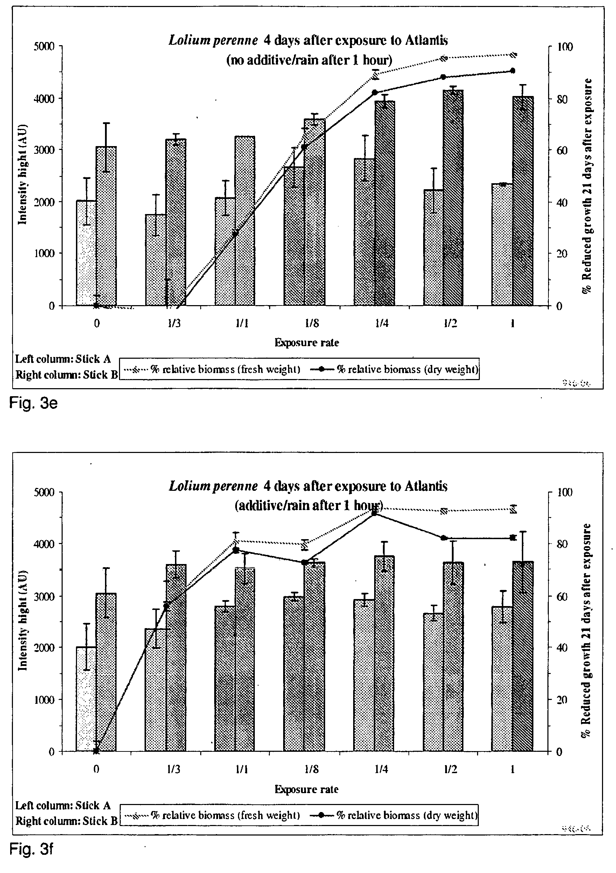

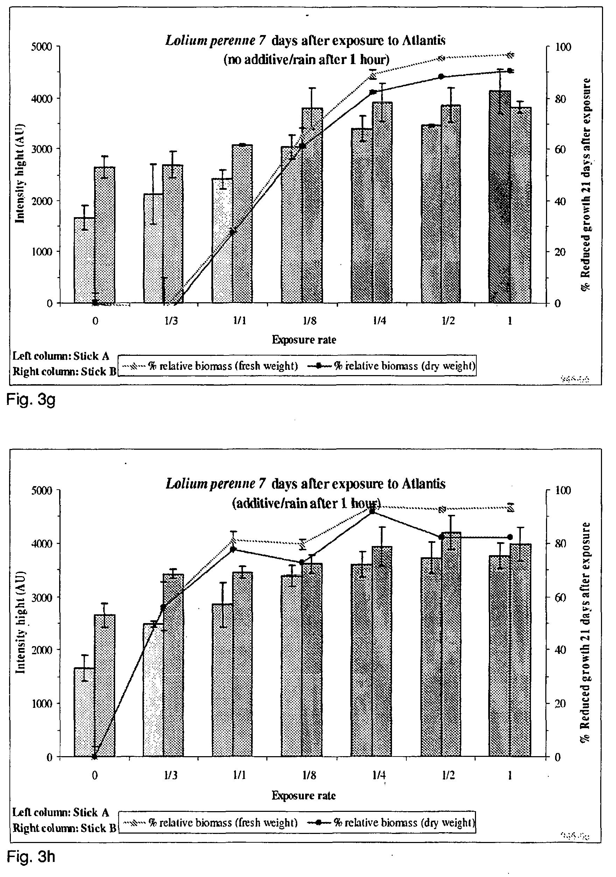

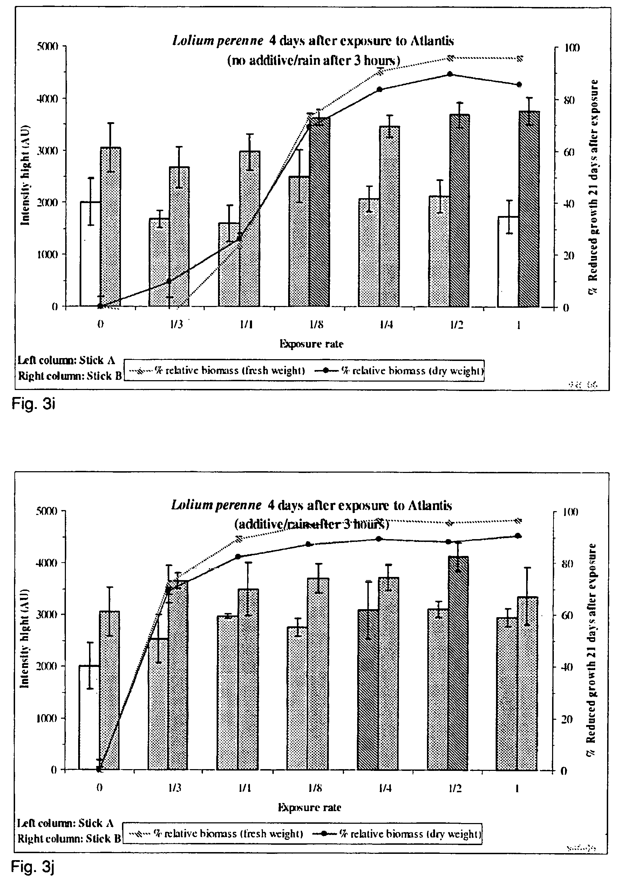

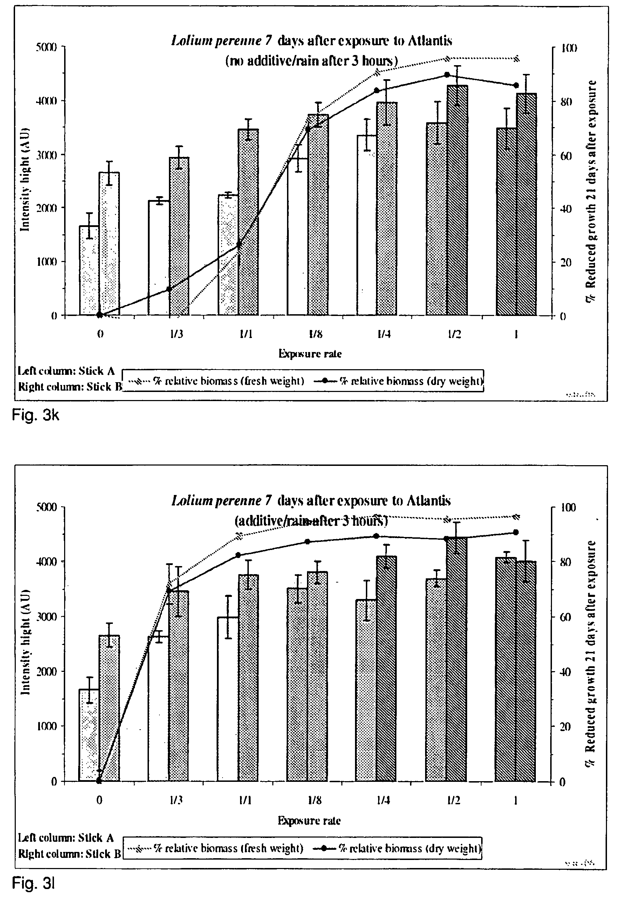

Figure 3: Results for study no. 946/06. Perennial ryegrass *(Lolium perenne)* exposed to Atlantis and with no rain or rain 1 or 3 hours after application. 1 N: 480 g/ha for Atlantis alone and without rain, 1920 g/ha with rain, 120 g/ha with Additive and without rain, 960 g/ha with Additive and with rain. ▲ = % effect (fresh weight), • = % effect (dry weight). Fig 3a: *Lolium perenne* 4 days after exposure to the herbicide Atlantis with no additive and no rain; Fig 3b: *Lolium perenne* 4 days after exposure to the herbicide Atlantis with additive and no rain; Fig 3c: *Lolium perenne* 7days after exposure to the herbicide Atlantis with no additive and no rain; Fig 3d: *Lolium perenne* 74 days after exposure to the herbicide Atlantis with additive and no rain; Fig 3e: *Lolium perenne* 4 days after exposure to the herbicide Atlantis with no additive and rain after 1 hour; Fig 3f: *Lolium perenne* 4 days after exposure to the herbicide Atlantis with additive and rain after 1 hour. Fig 3g: *Lolium perenne* 7days after exposure to the herbicide Atlantis with no additive and rain after 1 hour; Fig 3h: *Lolium perenne* 7 days after exposure to the herbicide Atlantis with additive and rain after 1 hour; Fig 3i: *Lolium perenne* 4 days after exposure to the herbicide Atlantis with no additive and rain after 3 hours; Fig 3j: *Lolium perenne* 4 days after exposure to the herbicide Atlantis with additive and rain after 3 hours; Fig 3k: *Lolium perenne* 7 days after exposure to the herbicide Atlantis with no additive and rain after 3 hours; Fig 3I: *Lolium perenne* 7 days after exposure to the herbicide Atlantis with additive and rain after 3 hours. Further information is given in the Examples. For a description of the columns and graphs see the legend to Figure 1.

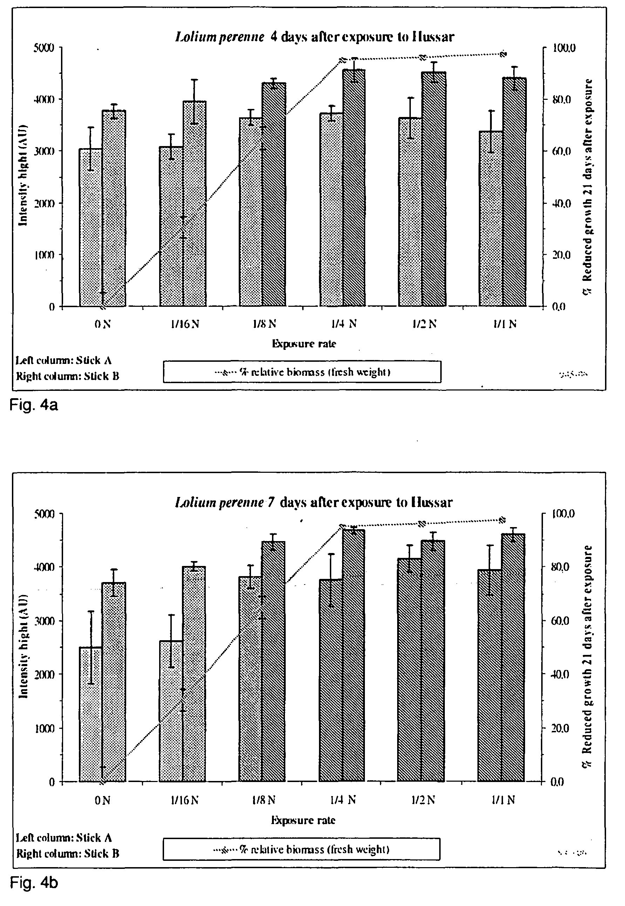

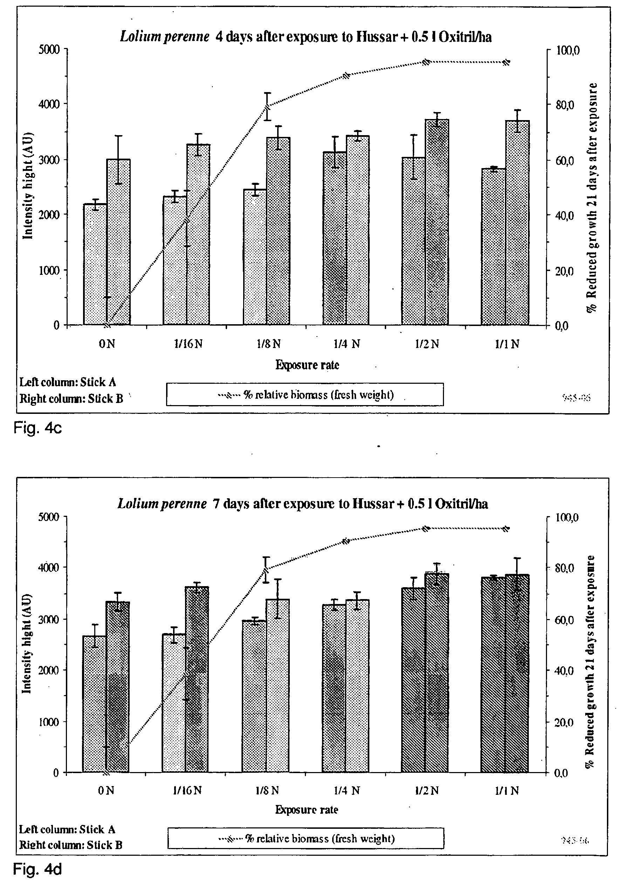

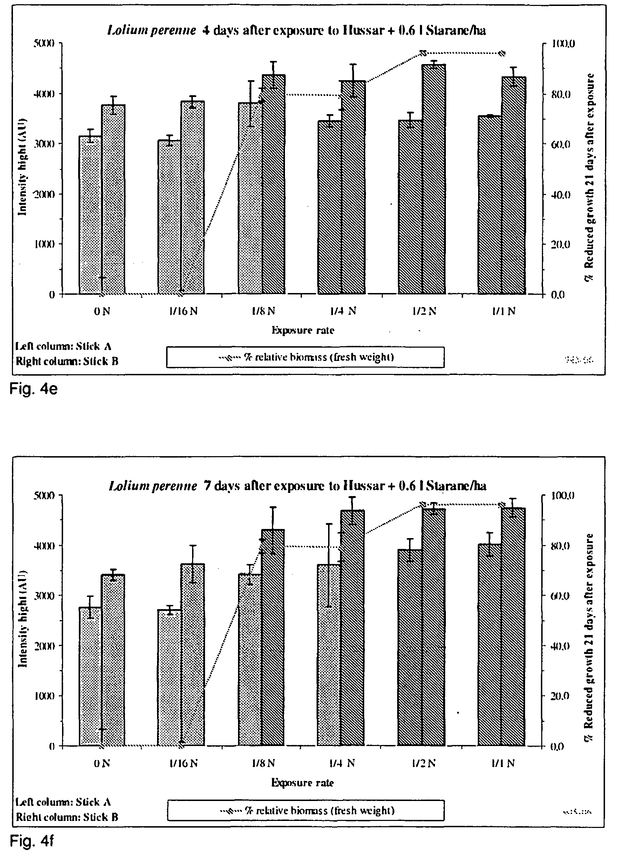

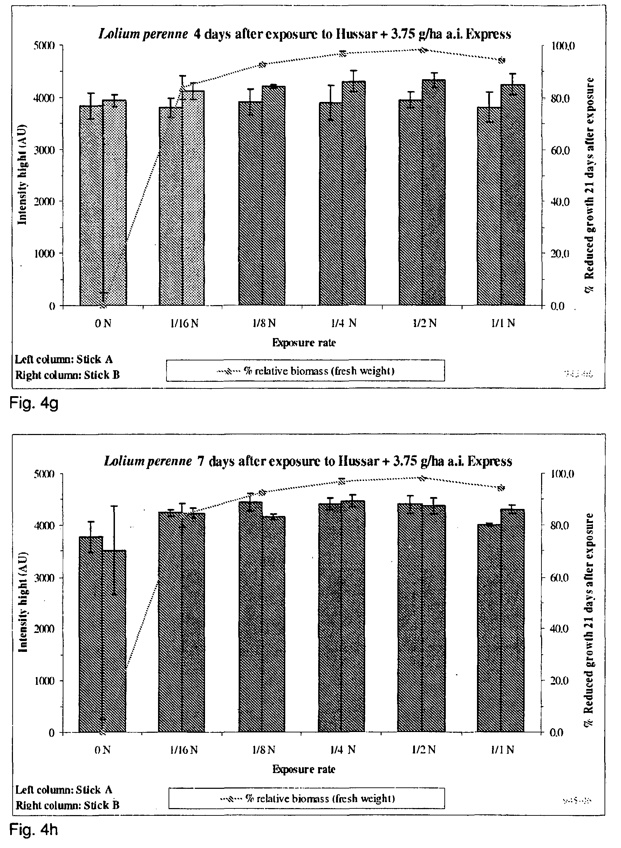

Figure 4: Results of study no. 945/06. Perennial ryegrass *(Lolium perenne)* exposed to Hussar OD alone and in mixture with 0.5 l/ha Oxitril, 0.6 l/ha Starane or 1 tablet/ha Express. ▲ = % effect (fresh weight), • = % effect (dry weight) 1 N = 30 ml/ha. Fig 4a: *Lolium perenne* 4 days after exposed to Hussar; Fig 4b: *Lolium perenne* 7 days after exposed to Hussar; Fig 4c: *Lolium perenne* 4 days after exposed to Hussar with 0.5 l/ha Oxitril; Fig 4d: *Lolium perenne* 7 days after exposed to Hussar with 0.5 l/ha Oxitril; Fig 4e: *Lolium perenne* 4 days after exposed to Hussar with 0.6 l/ha Starane; Fig 4f: *Lolium perenne* 7 days after exposed to Hussar with 0.6 l/ha Starane; Fig 4g: *Lolium perenne* 4 days after exposed to Hussar with 1 tablet/ha Express; Fig 4h: *Lolium perenne* 7 days after exposed to Hussar with 1 tablet/ha Express. For a description of the columns and graphs see the legend to Figure 1.

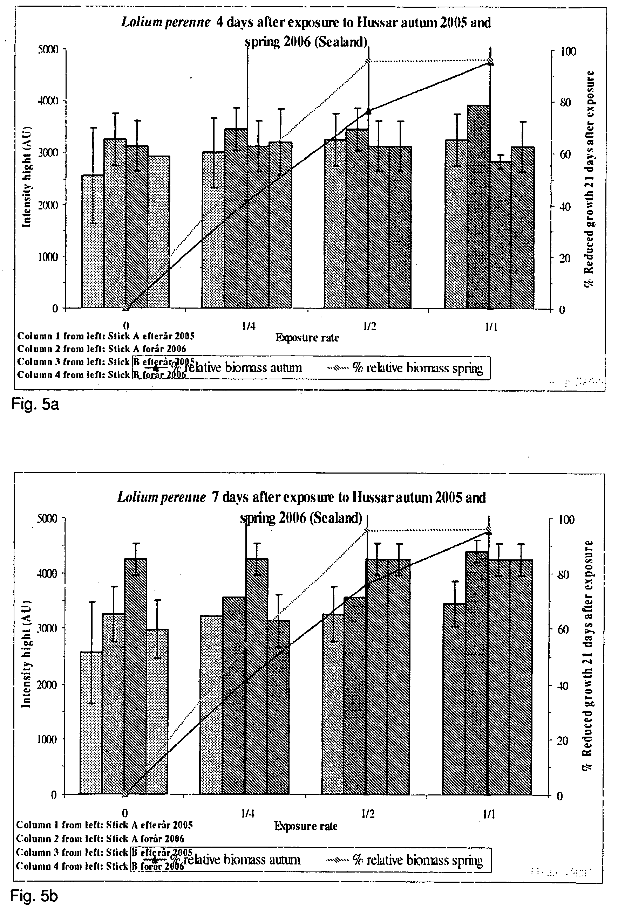

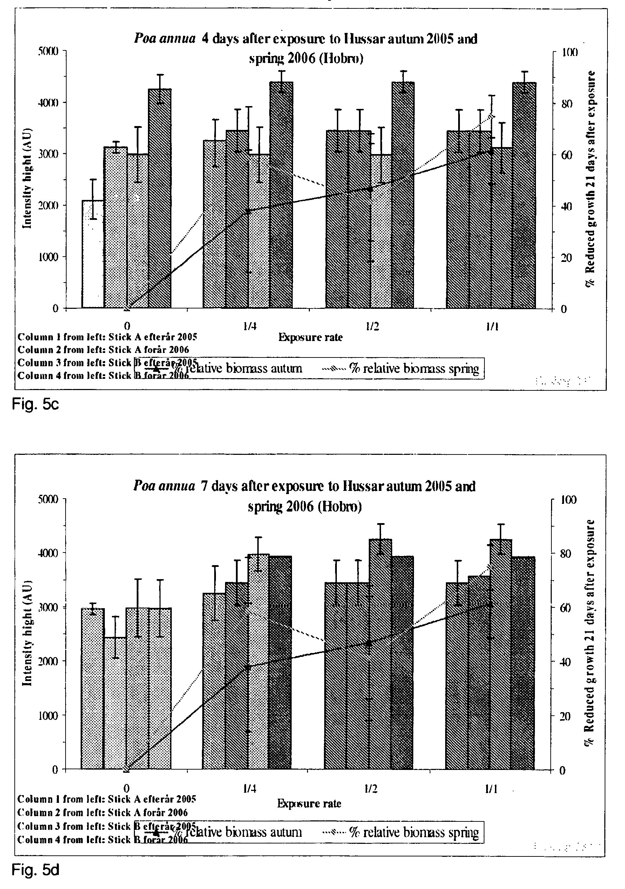

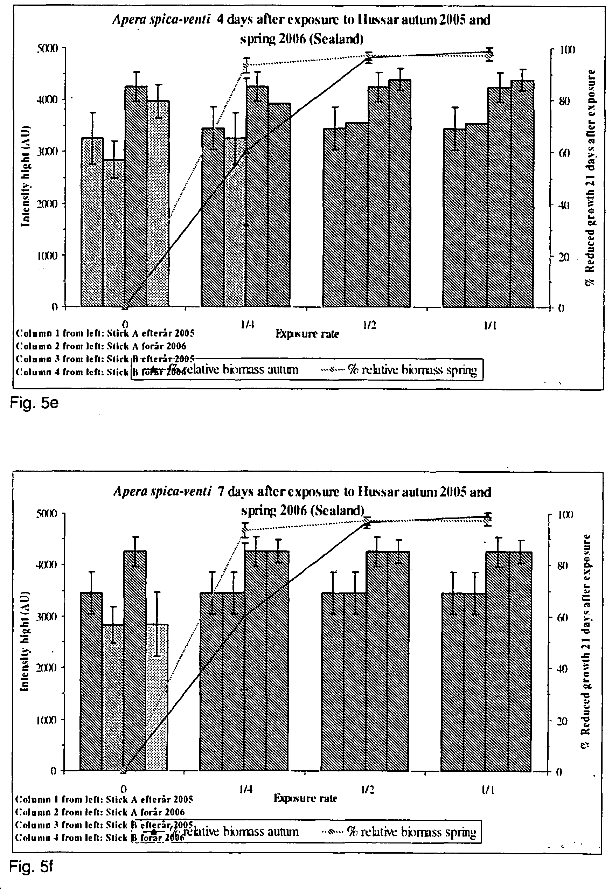

Figure 5: Results of field study. Perennial ryegrass *(Lolium perenne),* loose silky-bent grass *(Apera spica-venti)* and annual meadow grass (*Poa annua*) exposed to Hussar OD autumn ("efterår") 2005 and spring ("forår") 2006 (Hobro and Sealand). ▲ = % effect of autumn application(fresh weight), • = % effect of spring application (fresh weight). 1 N for perennial ryegrass was in the autumn 150 g/ha and 200 g/ha in the spring. The correponding doses for silky-bent grass were 100 g/ha in the autumn for silky-bent grass and 150 g/ha in the spring. 1 N for annual meadow grass was 150 g/ha in the autumn and 200 g/ha in the spring.. Fig 5a: *Lolium perenne 4* days after exposure to Hussar autumn 2005 and spring 2006; Fig 5b: *Lolium perenne* 7 days after exposure to Hussar autumn 2005 and spring 2006; Fig 5c: *Poa annua* 4 days after exposure to Hussar autumn 2005 and spring 2006; Fig 5d: *Poa annua* 7 days after exposure to Hussar autumn 2005 and spring 2006; Fig 5e: *Apera spica-venti 4* days after exposure to Hussar autumn 2005 and spring 2006; Fig 5f: *Apera spica-venti* 7 days after exposure to Hussar autumn 2005 and spring 2006. For a description of the columns and graphs see the legend to Figure 1.

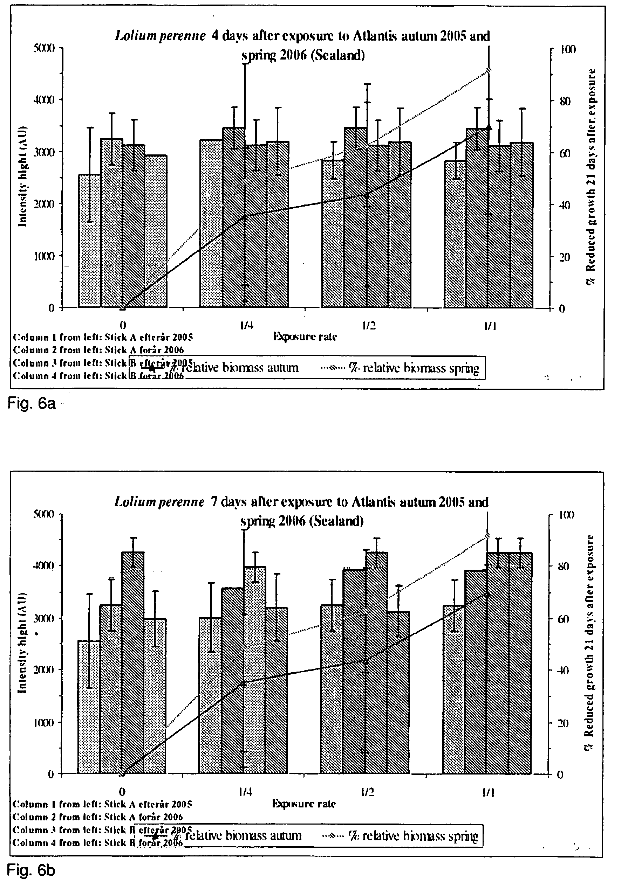

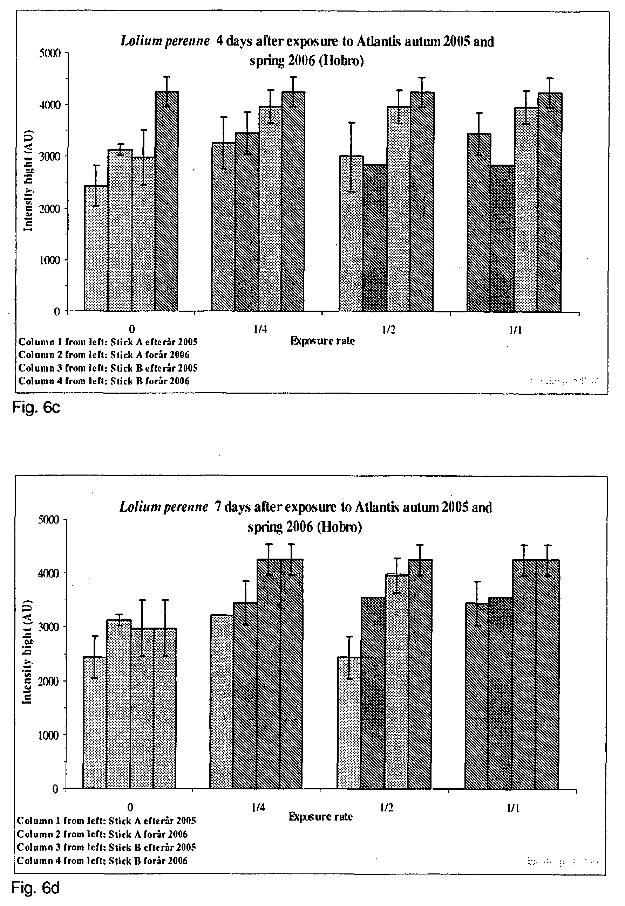

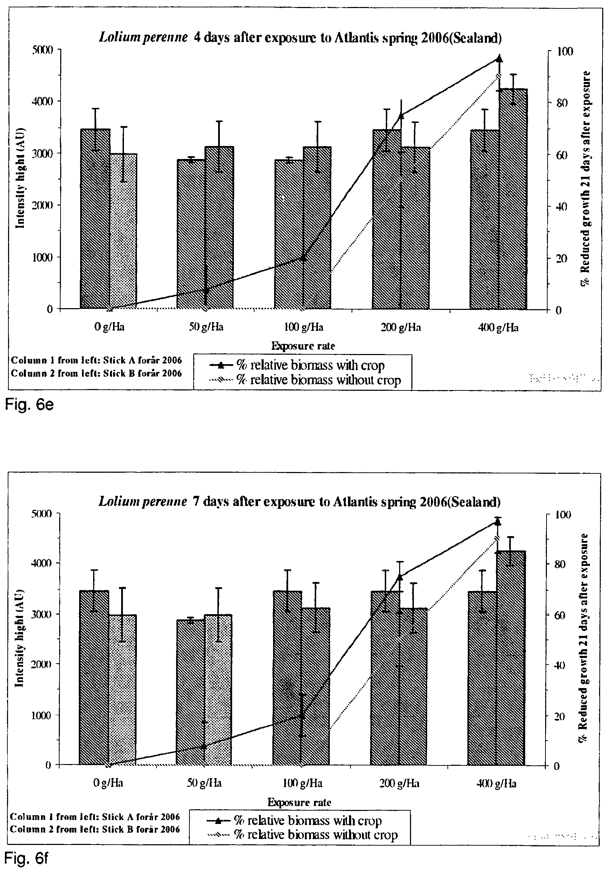

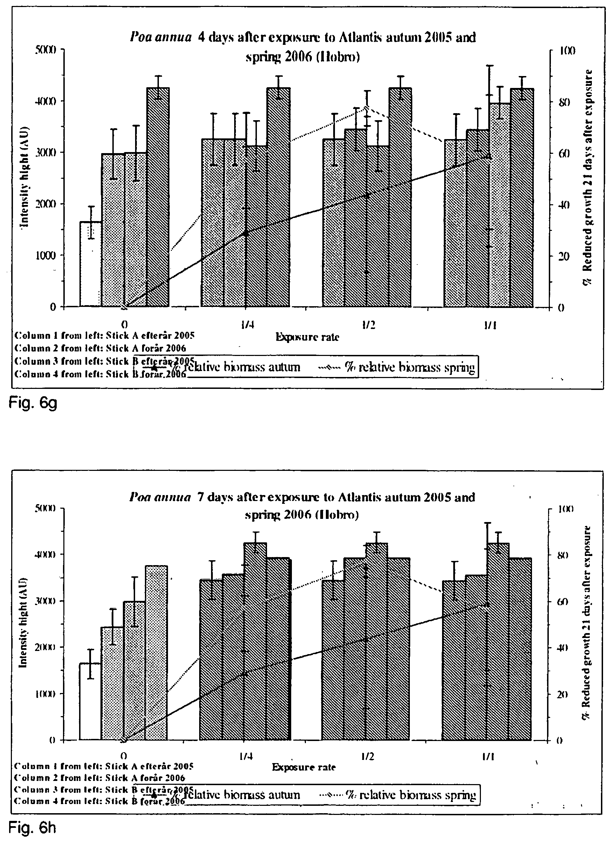

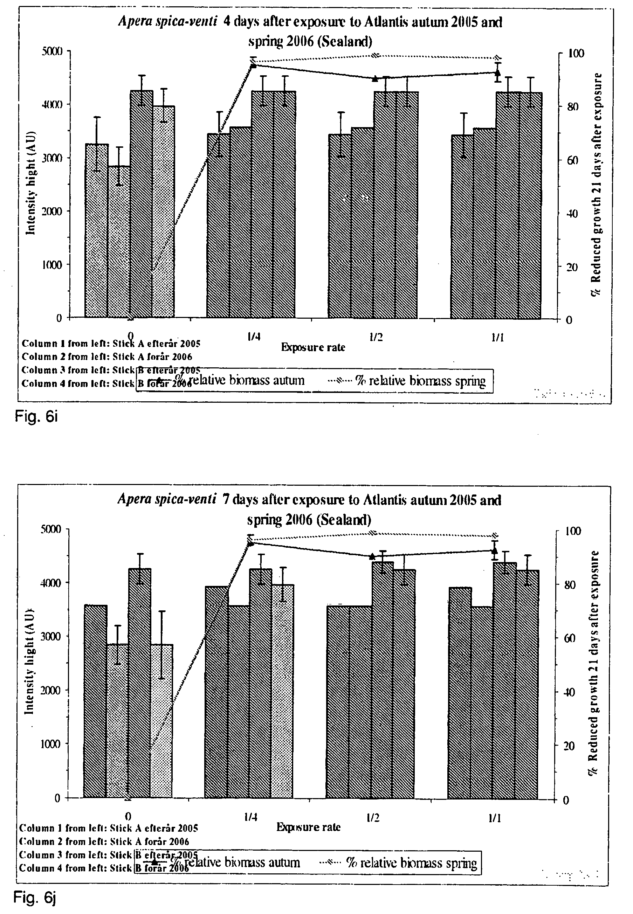

Figure 6: Results of field study. Perennial ryegrass *(Lolium perenne),* loose silky-bent grass *(Apera spica-venti)* and annual meadow grass *(Poa annua)* exposed for Atlantis autumn ("efterår") 2005 and spring ("forår") 2006 (Hobro and Sealand 917/06). ▲ = % effect of autumn application (fresh weight), ● = % effect of spring application (fresh weight). 1 N for perennial ryegrass autumn was 200 g/ha and 300 g/ha in the spring. The correponding doses for silky bent grass were 150 g/ha in the autumn and 150 g/ha in the spring. 1 N for annual meadow grass was 200 g/ha in the autumn and 300 g/ha in the spring. Fig 6a: *Lolium perenne* 4 days after exposure to Atlantis autumn 2005 and spring 2006; Fig 6b: *Lolium perenne* 7 days after exposure to Atlantis autumn 2005 and spring 2006; Fig 6c: *Lolium perenne* 4 days after exposure to Atlantis autumn 2005 and spring 2006; Fig 6d: *Lolium perenne* 7 days after exposure to Atlantis autumn 2005 and spring 2006; Fig 6e: *Lolium perenne* 4 days after exposure to Atlantis spring 2006; Fig 6f: *Lolium perenne* 7 days after exposure to Atlantis spring; Fig 6g: *Poa annua* 4 days after exposure to Atlantis autumn 2005 and spring 2006; Fig 6h: *Poa annua* 7 days after exposure to Atlantis autumn 2005 and spring 2006; Fig 6i: *Apera spica-venti 4* days after exposure to Atlantis autumn 2005 and spring 2006; Fig 6j: *Apera spica-venti* 7 days after exposure to Atlantis autumn 2005 and spring 2006. For a description of the columns and graphs see the legend to Figure 1.

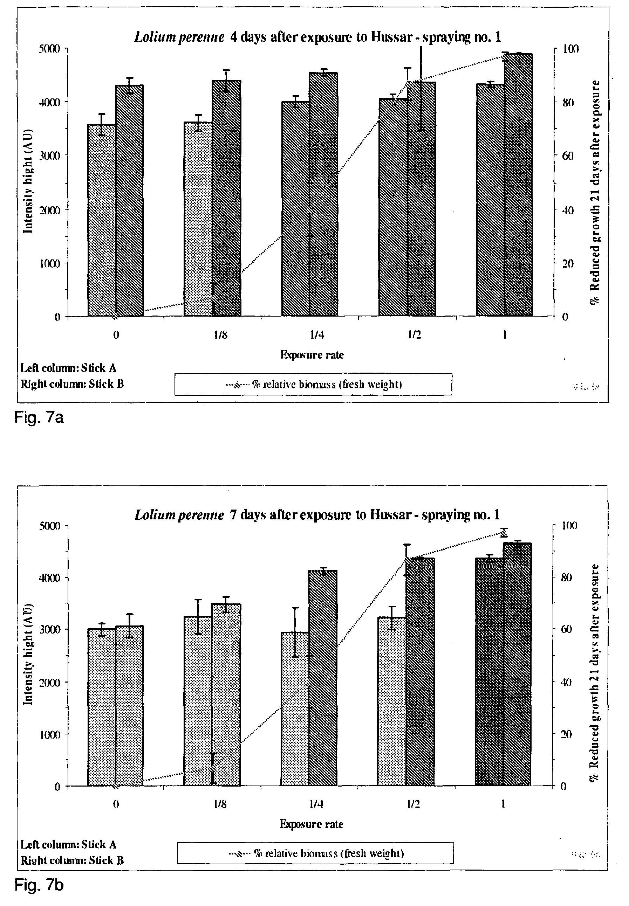

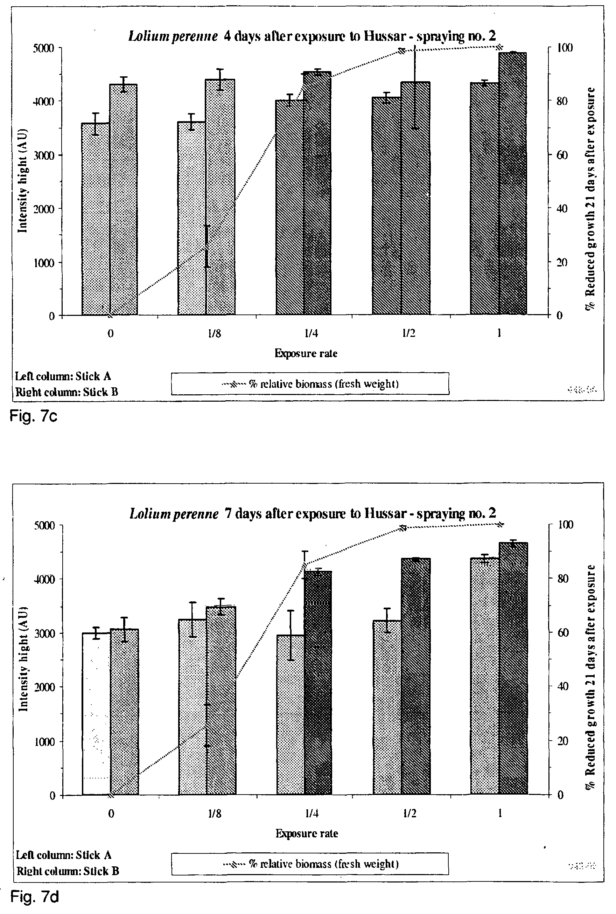

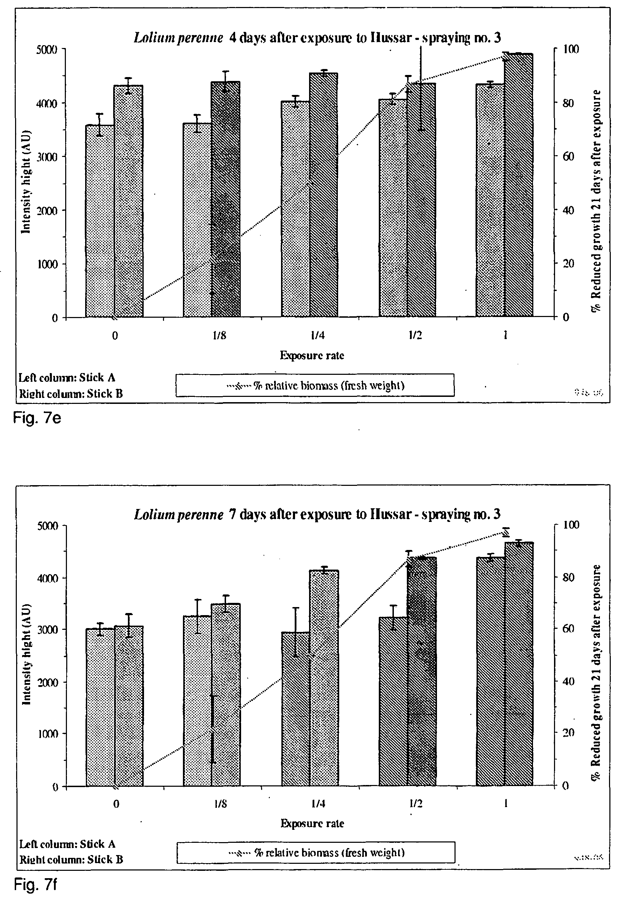

Figure 7: Results of field study no. (948/06). Perennial ryegrass *(Lolium perenne)* treated with Hussar OD (1 N= 75 ml/ha) at three different growth stages (1. application = stage 12; 2. application = stage 30.2; 3. application = stage 32), ▲ = % effect (fresh weight). Fig 7a: *Lolium perenne* 4 days after exposure to Hussar, treatment at plant growth stage 12; Fig 7b: *Lolium perenne* 7 days after exposure to Hussar, treatment at plant growth stage 12; Fig 7c: *Lolium perenne* 4 days after exposure to Hussar, treatment at plant growth stage 30.2; Fig 7d: *Lolium perenne* 7 days after exposure to Hussar, treatment at plant growth stage 30.2; Fig 7e: *Lolium perenne 4* days after exposure to Hussar, treatment at plant growth stage 32; Fig 7f: *Lolium perenne* 7 days after exposure to Hussar, treatment at plant growth stage 32; For a description of the columns and graphs see the legend to Figure 1.

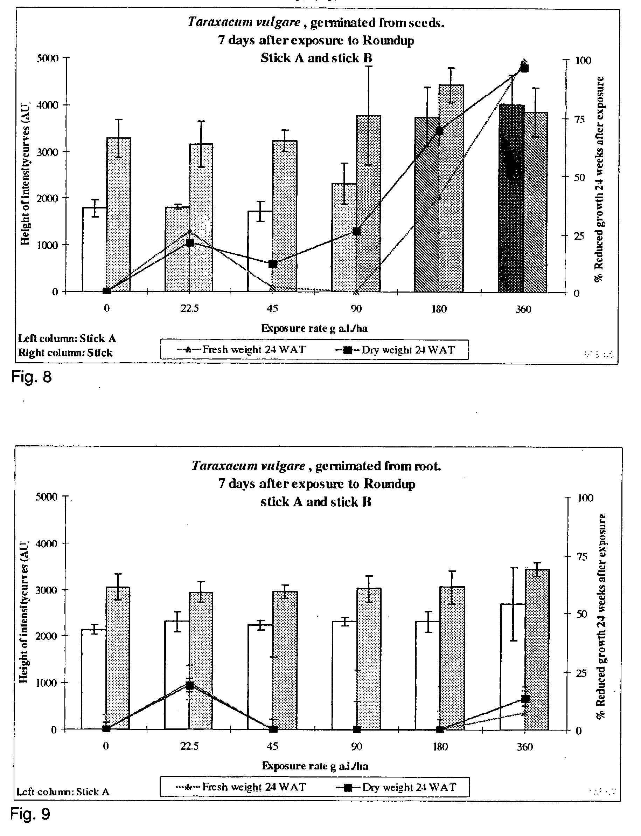

Figure 8: Results of semi-field study. Seed germinated dandelion, *Taraxacum vulgare* Weber exposed to the herbicide Roundup Bio. ■ = % biomass reduction (fresh weight) 24 weeks after exposure. ▲= % biomass reduction (dry weight). WAT = weeks after treatment.
Figure 9: Results of semi-field study. Root germinated dandelion, *Taraxacum vulgare* Weber exposed to the herbicide Roundup Bio. ■ = % biomass reduction (fresh weight) 24 weeks after exposure. ▲= % biomass reduction (dry weight). 1 N for 360 g/ha. WAT = weeks after treatment.

### Detailed description of the invention

By the present invention it has become possible to detect whether a living plant has been exposed to pesticides by applying a simple and highly sensitive method of testing. Plants may produce different amounts and/or different types of chemical compounds following exposure to pesticides when compared with pesticide-free living plants.

Especially it has become possible to detect and/or predict pesticide effects in plants before visual signs i.e. morphological signs are detectable on the plants. The invention relates to a simple and fast field method to detect chemical compounds in plants exposed to pesticides. The level and/or type of chemical/phytochemical compounds of the living organism can be correlated with final effects in the living organism e.g. reduced growth or death and can also be correlated to a corresponding dose of a stress factor such as the dose of a pesticide.

An aspect of the invention relates to a method for detecting whether material from a specific plant has been exposed to a specific pesticide, the method comprising the steps of:
a) obtaining material from a specific plant,
b) preparing a liquid suspension of the specific plant of a) wherein the liquid suspension of the specific plant comprises at least one phytochemical compound,
c) detecting the phytochemical compounds in the liquid suspension of b) by their visual and/or UV-light colour, wherein the detection is performed without separation of said phytochemical compounds,
d) correlating said colour of the detected phytochemical compounds with a standard scale of unique visual and/or UV-light colours of unseparated phytochemical compounds for the specific plant and specific pesticide,
e) assessing whether the specific plant has been exposed to the specific pesticide.

Another aspect of the invention relates to a method of preparing a standard scale for assessing if a specific living plant has been exposed to a specific pesticide, the method comprising the steps of:
a) subjecting a specific living plant to a specific pesticide,
b) obtaining material from the exposed plant of a),
c) determining chemical responses of the specific plant by a visual and/or UV light colour detection without performing a separation of the phytochemical com pounds of the plant,
d) obtaining a standard scale of unique visual and/or UV-light colours of unseparated phytochemical compounds for the specific plant and specific pesticide combination.

Another aspect of the invention relates to an assay kit for use in the method according to any one of claims 1 to 4, said kit comprising:
- at least one filter paper,
- at least one solvent,
- at least one squeezing means,
- at least one standard visualising scale of unique visual and/or UV-light colours of unseparated phytochemical compounds for detection and/or prediction of exposure to a specific pesticide,
- at least one container.

The present invention is based on providing a biomarker pattern or a visualising pattern e.g. as a colour pattern of a pooled or non-separated group of chemical or phytochemical compounds obtained in extracts from a plant. Optionally the non-separated group of chemical or phytochemical compounds can be reacted with a chemical reagent before obtaining the biomarker pattern or a visualising pattern. When an extract is obtained this may include chemical or phytochemical compounds which are assayed together in one step without separating the group of compounds into individual compounds. One or more groups of chemical or biochemical or phytochemical compounds obtained from a living organism such as in an extract and which are not separated into subgroups of compounds or into individual compounds may be denoted "pooled chemical compounds", "non-separated chemical compounds" or "gathered chemical compounds". A sample e.g. a plant extract of such non-separated chemical compounds can be denoted a non-separated sample. "Chemical" may be replaced by "phytochemical" when the chemical compounds are obtained from plants.

In a preferred embodiment the detection is being performed without performing a separation of the chemical compounds or of the reacted chemical compounds.

Although and in accordance with the invention as claimed, in an embodiment the invention could relate to a method for detection and/or prediction final effects of stress on a living organism by determining this effect directly based on the non-separated group of chemical, said method comprises
- obtaining material from a living organism, which may or may not have been exposed to an amount of a stress factor,
- providing an assayable form of at least a part of said living organism material, said assayable form of living organism material comprising at least one group of chemical compounds,
- detecting at least one group of chemical compounds optionally by a visual detection and/or UV-light detection,
- correlating said result to a standard result,
assessing final effect for said living plant material.

The compounds tested as at least one group of chemical compounds may be at least one of the group of chemical compounds being present naturally within an organism or being produced in an organism due to the effect of a stress treatment, said treatment being performed due to naturally occurring changes in the environment e.g. heat, rain, drought, influence by insects or other animals or due to changes performed by human e.g. watering, chemical treatment. The group of chemical compounds tested may be selected from the groups mentioned elsewhere herein.

In plants phytochemical compounds or biomarker pattern can be detected as a group of compounds showing a colour reaction (or detection in UV-light) on a stick/disk. The colour and intensity of the stick/disk indicate the final effect as a reduction on growth or death of the plants after exposure to the chemical stress such as pesticides e.g. herbicides.

The non-separated chemical compounds obtained from a living organism may depending on the actual amount and types of chemical compounds have some unique characteristics, which may be used to determine the level of stress imposed on the living organism. Any usable detection method may be used to distinguish between different samples of non-separated chemical compounds. The detection may be performed with chemical reacted non-separated chemical compounds or with non-separated chemical compounds which are not reacted with any other compounds to perform a chemical reaction.

To simplify the description of the present invention the following text will concerns material for which the living organism is exemplified by plants and the method of detection the non-separated phytochemical compounds is exemplified by a colour reaction which can be detected in visual light or in ultra-violet light (UV-light). The term "chemical compounds" is exemplified by biochemical compounds in living organisms and phytochemical compounds as compounds present in plants or plant cells. Further, the invention particularly discloses the use of phytochemical compounds in the control of herbicide spraying, although the effect of other stress factors can also be tested by the present invention.

When the non-separated phytochemical compounds are obtained from a plant, these may be reacted with a chemical reagent and a colour reaction may be detected either in visual light or in UV-light and semi-quantified using the intensity of the colour. In a preferred embodiment the colour is visualised on a solid support. In a more preferred embodiment the colour is visualised on a stick or a disk made of a material which may retain the phytochemical compounds optionally together with liquid comprising plant extract, solvents and/or a chemical reagent. Also non-separated compounds which are not reacted with a chemical reagent may be detected in a similar manner.

The colour and intensity of the reacted phytochemical compounds when applied on the solid support is different depending of the stress imposed upon the plant e.g. dependent on the dose of a pesticide or herbicide. The colour and colour intensity can be correlated with a standard colour scale to determine/predict the final effect as e.g. reduced growth of the plant or plant death. Extract obtained from a plant not exposed to a stress such as pesticide/herbicide has a different stick/disk colour and/or colour intensity when compared to extracts from pesticide/herbicide exposed plants. Extract from different stress free plant species may result in different colours and/or colour intensity on the stick/disk and hereby the method of the present invention can differentiate the different plant species before exposure to stress.

A standard scale of colours and/or colour intensity obtained on the solid support may be obtained when testing for and/or analysing groups of phytochemicals obtained from different plants of similar type e.g. of similar variety which are subjected to series of stress amount e.g. subjected to a range of stress from no spraying to recommended full dose of a herbicide or even above such recommended full dose. The colour scale obtained based on phytochemicals extracted from plants characterised by series of non-exposed to full exposed plants follow the relative biomass changes (growth reduction) of the plant. When the relative biomass production e.g. followed herbicide treatment is high and thus the reduction in growth is low, the colour of the stick/disk will be pale and less intensive when compared with plant material obtained from a plant where the relative biomasses is low and thus the reduction in growth is high.

For each plant species of e.g. a weed plant exposed to certain pesticide/herbicide doses a scale of colour and colour intensity obtained on a stick/disk can be correlated to a standard colour scale indicating the reduction in growth calculated in per cent of the relative biomass changes (growth reduction). Different colours and intensity of sticks can indicate different non-exposed plant species.

The present invention is based on the recognition that the phytochemical compounds in plants exposed to stress, such as pesticides, are related to and depending on the pesticides used and their modes of action in the plant. The inventor has found reproducible and unique colour reactions of non-separated composition of groups of phytochemical compounds in plants after exposure to a stress factor, such as a pesticide, said colour reactions being unique to the specific stress factor and level of applied stress, and unique to the individual plant family, more preferred the individual plant species, such as to individual plant varieties. The unique colour reaction may be regarded as a fingerprint of the effect of a specific pesticide in the plant in question, i.e. the specific plant to be tested. Thus, the present invention offers an opportunity to assess/determine whether a plant has been exposed to stress factors, such as pesticides in spite of the fact that the potential exposure cannot be assessed by visual inspection of said plant as visual signs.

Further the present invention offers an opportunity to predict or determine at an early stage of plant growth a final effect due to a herbicide treatment. Such a method allows a farmer to test whether an amount of a herbicide e.g. a reduced amount of herbicide in respect of a full recommended dose has the expected effect on a weed. Also the present invention gives a farmer the opportunity to predict or determine at an early stage of plant growth a final effect due to a herbicide treatment e.g. following rainy weather shortly after applying the herbicide to the plants. If the predicted final effect is lower than expected a re-spraying is possible at an early stage of plant growth, this being before a visual inspection of the effect of the spraying is possible.

By the term "visual inspection" is meant an ordinary visual inspection with the naked eye, whereby morphological changes, such as changes in colour, chloroses, necroses, withering etc. of the plant may be inspected.

Certain new chemical compounds may be produced in the plant after exposure to stress, or the concentration of already existing compounds may change, for example by an accumulation of certain chemical compounds in the plants. Furthermore, the colour obtained based on non-separated chemical compounds may also be related to a decrease or even an elimination of chemical compounds in the plants after exposure to stress. These changes of concentration of compounds, elimination of compounds and/or production of new compounds after stress exposure may be due to changes in the biochemical pathways of plants.

Accordingly, a colour obtained based on a single group of non-separated chemical compounds is a unique fingerprint of the composition of phytochemical compounds, i.e. endogenously produced compounds, in the plant after exposure to a stress factor, i.e. an external exposure, and said fingerprint is unique for each type of stress factors, such as pesticides, or for a group of stress factors.

In one aspect of the invention, the compounds present in the plants after exposure are the same as before exposure, but the concentration of the individual compounds is different, whereby a new fingerprint of the phytochemical compounds has arised after exposure.

In an aspect of the invention the presence of phytochemical changes and the extent of sensitivity of the plant to stress exposure may be dependent on the age of the plant. Young plants tend to be more sensitive to exposure of stress, such as herbicides, than older plants. This means that a fingerprint of the phytochemical compounds can be detected at an earlier stage after the time of exposure in a young plant as opposed to the later stage of detection of a fingerprint of the phytochemical compounds in an older plant. This knowledge of the correlation between plant age and the time neccessary for the plant to develop a fingerprint of the phytochemical compounds (i.e. sensitivity) may be used to determine how long ago a certain plant were exposed to stress factor(s). Due to their high sensitivity young plants show lower stability of the biochemical changes, i.e. the fingerprint of the phytochemical compounds is more stable in older plants and may be observed throughout the remains of the life of the older plant. However, younger plants have a higher sensitivity to stress and also a higher mortality rate. Fewer species of young plants will survive stress exposure the first weeks after emergence, while older plants are less affected.

In an embodiment the invention relates to a method for detection the time passed since a living organism has been exposed to a specific stress factor and/or for detection the amount of the specific stress factor, said method comprises
- obtaining material from a living organism, which may or may not have been exposed to an amount of a stress factor,
- providing an assayable form of at least a part of said living organism material, said assayable form of living organism material comprising at least one group of chemical compounds,
- optionally providing at least one chemical reagent applicable for a chemical reaction with the at least one group of chemical compounds.
- optionally provoking a chemical reaction between said at least one chemical reagent and said at least one group of chemical compounds of said living organism material,
- detecting said at least one group of chemical compounds optionally based on the result of said chemical reaction, wherein said detection optionally is by a visual detection and/or UV-light detection,
- correlating said result to a standard result,
- assessing the time passed since said living organism has been exposed to a specific stress factor and/or assessing the amount of the specific stress factor.

Accordingly, the present invention takes advantage of a number of parameters, such as the phytochemical responses and the time after stress exposure with which they occur, the physiological effects, the types, numbers and concentrations of compounds biosynthesised in plants after exposure to pesticides.

In one embodiment of the invention the fingerprint of the phytochemical compounds of the plant composition may relate to one group of phytochemicals, such as to at least 2 groups of phytochemicals. In another embodiment of the invention the fingerprint of the phytochemical compounds of the composition relates to at least 3 groups of phytochemicals, such as at least 4 groups of phytochemicals, for example at least 5 groups of phytochemicals, such as at least 6 groups of phytochemicals, for example at least 7 groups of phytochemicals, such as at least 8 groups of phytochemicals, for example at least 9 groups of phytochemicals, such as at least 10 groups of phytochemicals. The groups of phytochemicals may be selected among the phytochemicals mentioned elsewhere herein.

By the term "standard colour scale" is meant a colour scale of the composition of compounds present in a plant after exposure to known stress factors. A standard colour scale may be based on colours obtained when one or more groups of phytochemical compounds or other compounds of the organism are reacted with one or more chemical reagents. The colour scale may also be based directly on one or more groups of chemical compounds not reacted with a reagent. Visualisation of the colours is described elsewhere herein.

According to the invention the fingerprint relating to one or more groups of phytochemical compounds induced due to stress made by a known or unknown compound, i.e. the above described colour response is correlated to a standard colour scale. In order to interpret the fingerprint relating to phytochemical compounds of test material that has been exposed to known or unknown stress factors, it is a prerequisite to provide standard colour scales. The colour reaction or fingerprint relating to phytochemical compounds of test material that has been exposed to known or unknown stress factors may then be correlated to standard colour scales. The standard colour scales may be obtained for one particular stress factor or for a combination of at least two different stress factors, such as at least three different stress factors, e.g. at least four different stress factors, such as at least five different stress factors, e.g. at least six different stress factors, such as at least seven different stress factors, e.g. at least eitht different stress factors.

It is possible to prepare a standard colour scale for material from a living organism that has been exposed to stress comprising the steps of:
- subjecting a living organism to known types of stress, to known amount of a single or multiple stress type or to no stress,
- obtaining material from said living organism,
- determining the chemical responses of said material from said living organism for each stress type or for each combination of stress types or for each level of stress, and
- obtaining at least one standard colour scale relating to phytochemical compounds relating to said stress types or to said level of stress.

The description herein applies to both a method of providing a standard colour scale relating to phytochemical compounds of plants exposed to different stress types and/or different level of stress as well as to a method of testing whether material from a living organism has been exposed to stress, and further to a method of prediction of the final effect of living organism subjected to stress.

The material on which the testing is performed may be from any living material, such as from animals, for example mammals, soil invertebrates and insects, or from thallophytes, such as fungi or algae. However, in a preferred embodiment of the invention the material from a living organism is plant material.

In another preferred embodiment the material is selected from plants, fungi or algae. The following is a description of one embodiment of the invention, wherein the material from a living organism originates from plants. The description of this embodiment of the invention using plants, also relates to other embodiments of the invention, wherein the material from a living organism is not plant material.

Thus in one embodiment of the invention the method of testing is to determine the chemical fingerprint relating to phytochemical compounds after exposure to stress.

The plant material of the invention may be selected among any plant or plant cells. The plant material may be chosen from vascular plant, pteridophytes, seed plants, the gymnosperms, the angiosperms, mono- and dicotyledons. In one preferred embodiment of the invention the plant material is chosen from, but not limited to dicotyledons or monocotyledons. Also preferred is plant material chosen from plants considered to be a weed, especially weed in crop plants is of interest. Weed is considered as a plant competing with the crop plant such that the crop plant is negative influenced either in growth and/or composition.

According to the invention the dicotyledonous plants may be selected from the families of Asteráceae, Brassicaceae, Lamiaceae, Polygonaceae, Papaveraceae, Primuláceae, Plantagináceae and Scrophulariaceae and the monocotyledonous plants may be selected from the families of Poáceae.

The plants can also be selected from the families of Convolvolaceae, Umbelliferae, Oenotheraceae, Papilivanaceae, Violaceae, Malvaceae, Euphorbiaceae, Geraniaceae, Cruciferae, Fumariaceae, Urticaceae, Caryophyllaceae, Portulacaceae, Amarnthaceae, Cnenopodiaceae, Ranunculaceae, Boraginaceae, Labiatae, Solanaceae, Rubiaceae, Compositae, Graminea, Cyperaceae, Alismataceae, Lemnaceae, Potamogetonnaceae, Hydrocharitaceae, Juncaceae, Liliaceae, Convallariaceae, Iridacaea or all present families.

In a preferred embodiment the plant is selected from a plant of the genera *Apera, Alopecurus, Lolium, Bromus, Setaria, Echinochloa, Stellaria, Papaver, Polygonum, Galeopsis, Sinapis, Amaranthus, Brassica, Tripleurospermum, Matricaria* and *Poa.*

In a further preferred embodiment the plant is selected from the group of plant species *Apera spica-venti, Alopecurus myosuroides, Lolium perenne, Bromus hordaceus, Avena fatua, Stellaria media, Tripleurospermum inodorum, Chenopodium album, Amaranthus retroflexus, Galeopsis sp., Papaver rhoeas, Lolium sp., Setaria sp., Echinocloa crus-galli* and *Conyza canadensis* and *Poa annua.*

According to the invention the plant material used to perform the method of testing may be the entire plant or it may be at least a selected area of any part of the plant. The selected area of the plant may be an area such as from at least flowers, shoots, leaves, stems, roots, seeds, pollen, rhizomes, stamens, sepals, petals, carpels, styles, stigmas, microsporangia, anther, fruits, cotyledons, hypocotyle, epicotyle, xylem and/or phloem (wood), periderm (bark), buds, flower buds, cones, cone scales, tubers, bulbs, root nodules, resin or sap, or a combination thereof.

Preferred is plant material obtained from one or more flowers. Preferred is also plant material obtained from one or more shoots. Also preferred is plant material obtained from one or more leaves. Further preferred is plant material obtained from one or more stems. Yet further preferred is plant material obtained from one or more roots. Also preferred is plant material obtained from one or more seeds.

The test kit may be developed to test plants in growth stage BBCH 12 (2 leaves) to growth stage BBCH 23 (tillering). The stage/phase may also be 10, 13, 14, 15, 16, 17, 18, 19, 20, 21, 22, 24, 25, 26.

Once a sample of the plant material is obtained, a second step in the method according to the invention begins. It is an object of the present invention to provide a method of testing, wherein the plant material used is in a form suitable for assaying. One such form may be a liquid form, for example a liquid suspension. A liquid suspension of the plant material may be obtained by applying extraction solvents, such as water or ethanol to the plant material. The solvent may ensure that all compounds from one or more chemical groups present in the plant material is extracted. The assayable plant material may be fresh or non-fresh. Also a liquid suspension obtained directly from the organism or by squeezing the organism may be suitable for the test without any extraction by a solvent, e.g. plant sap. The liquid suspension may be filtered before utilised in the test, although non-filtered suspension may also be used.

In a preferred embodiment of the invention the plant material is fresh. The fresh material may be used for analysis immediately after harvest said material or it may be used for analysis up to a few minutes after harvesting. The fresh material can be analysed within at least 15 min, such as 30 min, e.g. 45 min, such as 1 hour, e.g. 2 hours, such as 3 hours. It is preferred that the fresh material is used as soon as possible after harvesting to avoid decomposition processes, such as enzymatic activity. Preferably fresh material is analysed within 1 hour.

In one embodiment the plant material is frozen. The frozen plant material may be frozen up to the point or time of analysing, such as frozen for a period of at least 1 week, such as at least 1 month, e.g. at least 1 year, such as at least 3 years, e.g. at least 5 years and it may be defrosted/thawed prior to performing the test. Thawed plant material may be analysed within at least 15 min, such as 30 min, e.g. 45 min, such as 1 hour, e.g. 2 hours, such as 3 hours. However, it is preferred that the frozen plant material is used for analysis immediately after being removed from the cold storage. Any freezing process can be used to freeze the plant material. Preferred is when the plant material is subjected to the freezing process immediately after harvest, e.g. within 5 min, such as within 15 min, e.g. within 30 min, such as within 45 min, e.g. within 60 min, such as within 75 min, e.g. within 90 min, such as within 105 min, e.g. within 120 min.

In another embodiment of the invention the plant material is dry. The drying process may be accounted for by air, or nitrogen, or it may be a freeze drying process, such as nitrogen dried. Additionally the plant material may be heat dried, such as sun dried. The plant material may be substantially dry, and the length of the drying process is dependent on the type of plant material. Air drying may be at about 20°C and e.g. without heat and light. Heat and light might destroy the compounds of the material.

The length of the time period before the plants react to the pesticide exposure and sensitivity of the plant species to the pesticide may be dependent on different factors, such as the species and age of the plant. The various plant species have different sensitivity to pesticide types. For example the plant species *Lolium perenne* is more sentitive to the sulfonylurea herbicide, iodosulfuron than *Apera spica-venti* or *Poa annua.* Therefore a lower dose of the herbicide exposed to *Lolium perenne* than to *Apera spica-venti* or *Poa annua,* may be detected as a phytochemical response corresponding to a higher reduced biomass to *Lolium perenne* than to *Apera spica-venti* or *Poa annua.* With respect to age, the seedlings may be more sensitive than older plants to the herbicides and therefore seedlings are more sensitive to the herbicide.

In an embodiment the method and kit is developed in respect of a living organism mentioned elsewhere herein at at least one development stage selected from the growth scales 0, 1, 2, 3,4, 5, 6, 7, 8 or 9 representing stages of germination, sprouting, bud development, leaf development, formation of side shoots/tillering, stem elongation or rosette growth, shoot development (main shoot), development of harvestable vegetative plant parts or vegetatively propagated organs/booting (main shoot), inflorescence emergence (main shoot)/heading, flowering (main shoot), development of fruit, ripening or maturity of fruit and seed, senescence, beginning of dormancy. Growth scales are further described in "Growth stages of mono- and dictolydonous plants", BBCH Monograph, 2. edition 2001. Edited by Uwe Meier, Federal Biological Research Centre for Agriculture and Forestry.

In an embodiment the method and kit is developed to be useable on a plant species or variety within a time period lasting for at least more than the time the organism has to growth within one growth scale. Hereby the method and kit can be developed to be used in e.g. growth scale 0 and 1 (partly or fully), or growth scale 1 and 2 (partly or fully); growth scale 2 and 3 (partly or fully); growth scale 3 and 4 (partly or fully). Also more the two growth scales may be covered by the method and kit, e.g. growth scale 0 to 2 (partly or fully), growth scale 1 to 3 (partly or fully), growth scale 2 to 4 (partly or fully), growth scale 0 to 3 (partly or fully), growth scale 1 to 4 (partly or fully), growth scale 0 to 4 (partly or fully).

The present invention further relates to a method of testing having an improved sensitivity, i.e. detection limit when compared to other tests. By detection limit is meant the lowest possible doses of pesticides the test kit of the invention is capable of determining. It is possible to detect a phytochemical effect below the recommended level of dosage of e.g. pesticides. The "recommended dose" is the effective dose needed to obtain a given result as e.g. final effect as plant death or at least > 80% growth reduction. According to the invention the method of testing may be performed on plants being exposed to doses from 0 to recommended dose or even. A dose down to 1/32 dose of recommended dose without any visual signs on the plant may be detected as a phytochemical effect on the stick/disk, depending on the plant species or by the colour of the liquid suspension or the extract. Some plants may recover from an exposure, and the detection may take place before such recovery. A phytochemical effect will then be detected and no changes in the final effect compared with unexposed plants. In such cases the detection limit of the test-kit related to the exposure dose of the pesticide is at the point where the phytochemical effect corresponds to a change in the final biomass.

A standard colour scale may be obtained by applying plants of similar species and/or variety to a series of stress, e.g. to a series of herbicide including stress free plants. The series of stress may be level of stress e.g. inducing 0, 25%, 50%, 75% and 100% plant death, or may be 0, 25%, 50%, 75% and 100% herbicide dose, where 100% is the recommended dose, e.g. the dose recommended by the manufacturer of the herbicide. A standard colour scale may be determined by applying series of stress of levels selected from 0, 1, 5, 10, 20, 25, 30, 40, 50, 60, 70, 75, 80, 90, 95 and 100% of effect or dose as described above, also doses above 100% i.e. above recommended level may be included when producing a standard colour scale.

As a non-limiting example, the process of preparing a standard colour scale for plant weed that has been exposed to stress in the form of a herbicide can comprise the steps of:
- subjecting different groups of weed plants to no herbicide or each other group to a different dose of the herbicide in the range from very low doses in respect of recommend dose to doses corresponding to or exceeding recommended application dose of the herbicide,
- obtaining material from said living weed plants e.g. at different time after exposure of the plants to the herbicide e.g. 2, 3, 4, 5, 6, 7, 8 days after the exposure,
- determining the chemical responses of the material from said living weed plants for each herbicide treatment by determining a colour based on the chemical changes performed directly and/or indirectly by the herbicide e.g. to phyto-chemical compounds,
- determining the final effect of each herbicide treatment on weed plants from where no plant material has been removed,
- correlating the colour indicating the chemical responses of the herbicide treated plant material with the final effect of the herbicide treatment, and
- obtaining at least one standard colour scale relating to phytochemical compounds relating to the herbicide and to the level of herbicide applied on the plants.

Although not in accordance with the present invention as claimed, the standard colour scale may be determined at least 1 day after the plants are subjected to the stress, such as at least 2 days, e.g. at least 3 days, such as at least 4 days, e.g. at least 5 days, such as at least 6 days, e.g. at least 7 days, such as at least 8 days, e.g. at least 9 days, such as at least 10 days, e.g. at least 11 days, such as at least 12 days, e.g. at least 13 days, such as at least 14 days, e.g. at least 15 days, such as at least 16 days, e.g. at least 17 days, such as at least 18 days, e.g. at least 19 days, such as at least 20 days, e.g. at least 21 days, such as at least 22 days, e.g. at least 23 days, such as at least 24 days, e.g. at least 25 days, such as at least 26 days, e.g. at least 27 days, such as at least 28 days, e.g. at least 29 days, such as at least 30 days, e.g. at least 31 days.

The length of the time period before the plants react to the stress exposure may be dependent on numerous factors, such as the species and age of the plant. Some plants may recover from an exposure, and the detection may take place before such a recovery. However, it may be possible to detect biomarkers after the plant has recovered from the exposure. Without being bound by theory the detection of biomarkers may for some plants be possible throughout the entire life span of the plants, whereas the detection of biomarkers of other plants may only be possible within a certain time frame. This of course may depend on the nature of the plant species and of the stress factors as such, for example the concentration level of pesticides.

The length of the time period before the plants react to the pesticide exposure and sensitivity of the plant species to the pesticide may be dependent on different factors, such as the species and age of the plant. The various plant species have different sensitivity to pesticide types. For example the plant species *Lolium perenne* is more sentitive to the sulfonylurea herbicide, iodosulfuron than *Apera spica-venti* or *Poa annua.* Therefore a lower dose of the herbicide exposed to *Lolium perenne* than to *Apera spica-venti* or *Poa annua,* may be detected as a phytochemical response corresponding to a higher reduced biomass to *Lolium perenne* than to *Apera spica-venti* or *Poa annua.* With respect to age, the seedlings may be more sensitive than older plants to the herbicides and therefore seedlings are more sensitive to the herbicide.

Although not in accordance with the invention as claimed, in respect of testing plants (3-4 leaves), these plant species/varieties were exposed to 1/32 (3.125%) of recommended herbicide dose and phytochemical compounds were detected and correlated to the final effects as reduced growth before visually signs appeared on the plants.

Although not in accordance with the invention as claimed, accordingly, the detection of phytochemical effect may be possible as long as the plant is living. This detection may be performed between less than one day and up to at least 21 days after exposure, such as between 1-20 days after exposure, for example between 4-7 days after exposure. The testing time may also be any of the days mentioned in respect of producing a standard colour scale or the testing time may be even later. A later test time may also be at a time when the organism has been processed e.g. when plant material from a crop has been processed into a product to sell at the market. This product may include further components e.g. grains of a crop are processed into flour from which bread is produced. By testing the bread it may be possible to test whether the crop material used has been exposed to stress, to which stress type and/or whether the plant was a genetic modified plant.

Although not in accordance with the invention as claimed, in an embodiment the method of testing for effects relates to all pesticides representing groups with different mode of action. For example glyphosate and glyphosate like herbicides or sulfonylurea herbicides.

Although not in accordance with the invention as claimed, the detection of a fingerprint in a plant may in one embodiment of the invention serve the purpose of an "early warning" signal of stress exposure before any visual signs thereof appear on the plant.

Although not in accordance with the invention as claimed, it has been reported that when plants are exposed to stress they may react by changing their phytochemical composition. The present invention presents a method by which reproducible fingerprint relating to phytochemical compoundss are obtained, thus possible providing analytical tools for the establishment of exposure to and identification of known as well as unknown compounds. There are a variety of stress factors that may all have an impact on the chemical composition of plants. The plant may be exposed to more than one stress factor, wherein in one embodiment the effect of the exposure is synergistic and thus results in a fingerprint relating to phytochemical compounds reflecting the synergistic effect of the individual stress factors. In another embodiment, wherein the plant may be exposed to more than one stress factor, the resulting fingerprint relating to phytochemical compounds reflects the antagonistic effect of the individual stress factors. It is within the scope of the invention to develop a standard fingerprint relating to phytochemical compounds for any combination of stress factors.

According to the invention one of the stress factors is abiotic, such as chemical stress and/or physical stress.

In the present context chemical stress may be caused by pesticides, such as herbicides. Herbicides are all designed to kill plants by altering and affecting the biochemical homeostasis of the plant cells. Plants react to the exposure of herbicides by producing or decomposing phytochemical compounds. They may also react by changing the concentration of already existing compound(s). The resulting effect on the plants is dependent on the individual mode of action of the herbicide.

In an embodiment of the invention the method of testing for the exposure of pesticides relates to herbicides and/or pesticides comprising active ingredients selected from the group consisting of Glyphosate, Bromoxynil, Pendimethalin, Metsulfuron methyl, Prosulfocarb, Clodinafop-propargyl, Fenoxaprop-p-ethyl, lodosulfuron, Sulfosulfuron and Flupyrsulfuron or a combination thereof. The active ingredients may all represent different modes of action on the target plants. Pesticides/herbicides with these active ingredients are all widely used in Northern America and Western Europe for the control of e.g. broad-leaved plants and grasses. Other pesticides than the ones mentioned above are also within the scope of the invention. They may be the ones described in The Pesticide Manual, British Crop Protection Council. For example insecticides, acaricides, nematicides/vermicides, rodenticides and fungicides may be the stress inducing factors.

Glyphosate (GLY) is a non-selective herbicide that controls emergent annual and perennial broad-leaved plants and grasses. Glyphosate inhibits the activity of the EPSP-enzyme (5-enolpyruvylshikimate-3-phosphate) of the aromatic acid biosynthetic pathway in plants. It is absorbed through the wax cuticle on the leaves and a rapid translocation occurs via phloem to roots, rhizomes and apical meristems. It is degraded by rapid microbial action, with a half-life of 3-5 weeks. It is non-volatile and does not degrade photochemically. The water solubility is 11.6 g/l at 25 °C. It binds strongly to soil particles and hereby it is immobile unless transported with the soil.

Bromoxynil (BRY) is a selective herbicide with some systematic activity. The herbicide is absorbed by the foliage through cuticular penetration. Bromoxynil kills by inhibition of photosynthesis and plant respiration in annual broad-leaved plants. It degrades rapidly in most soil types, with a half-life in the order of two weeks which can be considerable reduced at low temperatures. It is water-soluble (130 mg/l), potentially harmful to fish and aquatic invertebrates for which it is toxic if it reaches water bodies.

Pendimethalin (PEN) is a selective herbicide that inhibits cell growth by inhibiting cell division of any and all plant cells by acting as a mitotic toxin. It is absorbed by roots and leaves, but initially limits root growth, such as the development of lateral or secondary roots. Pendimethalin is moderately persistent in moist sandy loam (half-life 50 days) to highly persistent in moist silty soil (half-life 140 days) and in dry silty clay loam (250 days). It is a very stable herbicide except when it volatilises from moist soil surfaces (Barrett & Lavy 1983). The water solubility is 0.3 mg/l at 20 °C. Thus, it is likely to be transferred to other environmental compartments although it may move with soil particles to water bodies where it is toxic to fish.

Metsulfuron methyl (METS) is a potent inhibitor of plant growth used on wheat and barley crops for the control of broad-leaf species and the suppression of few grasses. The herbicide is taken up by the foliage or the roots and translocated via xylem and phloem. Metsulfuron methyl is a selective herbicide that acts by inhibiting the enzyme acetolactate synthase (ALS) which catalyses the synthesis of the three branched-chain amino acids valine, leucine and isoleucine. The precise mechanism of action is unknown, but soon after herbicide application, plant cell division quickly stops, and death occurs within one to three weeks. The accumulation of ALS substrates (e.g. α-ketobutyrate) in leaves may be responsible for the cessation of the plant growth with decreased production of new leaves and reproductive organs. Metsulfuron methyl is mobile in most soil and the mobility is enhanced as pH increases.

All the above mentioned herbicides are currently applied to major crops, such as maize, wheat, barley, soybeans, oats, peas, potatoes and tomatoes. When applying herbicides to a cultivated field of crops adjacent non-target areas may be affected by herbicides as well. Although not in accordance with the present invention as claimed, the present invention could be used to test whether weeds or crop plants in crops treated with the herbicide respond as expected to this herbicide treatment. Although not in accordance with the present invention as claimed, the invention could also be used to test whether plants in a non-target area as described above actually is affected by a treatment in adjacent cultivated field. Hereby the test may indicate whether ecologically cultivated crops actually are free of herbicides or other chemical exposure

Although not in accordance with the present invention as claimed, the method of testing could be applied to plants potentially being exposed to physical stress, such as temperature, wind, UV light, physical damage, soil quality and soil moistness.

Although not in accordance with the present invention as claimed, the stress factors could be biotic, such as biological stress and/or allelopathy. The term "biological stress" is meant as stress and possibly visual damage caused by herbivores, plant pathogens and/or competition from other plants. The latter may also be referred to as allelopathy, such as competition from other plants and/or chemical compounds of other plants effecting/stressing the plant on which a test is performed.

Although not in accordance with the present invention as claimed, there is a difference in the sensitivity of plants against various stress factors, and it could therefore be recommended to use sensitive plants. This allows for the detection of pesticides which have been applied to target plants in even very small concentrations. An example of a model-plant is *Anagallis arvensis.*

The term "phytochemical" as used herein relates to any chemical or compound or nutrient or fundamental compound present in the plants. There are a vast number of compounds present in plants. Some of the compounds are readily detectable under circumstances where the plants are not exposed to pesticides. If, however, plants are exposed to pesticides the biochemical pathways within the plant cells may be affected. The influence of pesticides on biochemical pathway may lead to an increase or change, such as elimination in the concentration of already existing compounds, or it may lead to the production of compounds not normally present in plants not exposed to pesticides.

Although not in accordance with the present invention as claimed, in an embodiment of the invention the composition of phytochemical compounds of at least one type and/or group could be determined.

Although not in accordance with the present invention as claimed, in one embodiment of the invention the phytochemical could be a substance, or at least part of a substance, or a derivative of the groups amino acids, amines, sugars, flavonoids, phenolic compounds, sapogenins, saponins, iridoids, glycosides, alcaloids, alkaline alcaloids, C-containing compounds, N-containing compounds, S-contaning compounds, P-containing compounds, O-containing compounds, any fundamental elements, terpenoids, lipids, steroids, cartenoids, quinones, coumarines, and nutrients, such as any compound necessary for the plant to survive, for example salts. The phytochemical compounds can also be associated to the chemical compound or parts of the chemical compounds used as chemical stress to the plants e.g. decomposition of the chemical compound to be detoxificated of the plants by a reaction with the reactive groups of the phytochemical compounds.

By the term fundamental elements is meant any compound depicted in the periodical system.

The chemical analysis of pesticides is very difficult when the presence of the pesticide in the environment is low. Furthermore, it is very expensive to perform chemical screenings for chemical compounds, such as pesticides and/or their decomposition compounds and/or adjuvants present in pesticides. By the present invention it is now possible to determine different stress factors, such as pesticides by a simple and affordable method of testing.

Although not in accordance with the present invention as claimed, in one embodiment of the invention the method of testing could comprise the following steps:
- contacting an assayable form of plant material with a support for receiving said plant material,
- subjecting said support to a solvent,
- optionally drying said support,
- optionally contacting said support with a chemical reagent,
- obtaining a fingerprint relating to phytochemical compounds of said assayable form.

In another embodiment of the invention the method of testing comprises the following steps:
- obtaining an assayable form of a at least one group of chemical components of plant material e.g. by extraction the chemical components with a solvent,
- optionally reacting said at least one group of chemical components with a reagent,
- contacting a support with the extracted chemical components or to the chemical components reacted with a reagent,
- optionally drying said support,
- optionally contacting said support with a chemical reagent,
- detecting said extracted chemical components or said chemical components reacted with a reagent, where said detection may be due to a colour of the extracted chemical components or a colour of said chemical components reacted with a reagent and hereby
- obtaining a fingerprint relating to phytochemical compounds of said assayable form of plant material.

In the present context an assayable form may be a liquid, or a liquid mixed with solids, such as liquids mixed with salts.

in another embodiment, the testing comprising similar steps as described above but the chemical reaction is performed before the assayable form of the plant material is contacted with a support, hereby the support need not be subjected to a solvent.

Obtaining a fingerprint relating to phytochemical compounds may be in the form of a colour reaction on the support.

In an embodiment the chemical reagent or solvent is based on one or more of the compounds selected from the group of vanillin, sulphuric acid, naphtoresorcinol, methylene blue, β-naphtol, thymol, fluorescein, ammonia, bromocresol green, bromophenol blue, potassium permanganate, 2,7-dichlorofluorescein, rhodamin 6G, diphenyl boric acid 2-aminoethylester, phosphoric acid, iode, potassium iodide, ammoniummolybdattin(II) chloride, cobalt(II) chloride, palladium(II) chloride, 1-naphthol, ninhydrin, bismuth(III) nitrate, potassium iodide, molybdat phosphor acid, rhodamin B, anise aldehyde, silver nitrate, ferri(III) chloride, zinkchloride, chlorofenolred , methylred, ethylred, bromothymol blue, 2.6-dichlorophenolindophenole sodium salt, bromocresolpurpur, ninhydrine, potassium hydroxide, glucose, 4-chloro-7-nitrobenzofurazan, 2.4-dinitrophenylhydrazine, 9-fluorenylmethylchloroformate, tetrabutylammoniumhydroxide, iode, ammonium ferri(III)sulphate, 2-methoxy-2.4-diphenyl-3(2H)furanon (MDPF), 2-aminoethyl-diphenylborinate, aluminium chloride, berberine chloride dihydrate, 1.2-naphthochinon-4-sulfonsodium salt, anthrone, 8-hydroxychinolin, 2-aminodiphenyl(biphenyl-2-amine), orcinol, urea, 4-hydroxybenzoic acid, 4-aminobenzoic acid, molybdatophosphoric acid, 2'.7'-dichlorofluoresceine, 8-anilinonaphthaline-1-sulfonic acid-ammonium salt, rhodamine, bismuth(III) nitrate potassium iodide and chemicals or mixtures hereof.

Chemical reagent needed for the reaction is 0.001 - 10 mg/ml extract depending of the reaction mechanism, conditions and supplementary.

The support for receiving the material may be a solid material, solid support or a less solid material, such as a soft material, for example a liquid material. The support may be pretreated with a substance capable of promoting reactions when put into contact with the plant material. Said reactions may be detectable visual, radioactive, fluorescent, or immunological. Preferred is when the solid support is made of a material suitable of function as filter paper, such as e.g. nitrocellulose or Whatman paper.

In a preferred embodiment the solid material or solid support is in the form of a stick or a disk. Preferred is also a stick or disk made of a fabric capable of absorbing at least a part of the solution with the phytochemicals optionally reacted with a chemical reagent.

The solid support is optionally in the kit described elsewhere herein. The colour of the plant extract or extract reacted with a chemical reagent may be determined by placing a container with the extract and with or without a solid support within the container close to the standard colour scale or a standard result and determining the colour of the plant extract or extract reacted with a chemical reagent. The solid support may also be removed from the container before comparing the colour of the solid support with the colours of the standard colour scale.

The solid support can be used to contact the reacted extract, but can also be used to obtain a background with a standard colour behind a container containing a coloured sample from a plant, such as a coloured extract. When the solid support is used as a background colour e.g. a white colour this minimises the risk of an erroneous determining of the colour of the extract within a container.

In an embodiment the standard colour scale may be an integral part of the solid support or holding means comprising the solid support. The solid support may comprise a section for applying the extract or reacted extract of the material to be tested and another section of the solid support may comprise a standard colour scale. Holding means may be a cassette e.g. enclosing a solid support and a standard colour scale may be attached to the cassette and/or to the enclosed solid support.

The solid support may also function as a stick or disk which is placed within the container with the extract. The solid support may absorb part of or all the volume of the extract or may be immersed within the extract. The colour of the solid support or of the extract may be determined when the solid support has absorbed part of or all the extract or when the solid support is immersed within the extract, also the colour can be detected when a volume of the extract is absorbed onto only a part of the solid support. In the latter case the extract can be sucked up by the solid support e.g. by dipping the solid support into the extract or by applying extract on the solid support. By extract is meant raw extract from the organism to be tested, extract in a solvent e.g. in water or extract which has been subjected to a reaction e.g. a chemical reaction and/or a colour reaction.

In the method of testing according to the invention the assessment of pesticide exposure for material from plants, may be qualitative and/or semi-quantitative and/or quantitative. In one embodiment of the invention the assessment is qualitative, and phytochemical effects are detected as different colours. In a further embodiment the assessment is semi-quantitative where both colour and intensity are evaluated partly quantitative by the means of either visual inspection or use of an apparatus. The concentration of the sample may be determined as an approximate intensity value within a given interval or point system. In one of the embodiment of the invention the assessment is quantitative, and the phytochemical effect is detected as intensity (concentration of the compounds) reflecting the concentration of the pesticide. The quantitative evaluation may be performed by using a video analysis, scanning-densitometer or spectrophotometric analysis.

In an embodiment the use of chemical reagents and stick/disks to determine the effect of stress performed on a living organism comprises the following steps:
- contacting an assayable form of said living organism e.g. plant material with chemical reagents,
- providing a chemical reaction between the assayable form of material from the living organism and the chemicals,
- contacting the solid support with the material from the living organism chemically reacted with the chemicals, hereby
- obtaining a solid support with a colour (or detection in UV-light)
- comparing the colour and colour intensity with a colour standard,
- evaluating of expected final effect of stress on said living organism.

The final effect of a stress to detect in the living organism may be a reduction in growth or death.

In the present context the term "solvent" is meant to cover one substance or a combination of two or more substances, wherein the solvent may be a combination of liquid and solid and gas substances. A solvent may be a reagent, an eluent or an extraction media. The latter three may be in a solid or liquid physical state, or they may be in the form of a gas.

In one embodiment of the invention an extract of material from a plant is provided. The extraction may be performed under cold or warm temperatures, such as by the means of ultrasound, or stiming.

The extraction solvent may be any useable solvents. Non-limiting examples are water, alcohol, acids, ethers, petroleum, salts or a combination thereof. The solvents mentioned may be in any concentration, such as e.g. 5, 10, 15, 20, 25, 30, 40, 50, 60, 70, 80, 90 or 100%.

Examples of solvents are petroleum-ether solvents, 10 % acidic acid in 96 % ethanol, 75-80 % ethanol. The extraction may be performed on fresh or non-fresh plant material.

According to the invention the solvents and the support may have different polarities, such as between -0.1-10, for example between 2-8, such as between 4-6 as defined by Snyder, (1974).

An object of the present invention is to provide an assay kit for the determination of whether material from a living organism has been exposed to stress or to determine the effect of a stress on a living organism, the kit comprising
- at least one solvent and/or reagent,
- at least one standard colour scale,
- at least one container/glass
- optionally at least one solid support (e.g. sticks and/or disks).

The solvent and/or reagent may be solvent and/or reagent as described elsewhere herein. The amount of solvent/reagent may be between a few drops e.g. withheld on a solid support or in a flask to 100 mL. Preferred is solvent/reagent volume less than 75 mL, e.g. less than 50 mL, such as less than 25 mL, e.g. less than 15 mL. Preferred is also solvent and/or reagent volume between 0.5 to 5 mL, such as 5-10 mL, e.g. 10-15 mL, e.g. 15-20 mL. The volume of solvent and reagent may be different. Thus preferred volumes may be selected for each solvent/reagent among the ones mentioned above.

Preferred volume of raw extract may be between 0.1 mL and 5 mL, such as between 0.15 mL and 4 mL, e.g. between 0.2 mL and 3 mL, such as between 0.25 mL and 2 mL, e.g. between 0.3 mL and 1 mL, such as between 0.35 mL and 0.8 mL, e.g. between 0.4 mL and 0.5 mL.

An extract may be obtained by extracting an amount of plant material in a solvent, the ratio between the weight of plant material and the volume of solvent may be between 1:100 and 1:1, such as at least 1:80, e.g. at least 1:60, such as at least 1:40, e.g. at least 1:30, such as at least 1:25, e.g. at least 1:20; such as at least 1:15, e.g. at least 1:10, such as at least 1:5, e.g. at least 1:2. An example illustrating the described ratio is 0.2 mg plant material extracted with 3.5 mL of solvent.

The extract may be further diluted with the same solvent as used for extraction or with another solvent. The final ratio between the weight of plant material used initially for the extraction and the total volume of solvent optionally before performing any further reaction of the extract may be between 1:400 and 1:1, such as at least 1:300, e.g. at least 1:250, such as at least 1:200, e.g. at least 1:150, such as at least 1:100, e.g. at least 1:80; such as at least 1:70, e.g. at least 1:60, such as at least 1:50, e.g. at least 1:40. An example illustrating the described ratio is 0.2 mg plant material extracted with 3.5 mL of solvent and further diluted with 10 mL solvent.

The solvent and/or reagent may be substituted by antibodies to substances in one or more of the groups of compounds mentioned elsewhere herein. The antibodies when bound to phytochemicals may be detected by methods known in the art.

In yet another aspect of the invention an immunological test, such as a "dipstick" is used.

The at least one standard colour scale may also be a description of the colours which can be the colours to determine i.e. possible colours of the extract and among which the user has to distinguish.

Although not in accordance with the present invention as claimed, the colour scale or the description of the colours may be classified in respect to the final effect of the plants. The classification may be into a number of groups, e.g. 2, 3, 4, 5, 6, 7, 8, 9, or more groups. In respect of a classification with 3 groups, each group may include subgroups relating to single colours indicating final effects. A classification with 3 groups, which again may be based on 3 to 10 colours of the extract, may indicate for the user that the final effect of the stress can be characterised as no or only little effect on the growth of the plant; a certain level of reduction in growth of the plants e.g. a reduction of 30-80 %; and death or at least above 90% reduction in growth.

In an embodiment the kit comprises:
- at least one solid support (e.g. sticks and/or disks),
- at least one solvent,
- at least one squeezing means,
- optionally at least one standard colour scale,
- at least one glass.

The assay kit may further comprising one or more of the components selected from the group of
- at least one chemical reagent,
- at least one mortar with pistil and/or at least one box with balls to shake and/or at least one hand-press
- at least one pipette,
- at least one UV-lamp,
- at least one heater and/or at least one warm cap made of chemical reagents and solvents,
- at least one balance,
- at least one scissor,
- at least one pair of forceps,
- at least one plastic bag,
- at least one identification information to identify plant species,
- at least one instruction describing how to use the assay kit,
- at least one syringe,
- at least one filter.

Examples of elements further included in the kit comprise one of the combinations, although any combination of the elements listed above is intended to be described:
- at least one chemical reagent, mortar with pistil and/or balls to shake and/or hand-press, pipette.
- at least one chemical reagent, heater and/or warm cap made of chemical reagents and solvents, identification information to identify plant species.
- at least one chemical reagent, mortar with pistil and/or balls to shake and/or hand-press, heater and/or warm cap made of chemical reagents and solvents, syringe, filter.
- heater and/or warm cap made of chemical reagents and solvents, balance, scissor, identification information to identify plant species, instruction describing how to use the assay kit.
- at least one chemical reagent, mortar with pistil and/or balls to shake and/or hand-press, pipette, heater and/or warm cap made of chemical reagents and solvents, scissor, pair of forceps, identification information to identify plant species, instruction describing how to use the assay kit, syringe, filter.

In a preferred embodiment the kit'comprises the following components:
- 3 solid support (e.g. sticks and/or disks),
- 3 containers with lids and each with 2,3 or 4 glass balls
- 6 glasses,
- 3 syringes each with e.g. 13.5 mL solvent,
- 1-3 containers with chemical reagents,
- 3 pipettes,
- 1 balance,
- 1 scissor,
- 1 pair of forceps,
- 3 plastic bags,
- 1 identification information to identify plant species,
- 1 instruction describing how to use the assay kit including a standard colour scale,
- 3 filters.

The components of the kit may be located in a box. The box may be of cardboard and/or plastic or any other suitable material.

The glass balls of the kit may have any suitable size such as between 1 mM and 3 cM in diameter., e.g. between 1.1 mM and 1 cM, such as between 1.2 mM and 9 mM, e.g. between 1.3 mM and 8 mM, such as between 1.4 mM and 7 mM, e.g. between 1.5 mM and 6 mM, such as between 1.6 mM and 6 mM, e.g. between 1.7 mM and 5 mM, such as between 1.8 mM and 4 mM, e.g. between 1.9 mM and 3 mM, such as between 2 mM and 2.5 mM.

The glass balls are used to smash or squeeze the plant material by shaking a closed container containing the glass balls together with plant material and optionally a solvent.

The filter of the kit may be a filter paper used to filter the extract or it may be a filter cartridge including the filter, where the filter cartridge can be connected to a syringe. The syringe may be used to force the extract or reacted extract through the filter.

Containers with chemical reagents may have any suitable size, e.g. a size between 0.5 and 30 mL. The containers may include a volume of chemical reagent corresponding to a number of test to be performed e.g. to 1, 2, 3, 4, 5, 6 or more tests.

Solvents from a container or syringe may be used by dividing the volume such that the plant material is squeezed in a part of the solvent e.g. 3.5 mL e.g. by shaking for e.g. about 2 minutes and then again about ½ minute when e.g. 10 mL solvent is added.

In a preferred embodiment the test kit could detect the final effect in plants due to treatment with sulfonylurea-herbicides. The plants to test may be *Apera spica venti, Lolium perenne,* and *Poa annua* or another weed plant or other plants. The herbicide may be e.g. Hussar, Atlantis, Monitor and Lexus. Preferred are Hussar and Atlantis. The tests may be e.g. one or more of the tests described elsewhere herein.

The test kit could be used to test treated plants at an early stage such as 1-15 days after a treatment is performed. Preferred is a test between 4 and 9 days after exposure to the herbicides, such as 6 to 8 days after the exposure. Preferred is when performing a test 2, 3, 4, 5, 6, 7, 8, or 9 days after exposure of the stress. Such a test can be used to predict the effect of the stress performed e.g. the effect of a herbicide treatment.

When using the test of the invention to determine whether an organism has been subjected to a specific type of stress and the amount of this stress type, the test may be performed at a later stage that that described above. In this situation the test may be performed e.g. 15, 20, 25, 30, 35, 40, 45, 50, 55, 60, 65, 70, 75, 80, 85, 90, 95, 100, 110, 120, 130, 140, 150 or even more days after the plant were subjected to the stress. In such a test the actual date of a possible stress exposure may not be possible to interpret.

The test kit may be developed to test plants in stage or phase 12 (2 leaves) to stage or phase 23 (bushy). The stage/phase may also be 10, 13, 14, 15, 16, 17, 18, 19, 20, 21, 22, 24, 25, 26.

The test kit may include components and reagents for performing three tests. In respect of a crop field treated with herbicide it is preferred that three samples of weed plants are collected three different places in the treated field. Each sample may be of e.g. 20-25 plants. The test kit may also include components for more than 3 tests, e.g. 4, 5, 6, 7, 8, 9, 10, 11, 12, 13, 14, 15, 16, 17, 18, 19, 20 or even more.

In an embodiment the assay comprises components, where some components are to be used several times and some components are disposable.

In one embodiment the components which can be re-used is squeezing means e.g. mortar with pistil and/or at least one box with balls to shake and/or at least one hand-press, standard colour scale, glass, pipette, UV-lamp, heater, balance, scissor, pair of forceps, identification information to identify plant species, instruction describing how to use the assay kit, syringe, filter.

In another embodiment the components which can be re-used is squeezing means e.g. mortar with pistil and/or at least one box with balls to shake and/or at least one hand-press, standard colour scale, UV-lamp, heater, balance, scissor, pair of forceps, identification information to identify plant species, instruction describing how to use the assay kit.

Although not in accordance with the present invention as claimed, in an embodiment the disposable components may be solid support (e.g. sticks and/or disks), solvent, squeezing means e.g. mortar with pistil and/or at least one box with balls to shake and/or at least one hand-press, standard colour scale, glasses, chemical reagent, pipette, UV-lamp, heater and/or at least one warm cap made of chemical reagents and solvents, scissor, pair of forceps, plastic bag, identification information to identify plant species, instruction describing how to use the assay kit, syringe, filter.

Although not in accordance with the present invention as claimed, in another embodiment the disposable components may be solid support (e.g. sticks and/or disks), solvent, squeezing means e.g. mortar with pistil and/or at least one box with balls to shake and/or at least one hand-press, glasses, chemical reagent, pipette, heater and/or at least one warm cap made of chemical reagents and solvents, plastic bag, syringe, filter.

The assay kit could be re-establish with disposable components after at least one use, hereby the kit is ready to use again.

It is a purpose of the invention to lower the costs and time of the testing procedure, and at the same time provide a method of testing having excellent sensitivity. The assay kit of the invention may for all practical purposes to be used as a field test, or as a laboratory test. One object of the invention is to have an easy accessible test to be used commercially or on a private scale. Thus, the assay kit of the invention is in one embodiment practical and portable in size and easy to operate. The test material is brought into contact with a support for receiving said material. The test material is in an assayable form, for example in the form of a liquid suspension.

Although not in accordance with the present invention as claimed, in an embodiment the assay test kit is produced as a practical and mobile system which can be fully or partly disposable as described elsewhere. The individual step of the test may be performed in the field, and need not require any particular technical skills of the person performing the test. The test may be completed in less than 3 hours from the living organism is obtained, such as in less than 2 hours, e.g. less than 1 hour, such as less than 45 min, e.g. less than 30 min, such as less than 15 minutes.

In an embodiment the test method of the invention is performed with the following steps:
- Plant material (test material) is collected,
- The test material is cut into small pieces with the scissor and weighed on the balance,
- The material is brought into contact with a solvent,
- The mortar with a pistil, or a box with balls to shake, together with a solvent, crush the sap of the plant material.
- A hand-press may be used to press out the sap of the plant material.
- The suspension is filtered through a filter.
- The extract is brought into contact with chemical reagents
- After a chemical reaction is obtained the reacted extract may have a different colour when compared to the extract
- Optionally the reacted extract is contacted with a solid support e.g. a stick or disk, and a colour will appear on the solid support,
- The colour of the reacted extract itself or the colour of the solid support is compared to a standard colour scale, from this comparison the type of stress and/or the effect of the stress upon the plant can be determined

In an embodiment the test kit comprises containers with solvent and/or reagents, such as solvents and/or reagents described elsewhere herein. The containers may be in the form of e.g. flasks, glasses, and syringes. The containers may have a lid.

In another embodiment the assay kit comprises containers wherein the chemical reactions are performed. The(se) container may be container(s) with solvent and/or reagent.

In one embodiment all components of the assay kit are contained in one enclosure, this optionally being as a pocket size unit.

In an embodiment the method of the present invention is used for testing whether plant material is exposed to pesticide stress. The invention also relates to the use of an assay kit for determination of whether a plant has been exposed to pesticide stress. It can also be tested what effect the pesticide stress will have on the plant. In another embodiment the invention may be used by a farmer to assess the effect of a given pesticide on plants being exposed to similar or different levels of a pesticide. In this way the farmer is able to determine at what dosages of the pesticide a certain effect is obtain on plants.

In a further embodiment, the invention may be used to identify non-exposed plants and/or to identify plant species and/or plant varieties by using the differences in composition and concentration of phytochemical compounds, i.e. chemotaxonomy. The test including the standard colour scale is in this embodiment developed with respect to the different chemicals of the non-exposed and exposed plants and/or the different chemicals of different plant species and/or the different chemicals of different plant varieties.

In an aspect of the invention the method and/or assay kit is used for testing whether material from a living organism, including plant material has been exposed to stress.

Examples of uses according to the invention may be in the control of crops such as determining effects of stress upon weeds and pests such as insect pests.

The use of the method and/or assay as described herein may also be in the control of gene modified plants. It is envisioned that the fingerprint relating to phytochemical compounds of plants being genetically modified may be determined by using the present test method. Plants may be genetically modified to become resistant to pesticides, and such gene modified plants may produce phytochemical compounds that differ from phytochemical compounds of non-gene modified plants. In one embodiment of the invention the fingerprint relating to phytochemical compoundss of gene-modified plants and non-gene-modified plants, which have not been exposed to pesticides, can be compared to detect the gene-modified or non-gene-modified plant. The test including the standard colour scale may be designed to respond with a yes or no answer in respect of whether the plant is a gene-modified plant. The test may also be directed towards a response indicating which type of gene or exactly which gene the plant is modified with.

In another aspect the invention may be used in the control of the geographic distribution of pesticides. Non-target habitats adjacent to cultivated fields may be affected by pesticides during application. This exposure may occur due to over-spraying, or through spray drift from the application on target crops adjacent to wild-life habitats. It may also stem from pesticides being run-off or washed-off. Pesticides are able to travel considerable distances by air, either by drift or by volatilisation. The test including the standard colour scale may be designed to respond with a yes or no answer in respect of whether the plant has been subjected to pesticide(s). The test may also be designed to indicate the amount of the pesticide applied to the plant.

In a further aspect the invention may be used by the farmer to assess the optimal effect of a given pesticide on plants being exposed to reduced levels of a pesticide. In this way the farmer is able to determine at what minimum dosages of pesticide a plant is still responding, and may thus be able to reduce the amount of pesticide necessary to obtain a given effect in the plant. The test including the standard colour scale may be designed to respond with a yes or no and optionally also a perhaps answer in respect of whether the plant has been subjected to pesticide(s) in an amount that will result in desired effect of the plant growth. The test may also be designed to indicate the amount of the pesticide applied to the plant.

In yet another aspect the invention may be used in food quality control, such as in the control of farmers produce, such as crops. Particularly the invention may be used for the control of whether organic crop has been exposed to stress, such as herbicides. It is important to be able to determine whether organic crops are free from residues of chemicals, such as herbicides and/or defoliating agents and/or growth regulating agents, such as respiration and germination inhibition agents, growth retarding agents, root formation agents, flowers and fruit formation agents, germination promoting agents, flowering delaying agents, thinning out agents, hold- on compounds and grafting agents, or free from residues of chemicals used for: the control or treatment of plant diseases, wood destroying fungi, unwanted plant growth, growth of algae, slime promoting organisms in paper pulp, animals capable of damaging utility-and cultivated plants, vermin on domestic animals, infested cereal, cereal products, seeds and feed-stuff, textile infestant, infestant of lumber and woodwork, insects, snails, mites, rain worms, rabbits, water voles, moles, mice and rats, or free from residues of chemicals for the prevention of damages caused by vermin and chemicals for the exclusion of vermin from specific geographical areas. The test including the standard colour scale may be designed to respond with a yes or no answer in respect of whether the plant has been subjected to stress. The test may also be designed to indicate what type of stress the plant has been subjected to.

Conventional control methods of testing for residues of for example herbicides include labour intensive and expensive analytical methods, based on gas chromatography or liquid chromatography, such as HPLC. However, since the active chemical groups of most herbicides, defoliating agents and/or growth regulating agents are being broken down to residual concentrations below detection limit, the task of detecting break down products requires the performance of several different chemical analysis for every individual herbicide, defoliating agents and/or growth regulating agent.

The method and/or assay kit of the invention can also be used to determine from which plant species and/or plant variety a plant material is obtained.

The method of the tests described herein may also be designed to be based on a cut-off value by which a colour development indicates one answer and where no colour development indicates another answer. This may especially be of value when performing a test to determine a "yes or no answer" as described elsewhere herein such as e.g. whether a plant is a gene-modified plant. The colour indicating the value above or below a cut-off colour may be performed by the colour of the extract itself optionally reacted with one or more chemical reagents, or the colour may be a colour of the standard colour scale.

### Examples

The following is examples illustrating different embodiments of the invention.

### Example 1

### Method

### Plant material:

The method was tested on 10 different plant species, representing 4 different plant families including both mono- and dicotyledons:

| | |
|---|---|
| *Plant species* | Plant families |

| Monocotyledons | |
|---|---|
| *Apera spica venti* | Poaceae |
| *Lolium perenne* | Poaceae |
| *Poa annua* | Poaceae |
| *Lolium multiflorum* | Poaceae |
| *Alopercurus myosuroides* | Poaceae |
| *Bromus hordeaceus* | Poaceae |
| *Avena fatua* | Poaceae |
| | |

| Dicotyledons | |
|---|---|
| *Taraxacum vulgare* | Asteraceae |
| *Urtica dioeca* | Urticaceae |
| *Stellaria media* | Caryophyllaceae |

The plants were sown and cultivated in green-house, some were cultivated as semi-field studies with Danish out-door conditions or collected directly in the field in Jutland close to Hobro or Sealand close to Naestved and Kalundborg.

### Green-house conditions:

The experiments were performed in the green-house at the Danish Institute of Agricultural Research (now University of Aarhus), Research Centre Flakkebjerg, Slagelse, Denmark. Plants were grown in 2L pots in a potting mixture consisting of soil, sand and peat (2:1:1 w/w %) containing all necessary macro- and micro-nutrients. The pots were placed in a heated glasshouse (14°C) with supplemental artificial light (16 hours photoperiod). The pots were sub-irrigated twice a day with de-ionised water.

### Semi-field conditions:

The experiments were performed out-door under semi-field conditions at the Danish Institute of Agricultural Research (now University of Aarhus), Research Centre Flakkebjerg, Slagelse, Denmark. Plants were grown in 2L pots in a potting mixture consisting of soil, sand and peat (2:1:1 w/w %) containing all necessary macro- and micro-nutrients. The pots were placed out-door. The pots were sub-irrigated twice a day with de-ionised water.

### Field conditions:

The experiments were performed by the Danish Institute of Agricultural Research (now University of Aarhus), Research Centre Flakkebjerg, Slagelse, Denmark and Hobro - Aalborg Union of Farmers, Hobro, Denmark. Famers fields were selected containing the weed plant species to be investigated.

**Herbicides tested (Table 1)**

| Trade-mark | Active ingredient (a.i. g/kg-l) | Additives | Recommended rates |
|---|---|---|---|
| Atlantis WG | Mesosulfuron 30g/kg + iodosulfuron 6 g/kg | Mefenpyr-diethyl 90g/kg (safener) Biopower (1l/ha) or Renol (0.5l/ha) | 150g/ha (autumn) 150-300g/ha (spring) |
| Boxer EC | Prosulfocarb 800g/l | None | 3.5 l/ha |
| Hussar | Iodosulfuron 50g/kg | Mefenpyr-diethyl 150g/kg (safener) Renol (0.5l/ha) | 200g/ha |
| Hussar OD | Iodosulfuron 100g/kg | Mefenpyr-diethyl 300g/kg (safener) Renol (0.5l/ha) | 0.1 l/ha (Winter crops) |
| Lexus 50 WG | Flupyrsulfuron-methyl-Na 500g/kg | 0.1% Lissapol Bio | 20g/ha |
| MaisTer | Foramsulfuron 300g/kg + iodosulfuron 10g/kg | Isoxadifen 272 g/kg (safener) | 150 g/ha |
| Monitor | Sulfosulfuron 800 g/kg | 0.1% Lissapol | 21.88 g/ha |
| Primera Super | Fenoxaprop-P-ethyl 69g/l | Safener 69g/l 0.2% Isoblette | 1.0 l/ha |
| Roundup Bio | Glyphosat 360 g/l | None | 3.5 l/ha |
| Stomp 400 EC | Pendimethalin 400g/l | None | 4.0 l/ha |
| Topik 100 EC | Clodinafob-propargyl 100g/l | Cloquintocet-mexyl 25g/l (safener) 0.5 l/ha Renol | 0.4 l/ha |

The herbicides were commercial available formulations obtained directly from the producers. The amount of the herbicides was adjusted according to the growth stage of the crops treated with the herbicides.

### Green-house

Herbicide application was carried out at the four-leaf stage. A recommended dose of the herbicide was applied in de-ionized water using a laboratory pot sprayer fitted with two Hardi-ISO F-110-02 flat fan nozzles in a spray volume of 145 L ha⁻¹. The plants were harvested 14 days after exposure and immediately freeze-dried and kept dry protected to light.

### Semi-field

Herbicide application was carried out at the three to four-leaf stages. A dose of 0, 1/32 N, 1/16 N, 1/8 N, ¼ N, ½ N, 1 N (N = recommended dose) of the herbicide was applied in de-ionized water using a laboratory pot sprayer fitted with two Hardi-ISO F-110-02 flat fan nozzles in a spray volume of 145 L ha⁻¹. The plants were evaluated for visual effects before harvested 4, 7, 14 and 21 days after exposure and immediately frozen in plastic bags to avoid water evaporation and kept in freezer (- 18°C).

### Field

Herbicide application was carried out at the three to four-leaf stages. A dose of 0, ¼ N, ½ N, 1 N (N = recommended dose) of the herbicide was applied in de-ionized water using a field-sprayer. The plants were harvested 4 and 7 after exposure and tested directly in the field. Biomass reduction was determined 21 days after exposure.

### Visual effects on plants exposed to the herbicides.

Four, seven, fourteen and twenty-one days after exposure, visual effects on plants were noted before harvest, using the rating chart below in Table 2 as used in Ravn (2000).

**Table 2. Rating system used to assess herbicide visual effects (VE).**

| **Rating/ scale** | **Detailed description of visual effects observed on plants exposed to herbicides** |
|---|---|
| 0 | No effect |
| 1 | Trace effect: Normal appearance, generally associated with a slight growth stimulation |
| 2 | Slight effect i.e. weak reduced biomass |
| 3 | Moderate effect: plant 75% the size of control (decrease by 25%) |
| 4 | Injury/damage: plants more than 50% of control and with some clear visible injury on leaves and stem |
| 5 | Definite injury: plants half size of control, leaf epinasty (curved leaves), plant parts deformed and discoloured |
| 6 | Herbicidial effect: plants 25% size of control, leaf epinasty, plant parts deformed and discoloured |
| 7 | Good hebicidial effect: very small plants, leaf epasty, plant parts deformed and discoloured |
| 8 | Approaching complete kill, only few green parts left |
| 9 | Complete kill/dead |

### Extraction procedure/sample preparation

In one aspect of the invention the extraction solvent may be a solvent with a high lipid solubility, or a solvent with moderate lipid solubility as e.g. 75% ethanol or a hydrophilic solvent as e.g. 10% acetic acid in water or pure water.

### Fresh or frozen plant material

The fresh or frozen plant material was cut into small pieces with a sissor. An amount of 200 mg was weighed and crushed in a mortar/pistil with 4.00 ml pure water. The green water suspension was filtered using a syringe with a Whatman 0.45 µm GMF w/GMF filter before immediately use.

### Freeze-dried plant material

Freeze-dried plant material was weighed and extracted with either 75% ethanol or pure water (50 mg/l). The extraction was performed in an ultra-sonic bath for 2 hours. The temperature was kept < 0ºC (using ice) during the extraction to avoid decomposition of biomarkers. The green water suspension was filtered using a syringe with a Whatman 0.45 µm GMF w/GMF filter before use.

### Stick A

Three drops of extracts (one drop = 80.22 mg +/- 22mg) were added in plastic medical glas (d = 25 mm in the botton) and mixed with 3 drops of reagent A (= glas I) (ninhydrin 8% in 96% ethanol). A heating cap was prepared using a new plastic medical glas with 10 drops of water and 15 drops of concentrated sulfuric acid (the temperature will be ca. 70 °C) (glas II). Glas I is immediately placed in glas II. After 15 minutes* a stick (4 cm x 1 cm Advantec filter no. 526 dipped in paraffin so that 1 x 1 cm is pure filter) was dipped and the colour was identified using a PANTONE^{®} formula guide solid uncoated (www.pantone.com/register). The sticks were photographed in a special created paper box (holes with diameter = 5 mm) using CAMAG Digistore 2 with Camag WinCats software using CAMAG Lamp (white light), a CAMAG Reprostar 3 box with a digital camera fitted (DXA252 with a 12 mm linse. A CAMAG VideoScan Programme with software was used for computer analysis of the intensity in AU units (hights of peaks). Stick A detected the group of amino acids and amino acid derivatives.

The colours referred to as PANTONT-colours are as in PANTONE Formula Guide/ Solid Uncoated ISBN 978-1-590650-63-9 Fourth Edicion Second Printing.

### Stick B

Three drops of extracts were added in plastic medical glas and mixed with 10 drops (0.5 ml) of pure water and 2 drops of reagent B (α- naphthol 5% in 96% ethanol). Fifteen drops of concentrated sulfuric acid (70 °C) were added. The warm strong acid performed the chemical reaction and a colour reaction occured*. After 15 minutes a stick was dipped into the sample and the colour was identified using a PANTONE^{®} formula guide solid uncoated and photographed and analysed as described for Stick A. Stick B detected the group of carbohydrates and carbohydrate derivatives.

*If only the PANTONE^{®} colour was evaluated a disk d = 2.5 cm of Advantec filter no. 590 was placed into the bottom of the glass with the colour reaction for both Stick A and B.

### Biomass determination

The biomasses of the plants were weighed as gram weight per pot. Six to ten plants were grown in each pot depending on the plant species. The plants were harvested close to the soil and weighed (fresh weight). Then the plants were placed in a drying cupboard at 80°C in 18 hours and then weighed again (dry weight).

### Results

In table 3 the herbicide, plant species, dose, relative fresh and dry weight of control plants and visual effects on the plants due to herbicide treatment are presented. In Table 4 and 5 the reduction in fresh and dry biomass is indicated as well as colours obtained on the sticks after 4 and 7 days after exposure for stick A and stick B.

**Table 3. The herbicide, plant species, dose of herbicide, relative fresh and dry weight of plants in respect of control plants and treated plants. Visual effects are indicated for 4, 7, 14 and 21 days after treating the plants with the herbicide.**

| Herbicide | Plant species | Dose (g/ha) | Relative fresh weight (g/pot) | Relative dry weight (g/pot) | Visual effect 4 days after exposure | Visual effect 7 days after exposure | Visual effect 14 days after exposure | Visual effect 21 days after exposure |
|---|---|---|---|---|---|---|---|---|
| Atlantis | *Apera spica-venti* | 0 | 100.0 (38.2) | 100.0 (34.8) | 0 | 0 | 1.0 (1.3) | 0.8 (1.0) |
| | | 0.625 | 54.1 (9.3) | 56.8 (10.9) | 0 | 0 | 2.3 (0.6) | 2.0 (0.0) |
| | | 1.25 | 51.5 (9.6) | 51.6 (10.0) | 0 | 0 | 2.0 (1.0) | 2.3 (0.6) |
| | | 2.5 | 30.8 (3.6) | 34.1 (4.4) | 0 | 0 | 3.3 (0.6) | 4.0 (0.0) |
| | | 5 | 32.086.6) | 34.6 (7.1) | 0 | 0 | 5.3 (0.6) | 4.3 (0.6) |
| | | 10 | 16.1 (7.3) | 18.6 (8.8) | 0 | 3.0 (0.0) | 6.0 (0.0) | 6.0 (0.0) |
| | | 20 | 11.8 (4.7) | 12.8 (5.4) | 0 | 4.0 (0.0) | 6.7 (0.6) | 7.0 (0.0) |
| | | | | | | | | |
| | *Poa annua* | 0 | 100.0 (24.1) | 100 0 (13.6) | 0 | 0.0 (0.0) | 0.0 (0.0) | 0.3 (0.6) |
| | | 3.9 | 38.1 (14.3) | 39.4 (14.2) | 0 | 3.0 (0.0) | 3.0 (0.0) | 1.0 (1.7) |
| | | 7.8 | 19.0 (5.9) | 22.4 (4.8) | 0 | 4.0 (0.0) | 4.0 (0.0) | 3.7 (0.6) |
| | | 15.6 | 8.6 (0.8) | 12.4 (2.5) | 0 | 4.0 (0.0) | 4.0 (0.0) | 6.0 (0.0) |
| | | 31.3 | 5.7 (5.3) | 9.0 (6.8) | 0 | 4.0 (0.0) | 4.0 (0.0) | 7.0 (0.0) |
| | | 62.5 | 5.1 (2.0) | 10.0 (2.8) | 0 | 4.0 (0.0) | 5.0 (0.0) | 7.0 (0.0) |
| | | 125 | 5.0 (1.2) | 11.2 (2.7) | 0 | 4.0 (0.0) | 5.0 (0.0) | 7.0 (0.0) |
| | | | | | | | | |
| Hussar | *Apera spica-venti* | 0 | 100.0 (38.2) | 100.0 (34.8) | 0 | 0.0 (0.0) | 1.0 (1.3) | 0.8 (1.0) |
| | | 3.125 | 52.4 (12.2) | 55.0 (8.9) | 0 | 0.0 (0.0) | 2.0 (0.0) | 2.0 (0.0) |
| | | 6.25 | 41.7 (12.6) | 43.7 (7.0) | 0 | 3.0 (0.0) | 2.0 (0.0) | 3.0 (0.0) |
| | | 12.5 | 20.9 (6.0) | 24.5 (7.3) | 0 | 4.0 (0.0) | 3.0 (0.0) | 4.0 (0.0) |
| | | 25 | 23.9 (6.4) | 25.7 (6.3) | 0 | 4.0 (0.0) | 4.7 (1.5) | 4.0 (0.0) |
| | | 50 | 14.5 (3.9) | 16.7 (2.1) | 0 | 4.0 (0.0) | 6.7 (0.6) | 6.0 (0.0) |
| | | 100 | 14.8 (9.5) | 18.4 (8.2) | 0 | 4.0 (0.0) | 6.7 (0.6) | 7.3 (0.6) |
| | | | | | | | | |
| | *Lolium perenne* | 0 | 100.0 (16.5) | 100.0 (15.2) | 0 | 0.0 (0.0) | 0.0 (0.0) | 0.0 (0.0) |
| | | 1.56 | 83.8 (13.2) | 85.5 (10.6) | 0 | 0.0 (0.0) | 0.0 (0.0) | 0.0 (0.0) |
| | | 3.125 | 31.2 (11.1) | 36.0 (9.4) | 0 | 2.0 (0.0) | 3.7 (0.6) | 4.0 (0.0) |
| | | 6.25 | 24.7 (11.5) | 29.0 (12.1) | 0 | 3.0 (0.0) | 4.7 (0.6) | 5.0 (0.0) |
| | | 12.5 | 8.8 (4.1) | 12.9 (3.2) | 0 | 4.0 (0.0) | 6.0 (0.0) | 6.3 (0.6) |
| | | 25 | 1.9 (0.2) | 6.6 (0.7) | 0 | 4.0 (0.0) | 7.0 (0.0) | 8.0 (0.0) |
| | | 50 | 1.4 (0.1) | 5.3 (0.7) | 0 | 5.0 (0.0) | 7.0 (0.0) | 9.0 (0.0) |
| | | | | | | | | |
| Monitor | *Apera spica-venti* | 0 | 100.0 (33.8) | 100.0 (30.4) | 0.0 (0.0) | 0.0 (0.0) | 0.0 (0.0) | 0.5 (0.8) |
| | | 0.313 | 58.1 (15.2) | 60.6 (15.4) | 0.0 (0.0) | 1.0 (0.0) | 2.3 (0.6) | 2.0 (1.0) |
| | | 0.625 | 37.6 (5.9) | 40.1 (8.2) | 0.0 (0.0) | 1.0 (0.0) | 3.0 (0.0) | 3.0 (0.0) |
| | | 1.25 | 26.4 (3.3) | 31.3 (4.0) | 0.0 (0.0) | 1.0 (0.0) | 5.0 (0.0) | 5.0 (0.0) |
| | | 2.5 | 11.6 (4.5) | 14.5 (3.8) | 1.0 (0.0) | 2.3 (0.6) | 6.0 (0.0) | 6.3 (0.6) |
| | | 5 | 8.5 (2.9) | 14.1 (5.0) | 2.0 (0.0) | 3.7 (0.6) | 6.0 (0.0) | 7.0 (0.0) |
| | | 10 | 4.7 (1.8) | 8.6 (1.4) | 2.3 (0.7) | 4.7 (0.6) | 6.7 (0.6) | 8.7 (0.6) |
| | | | | | | | | |
| | *Bromus hordeaceus* | 0 | 100.0 (2.1) | 100.0 (2.0) | 0.0 (0.0) | 0.0 (0.0) | 0.0 (0.0) | 0.0 (0.0) |
| | | 0.39 | 50.6 (4.3) | 53.1 (9.6) | 0.7 (0.6) | 2.0 (0.0) | 3.0 (0.0) | 2.3 (0.6) |
| | | 0.78 | 31.8 (5.7) | 38.7 (9.2) | 1.0 (0.0) | 2.3 (0.6) | 3.0 (0.0) | 3.3 (0.6) |
| | | 1.56 | 16.3 (6.0) | 21.8 (7.1) | 1.0 (0.0) | 2.7 (0.6) | 4.0 (0.0) | 5.0 (0.0) |
| | | 3.13 | 9.5 (2.0) | 16.6 (3.7) | 2.0 (0.0) | 3.0 (0.0) | 4.0 (0.0) | 5.0 (0.0) |
| | | 6.25 | 7.1 (2.0) | 15.7 (0.8) | 2.0 (0.0) | 3.0 (0.0) | 5.0 (0.0) | 7.3 (0.6) |
| | | 12.5 | 5.7 (0.5) | 13.3 (1.0) | 2.0 (0.0) | 3.7 (0.6) | 6.0 (0.0) | 8.0 (0.0) |
| | | | | | | | | |
| Lexus | *Apera spica-venti* | 0 | 100.0 (33.8) | 100.0 (19.4) | 0.0 (0.0) | 0.0 (0.0) | 0.0 (0.0) | 0.5 (0.8) |
| | | 0.625 | 60.4 (10.1) | 65.4 (10.9) | 0.3 (0.6) | 0.7 (0.6) | 3.7 (0.6) | 2.0 (0.0) |
| | | 1.25 | 70.9 (8.2) | 84.3 (4.6) | 0.0 (0.0) | 1.0 (0.0) | 3.0 (0.0) | 2.0 (0.0) |
| | | 2.5 | 60.6 (9.9) | 78.3 (13.5) | 1.0 (0.0) | 2.0 (0.0) | 3.7 (0.6) | 2.0 (0.0) |
| | | 5 | 30.1 (9.5) | 37.1 (7.6) | 1.0 (0.0) | 2.7 (0.6) | 5.3 (0.6) | 4.7 (1.2) |
| | | 10 | 22.2 (18.4 | 31.6 (23.5) | 1.7 (0.6) | 3.3 (0.6) | 5.0 (1.0) | 6.3 (1.2) |
| | | 20 | 11.3 (8.2) | 18.2 (12.3) | 2.3 (0.6 | 5.0 (0.0) | 5.7 (0.6) | 7.0 (1.0) |
| | | | | | | | | |
| | *Alopecurus myosurorides* | 0 | 100.0 (26.9) | 100.0 (25.1) | 0.0 (0.0) | 0.0 (0.0) | 0.0 (0.0) | 0.0 (0.0) |
| | | 0.5 | 16.2 (7.2) | 17.1 (6.2) | 4.3 (0.6) | 4.3 (0.6) | 5.0 (0.0) | 6.3 (0.6) |
| | | 1 | 7.1 (3.3) | 9.8 (3.8) | 4.7 (0.6) | 4.7 (0.0) | 6.0 (0.0) | 7.7 (0.6) |
| | | 2 | 5.4 (3.1) | 6.8 (4.01) | 4.7 (0.6) | 5.0 (0.0) | 6.0 (0.0) | 8.0 (0.0) |
| | | 4 | 1.9 (0.8) | 3.6 /(0.3) | 5.0 (0.0) | 5.3 (0.6) | 7.0 (0.0) | 9.3 (0.0) |
| | | 8 | 2.7 (1.0) | 6.9 (3.2) | 4.7 (0.6) | 5.0 (0.0) | 7.0 (0.0) | 9.0 (0.0) |
| | | 16 | 3.5 (2.5) | 6.8 (4.6) | 5.3 (0.6) | 5.7 (0.6) | 7.0 (0.0) | 10.0 (0.0) |

In figure 1 the PANTONE®-colours, the intensity (AU) calculated on the CAMAG equipment (equipment for analysis of pictures) and the reduced growth (fresh- and dry weight as 100%-the relative weight calculated 21 days after exposure) are presented for 4 and 7 days after application of the herbicide. For *Poa annua* exposed to Atlantis and *Lolium perenne* exposed to Hussar the results are presented 4 days after exposure.

**Table 4. Semi-field dose/response of Apera spica-venti, Lolium perenne and Poa annua (3 to 4 leaves) exposed to Hussar and Atlantis. All data is mean values of three replicates. The values in brackets are standard deviations. The numbers mentioned in respect of the colour stick are the PANTONE numbers.**

| Herbicide | Plant species | Dose (g a.s./h a) | Visual effect 4 days after exposure | Visual effect 7 days after exposur e | Reduction in fresh biomasse (%) | Reduktion in dry biomasse (%) | PANTONE ^{®}-colour Stick A 4 days after exposure | PANTONE^{®}-colour Stick A 4 days after exposure | PANTONE ^{®}-colour Stick A 7 days after exposure | PANTONE^{®}-colour Stick A 7 days after exposure | PANTONE ^{®}-colour Stick B 4 days after exposure | PANTONE ^{®}-colour Stick B 4 days after exposure | PANTONE^{®}-colour Stick B 7 days after exposure | PANTONE^{®}-colour Stick B 7 days after exposure |
|---|---|---|---|---|---|---|---|---|---|---|---|---|---|---|
| Atlantis | *Aperaspica-venti* | 0 | 0 | 0 | 0 (38.2) | 0 (34.8) | Very light violet | 270 | Very light red violet | 7437-7439 | Light blue green | 5517 | Light gray green | 7542 |
| | | 0.625 | 0 | 0 | 45.9 (9.3) | 43.2 (10.9) | Light violet | 271 | Very light red violet | 7439 | Blue green | 443 | Light gray green | 7542 |
| | | 1.25 | 0 | 0 | 48.5 (9.6) | 48.4 (10) | Violet | 272 | Red violet | 7440 | Light gray | 7544 | Gray | 7545 |
| | | 2.5 | 0 | 0 | 69.2 (3.6) | 65.9 (4.4) | Dark violet | 273 | Red violet | 7440 | Light gray | 7544 | Gray | 7545 |
| | | 5.0 | 0 | 0 | 68 (6.6) | 65.4 (7.1) | Dark violet | 273 | Dark violet | 260 | Light gray | 7544 | Blue | 535 |
| | | 10.0 | 0 | 3 (0) | 83.9 (7.3) | 81.4 (8.8) | Darker violet | 2745 | Darker violet | 261 | Gray | 7545 | Dark blue | 655 |
| | | 20.0 | 0 | 4 (0) | 88.2 (4.7) | 88 (5.4) | Darker violet | 2755 | Darker violet | 262 | Blue | 535 | Dark blue | 655 |
| Atlantis | *Poa annua* | 0 | 0 | 0 | 0 (24.1) | 0 (13.6) | Light creme | 7527 | - | - | Light yellow green, | 5783-5645 | - | - |
| | | 3.9 | 0 | 3 (0) | 61.9 (14.3) | 60.6 (14.2) | Very light red violet | 665 | - | - | Yellow green | 443-5645 | - | - |
| | | 7.8 | 0 | 4(0) | 81 (5.9) | 77.6 (4.8) | Red violet | 667 | - | - | Gray blue | 7546-535 | - | - |
| | | 15.6 | 0 | 4 (0) | 91.4 (0.8) | 87.6 (2.5) | Red violet | 667 | - | - | Gray blue | 7546-535 | - | - |
| | | 31.3 | 0 | 4 (0) | 94.3 (5.3) | 91 (6.8) | Dark violet | 273 | - | - | Dark blue | 534 | - | - |
| | | 62.5 | 0 | 4 (0) | 94.9 (2) | 90 (2.8) | Dark | 273 | - | - | Dark | 534 | - | - |
| | | | | | | | violet | | | | blue | | | |
| | | 125 | 0 | 4 (0) | 95 (1.2) | 88.8 (2.7) | Dark violet | 273 | - | - | Dark blue | 534 | - | - |
| Hussar | *Apera spica-venti* | 0 | 0 | 0 | 0 (24.1) | 0 (13.6) | Very light violet | 270 | Very light red violet | 7437-7439 | Light blue green | 5517 | Light gray green | 7542 |
| | | 3.125 | 0 | 0 | 61.9 (14.3) | 60.6 (14.2) | Violet | 272 | Very light red violet | 7439 | Blue green | 443 | Light gray green | 7542 |
| | | 6.25 | 0 | 3 (0) | 81 (5.9) | 77.6 (4.8) | Violet | 272 | Red violet | 7440 | Gray | 7545 | Gray | 7545 |
| | | 12.5 | 0 | 4 (0) | 91.4 (0.8) | 87.6 (2.5) | Dark violet | 273 | Red violet | 7440 | Blue | 535 | Gray | 7545 |
| | | 25 | 0 | 4 (0) | 94.3 (5.3) | 91 (6.8) | Dark violet | 273 | Dark violet | 260 | Blue | 535 | Blue | 535 |
| | | 50 | 0 | 4 (0) | 94.9 (2) | 90 (2.8) | Dark violet . | 273 | Darker violet | 261 | Dark blue | 534 | Dark blue | 655 |
| | | 100 | 0 | 4 (0) | 95 (1.2) | 88.8 (2.7) | Dark violet | 273 | Darker violet | 262 | Dark blue | 534 | Dark blue | 655 |
| Hussar | *Lolium perenne* | 0 | 0 | 0 | 0 (16.5) | 0 15.2 | Light creme | 7527 | - | - | Very light yellow green | 5803 | - | - |
| | | 1.56 | 0 | 0 | 16.2 (13.2) | 14.5 (10.6) | Very light red violet | 664 | - | - | Light gray | 7544 | - | - |
| | | 3.125 | 0 | 2 (0) | 68.8 (31.2) | 64 (9.4) | Red violet | 7446 | - | - | Blue | 535 | - | - |
| | | 6.25 | 0 | 3 (0) | 75.3 (11.5) | 71 (12.1) | Dark violet | 272 | - | - | Blue | 535 | - | - |
| | | 12.5 | 0 | 4 (0) | 91.2 (4.1) | 87.1 (3.2) | Dark violet | 272 | - | - | Dark blue | 534 | - | - |
| | | 25 | 0 | 4 (0) | 98.1 (0.2) | 93.4 (0.7) | Darker violet | 273 | - | - | Dark blue | 534 | - | - |
| | | 50 | 0 | 5 (0) | 98.6 (0.1) | 94.7 (0.7) | Darker violet | 273 | - | - | Dark blue | 534 | - | - |

**Table 5. Semi-field dose/response of Apera spica-venti, Bromus hordeaceus and Alopecurus myosurorides (3 to 4 leaves) exposed to Monitor and Lexus. All data is mean values of three replicates. The values in brackets are standard deviations. The numbers mentioned in respect of the colour stick are the PANTONE numbers.**

| Herbicide | Plant species | Dose (g a.s./h a) | Visual effect 4 days after exposure | Visual effect 7 days after exposure | Reduction in fresh biomasse (%) | Reduktion in dry biomasse (%) | PANTONE^{®}-colour Stick A 4 days after exposure | PANTONE ^{®}-colour Stick A 4 days after exposure | PANTONE^{®} -colour Stick A 7 days after exposure | PANTONE^{®} -colour Stick A 7 days after exposure | PANTONE^{®} -colour Stick B 4 days after exposure | PANTONE^{®} -colour Stick B 4 days after exposure | PANTONE^{®}-colour Stick B 7 days after exposure | PANTONE^{®} -colour Stick B 7 days after exposure |
|---|---|---|---|---|---|---|---|---|---|---|---|---|---|---|
| Monitor | *Apera spicaventi* | 0 | 0 | 0 | 0 (33.8) | 0(30.4) | Very light red violet | 664 | Light creme | 7527 | Light blue green | 5517 | Very light green | 5665 |
| | | 0.313 | 0 | 1 (0) | 41.9 (15.2) | 39.4 (15.4) | Light red violet | 665 | Light creme | 7527 | Light gray | 7544 | Light blue | 536 |
| | | 0.625 | 0 | 1 (0) | 62.4 (5.9) | 59.9 (8.2) | Red violet | 7445 | Light creme | 7527 | Blue | 535 | Light blue | 536 |
| | | 1.25 | 0 | 1 (0) | 73.6 (3.3) | 68.7 (4) | Darker red violet | 7446 | Light rose | 5245 | Blue | 535 | Blue | 535 |
| | | 2.5 | 1 (0) | 2.3 (0.6) | 88.4 (4.5) | 85.5 (3.8) | Dark violet | 272 | Very light red violet | 664 | Dark blue | 534 | Dark blue | 534 |
| | | 5 | 2(0) | 3.7 (0.6) | 91.5 (2.9) | 85.9 (5) | Darker violet | 273 | Red violet | 665 | Dark blue | 534 | Very dark blue | 2766 |
| | | 10 | 2.3 (0.7) | 4.7 (0.6) | 95.3 (1.8) | 91.4 (1.4) | Dark violet | 272 | Red violet | 666 | Dark blue | 534 | Dark blue | 534 |
| Monitor | *Bromus hordeaceus* | 0 | 0 | 0 | 0 (2.1) | 0 (30.4) | Light creme | 7527 | Light creme | 7527 | Light blue | 536 | Very light yellow green | 5803 |
| | | 0.39 | 0.7 (0.6) | 2(0) | 49.4 (4.3) | 39.4 (15.4) | Light creme | 7527 | Light creme | 7527 | Blue | 535 | Blue | 535 |
| | | 0.78 | 1 (0) | 2.3 (0.6) | 68.2 (5.7) | 59.9 (8.2) | Light creme | 7527 | Light rose | 5245 | Blue | 535 | Blue | 535 |
| | | 1.56 | 1 (0) | 2.7 (0.6) | 83.7 (6.0) | 68.7 (4) | Light creme | 7527 | Light creme | 7527 | Blue | 535 | Very dark blue | 2766 |
| | | 3.13 | 2(0) | 3 (0) | 90.5 (2.0) | 85.5 (3.8) | Light creme | 7527 | Light rose | 5245 | Blue | 535 | Very dark blue | 2766 |
| | | 6.25 | 2(0) | 3 (0) | 92.9 (2) | 85.9 (5) | Light creme | 7527 | Light rose | 5245 | Dark blue | 534 | Dark | 534 |
| | | 12.5 | 2(0) | 3.7 (0.6) | 94.3 (0.5) | 91.4 (1.4) | Light creme | 7527 | Light rose | 5245 | Blue | 535 | Very dark blue | 2766 |
| Lexus | *Apera spica-venti* | 0 | 0 | 0 | 0 (33.8) | 0(19.4) | Very light red violet | 664 | Light creme | 7527 | Light gray green | 7542 | Very light yellow green | 5803 |
| | | 0.625 | 0.3 (0.6) | 0.7 (0.6) | 39.6 (10.1) | 34.6 (10.9) | Very light red violet | 663 | Light creme | 7527 | Light gray green | 7542 | Very light yellow green | 5803 |
| | | 1.25 | 0 | 1 (0) | 29.1 (8.2) | 15.7 (4.6) | Very light red violet | 663 | Very light red violet | 663 | Gray green | 441 | Very light green | 5655 |
| | | 2.5 | 1 (0) | 2 (0) | 39.4 (9.9) | 21.7 (13.5) | Very light red violet | 663 | Light creme | 7527 | Very light gray | 7543 | Light gray green | 7542 |
| | | 5 | 1 (0) | 2.7 (0.6) | 69.9 (9.5) | 62.9 (7.6) | Violet | 271 | Very light red violet | 664 | Blue | 535 | Blue | 535 |
| | | 10 | 1.7 (0.6) | 3.3 (0.6) | 77.8 (18.4) | 68.4 (23.5) | Violet | 271 | Light red violet | 665 | Blue | 535 | Dark blue | 534 |
| | | 20 | 2.3 (0.6) | 5 (0) | 88.7 (8.2) | 81.8 (12.3) | Dark violet | 272 | Dark violet | 272 | Blue | 535 | Very dark blue | 2766 |
| Lexus | *Alopecurus myosurorides* | 0 | 0 | 0 | 0 (26.9) | 0(25.1) | Very light red violet | 664 | Very light red violet | 665 | Light blue | 536 | Very light green | 5665 |
| | | 0.5 | 4.3 (0.6) | 4.3 (0.6) | 83.8 (7.2) | 82.9 (6.2) | Dark violet | 272 | Light violet | 7445 | Blue | 535 | Dark blue | 534 |
| | | 1 | 4.7 (0.6) | 4.7 (0.6) | 92.9 (3.3) | 90.2 (3.8) | Dark violet | 272 | Light violet | 7444 | Blue | 535 | Very dark blue | 2766 |
| | | 2 | 4.7 (0.6) | 4.7 (0.6) | 94.6 (3.1) | 93.2 (4) | Dark violet | 272 | Violet | 270 | Dark blue | 534 | Very dark blue | 2766 |
| | | 4 | 5 (0) | 5 (0) | 98.1 (0.8) | 96.4 (0.3) | Dark violet | 272 | Violet | 271 | Dark blue | 534 | Very dark blue | 2766 |
| | | 8 | 4.7 (0.6) | 4.7 (0.6) | 97.3 (1) | 90.4 (3.2) | Violet | 271 | Violet | 270 | Dark blue | 534 | Very dark blue | 2766 |
| | | 16 | 5.3 (0.6) | 5.3 (0.6) | 96.5 (2.5) | 93.2 (4.6) | Violet | 270 | Violet | 270 | Dark blue | 534 | Very dark blue | 2766 |

### Discussion

In general for both Stick-types the intensity (AU) of the colours increased with increasing dose and per cent of reduced growth 21 days after exposure. For stick A the colours varied from beige, light red violet to dark violet and for Stick B the colours varied from green to light grey to dark grey to light blue and finally dark blue. The CAMAG equipment can not separate the colours but only the intensity and consequentlythe intensity of some of the green and grey colours may have the same intensity as the darker blue colour. It is therefore important to use the qualitative colours detection (PANTONE^{®}) of the Sticks to evaluate the final effect of the weed plants.

For Stick B the non-exposed plants showed a different green colour. This colour difference can be used to identify the different plant species without any stress exposure.

For all the plant species, except for *Apera spica-venti* exposed to Atlantis and *Bromus hordeaceus* exposed to Monitor, the final effect in per cent reduced growth 21 days after exposure could be predicted 4 days after exposure without any visual effects to be detected on the plants (see Table 3). For *Apera spica-venti* exposed to Atlantis and *Bromus hordeaceus* exposed to Monitor seven days were needed.

Analysis of plants exposed to herbicides of different mode of action (i.e. iodosulfuron, glyphosate, prosulfocarb etc.) have shown the same pattern of responses with slightly different colours depending on the other compounds in the plants, the sensitivity of the plants to the herbicide and the age of the plants. Other ongoing experiments show that natural stressors such as temperature and drought may slow down the phytochemical reaction time, but the pattern of dark colours of the Sticks related to a high per cent of reduced growth is the same.

Minor differences in pattern reaction were detected when plants were developed in green-house, outdoor or in the field. The plants cultivated in the green house were more sensitive than the plants cultivated in the field.

The change in colour and colour intensity appeared in the annual plants about four days after exposure depending the sensitivity to the herbicides. The older plants were less sensitive to the herbicides.

### Example 2

The embodiments disclosed as example 2 are outline of the results from different test evaluating the method described elsewhere herein. First the different types of experiments are described. The results are presented afterwards.

### Semi-field study (dose - response)

The results from the semi-field experiments shows for certain plant species and herbicides a connection between the stick/disk-colour and the biomass reduction (relative biomass in per cent of control) can be detected already 4 days after the exposure. In dose-response studies 4 and 7 days after exposure a significant response is seen in relation to untreated plants with more than 80% effect on the biomass for both stick/disk A and B after exposure with Atlantis and Hussar.

The semi-field study was performed with concentrations of herbicide and/or additives which were higher that used normally when treating plants in the fields. The purpose of these studies were to obtain a reaction within the plants and thus to evaluate the possible colours which could be obtained directly in the plant extract or developed due to the chemical reactions performed when adding at least one chemical reagent to the plant extract. With the semi-fiels study also the final effect of the herbicides has been in focus i.e. the effect on the plant growth measured as reduced biomass when compared to non-treated plants.

### Semi-field studies (rain stability)

For the semi-field studies with rain stability there was a large deviation in biomass reduction using herbicides without and with additives and rain treatment different time after exposure.

A significant result for colour intensity down to 50% reduction effect of the biomass for the rain stability studies with Atlantis on *Lolium perenne* was seen. For the analysis seven days after herbicide treatment for stick/disk B, there was a good agreement between the doses of the herbicide where the curve for colour intensity was flatten and where more than 80% biomass reduction is seen.

### Semi-field studies (mixture with other herbicides)

In a semi-field study it was investigated whether a mixture with other herbicides influence the colour response for the different stick-types. The tested mixtures were Hussar in combination with Oxitril, Starane or Luxus (See the Table 15 of study No. 945/06). The results showed a good correlation between the colour on the stick/disk and the final biomass reduction effect for Hussar as a single herbicide or in a mixture with Starane and Express. Significant results of the colour intensity for the treatments where a final biomass reduction more than 80% was detected. There were no results with false positive responses with the stick-colours.

A mixture of Hussar and Starane gives the same results as for Hussar alone (see the Table 15 of study No. 945/06). However a mixture of Express and Hussar increases the final biomass reduction and the colour intensity for the sticks. The herbicides Express, Hussar and Atlantis are sulfonylurea-herbicides. Express has a main effect to dicotyledons of weed species, but also some effect to the monocotyledons *Lolium* sp.. There is a good correlation between the colour reaction and the final biomass reduction effect

### Field-studies

Some field experiments performed in both the autumn 2005 as in the spring 2006 support the results observed in the semi-field studies. The analyses were performed in the field on fresh colleted plant material.

For *Apera spica-venti* the herbicide doses tested turned out to be too high, and therefore there were no good differentiation between the final biomass reduction and the colours on the sticks/disks for medium herbicide doses, but the test clearly indicated the usability of the kit and method. For the field studies, seven days after herbicides treatment was needed before the analysis were performed. Hereby it was secured that the reactions of the plants had time to develop and any deviation due to decreased reaction within the plants in the lower temperatures of the autumn was eliminated.

At Sealand for *Lolium perenne* significant colour changes was observed for both stick/disk-type A and B analyses with a final reduction in biomass of more than 80%.

In total, the stick/disk B type shows a high correlation between the colour of the sticks and the final biomass reduction within three effect levels: No effect, medium effect and full effect. These experiments showed the possibility to develop a kit which is a tool for farmers to fast and early to detect herbicide effects in weed plants at least for *Lolium perenne, Poa annua* and *Apera spica-venti* exposed to Hussar and/or Atlantis.

### The reaction of plants to herbicide stress

Plant reacts to stress as e.g. exposure to herbicides as biochemical responses. Herbicides are developed to affect general or specific mechanisms in plants which involve that sensitive plants will die. When these mechanisms in the plants are affected, naturally the plant will react changing the concentration of already present compounds, produce new compounds or stop the production of compounds. The specific changes depend of the mode of action of the herbicide and the used dose.

The plant has a natural content of phytochemical compounds. The content of the phytochemical compounds is different in the different plant species, even though these plant species may be plant species of the same family. It has been observed that herbicide treatment of the plants result in a change in the phytochemical composition and concentration of these compositions in the plants. These changes can be detected and visualised to the human eye. The changes in phytochemical composition and concentration in the plants after herbicide stress exposure is called biomarkers. The biomarker method of the present invention is based on a pattern of biomarkers developed in plants exposed to herbicides.

Significant changes in the phytochemical composition and content in wild mono- and dicotyledon plants could be detected four days after exposure down to 1% of recommended field-dose of herbicides. This is before visual signs could be detected on the plant. In plants exposed to glyphosate (the herbicide Roundup Bio), phytochemical changes in composition and content (biomarker pattern) could be detected four hours after herbicide exposure.

Experiments have been performed with 16 different wild plant species selected among monocotyledons and dicotyledons plants, and a clear pattern of biomarkers was detected for four different herbicides with four different mode of action. The results showed, the higher concentration of the herbicides, the shorter time after the exposure the biomarkers could be detected.

Experiments based on young plants and older plants showed that younger plants react faster after herbicide exposure than older plants.

### Plant material, techniques and methods

### Plants

The plant species used for the studies are all common weed plants in crops in Denmark. The plant species are: Silky-bent grass *(Apera spica-venti* L., Beauv), foxtail grass (*Alopecurus myosuroides* Hudson), perennial ryegrass (*Lolium perenne* L.), lobgrass (*Bromus hordeaceus* L.) and annual meadow grass (*Poa annua* L.).

### Herbicides

The herbicides tested were: Hussar (iodosulfuron + mefenpyr-diethyl (safener), (50 + 150) g/kg, Bayer CropScience A/S), Hussar OD (iodosulfuron + mefenpyr-diethyl, (100 + 300) g/l, Bayer CropScience A/S); Atlantis WG (mesosulfuron + iodosulfuron + mefenpyr diethyl, (30 + 6 + 90) g/kg, Bayer CropScience A/S); Lexus 50 WG (flupyrsulfuron-methyl, 500 g/kg, DuPont Denmark A/S); Monitor (sulfosulfuron, 800 g/kg, Monsanto Crop Sciences Denmark A/S). In table 6 an overview of plant species, herbicides and treatments of the studies are presented.

**Table 6: Overview of studies, plant species, herbicides and treatments.**

| **Code** | **Plant species and stages at herbicide treatment** | **Herbicide** | **Treatment** | **Treatment of material before testing** |
|---|---|---|---|---|
| 972/04 | *Lolium perenne* (3-4 leaves), *Apera spica-venti* (3-4 leaves, *Poa annua* (3-4 leaves) | Hussar | Semi-field (rain stable) | Freeze-dried |
| 927/05 | *Lolium perenne* (3-4 leaves), *Apera spicaventi* (3-4 leaves) | Hussar | Semi-field (dose/response) | Fresh, frozen |
| 927/05 | *Apera spica-venti* (3-4 leaves), *Poa annua* (3-4 leaves) | Atlantis | Semi-field (dose/response) | Fresh, frozen |
| 928/05 | *Apera spica-Venti* (3-4 leaves), *Bromus hordeaceus* (3-4 leaves) | Monitor | Semi-field (dose/response) | Fresh, frozen |
| 928/05 | *Apera spica-venti* (3-4 leaves), *Alopecurus myosuroides* (3-4 leaves) | Lexus | Semi-field (dose/response) | Fresh, frozen |
| 945/06 | *Lolium perenne* (3-4 side shots) | Hussar OD; Oxitril; Starane; Express | Semi-field (herbicide mixture) | Fresh, frozen |
| 946/06 | *Lolium perenne* (3-4 side shots) | Atlantis | Semi-field (rain stable) | Fresh, frozen |
| Hobro | *Poa annua* (*Lolium perenne*) | Hussar | Field | Fresh, frozen |
| Hobro | *Poa annua* (*Lolium perenne*) | Atlantis | Field | Fresh, frozen |
| 964/05 | *Lolium perenne* | Hussar | Field | Fresh, frozen |
| 964/05 | *Lolium perenne* | Atlantis | Field | Fresh, frozen |
| 966/05 | *Apera spica*-*venti* | Hussar | Field | Fresh, frozen |
| 966/05 | *Apera spica-venti* | Atlantis | Field | Fresh, frozen |
| 948/06 | *Lolium perenne* (2 leaves, section, 2 knots) | Hussar OD | Field (Stage of development) | Fresh, frozen |
| 917/06 | *Lolium perenne* | Atlantis | Field | Fresh |

### Semi-field study - Cultivation and exposure of plant species

The weed plant species to be tested were sown in 2 L pots in a mixture of field soil, sand and sphagnum (2:1:1 weight per cent). After sowing, the pots were placed on out-door tables, where they were watered several times per day. After sprouting the plants in the pots were thinned out to have the same number of plants in all the pots. The herbicides were applied when the plants were at the right stage (se table 6). The herbicides were applied in deionised water using a laboratory pot sprayer fitted with two ISO F-02-110 flat fan nozzles delivering a spray volume of 145 L per Ha with a pressure of 3bar.

In the studies different effect levelsof the herbicide was aspired to be able to correlate the colour reaction on the sticks/disks with the final reduced growth of the plants (relative per cent of controls). Variable effects are obtained with different doses and in certain cases at following rain treatment, where a part of the herbicide is washed off. In study no. 945/06 Hussar OD is squirt out in mixtures with herbicides to combat dicotyledons to investigate if the tank mixture with other herbicides affects the colour reaction on the sticks/disks.

The normal dose (1 N) used is variable between the studies depending of the sensitivity of the weed plant species, stage of development of the plants and rain treatment. The doses are chosen with a view to obtain large variation in the effects of the treatments, which enter in an alliance with the different studies. The normal dose is therefore not in all the studies the same as the recommended dose of the herbicide. Although the purpose of the studies primarily has been to correlate final effect of the plant growth and colour reaction on the stick, the doses in all the results are presented as relative doses in relation to the normal dose, where normal dose is the dose indicated by the manufacturer of the herbicide.

The plants used for the biomarker test were harvested 4 and 7 days after exposure with the herbicides. The plants were cut at the surface of the soil and frozen immediately by the use of dry-ice (study no. 972/04 was freeze-dried). Before harvest a visual evaluation of effect (morpholodical changes), as described below were performed. The used scale for evaluation is outlined in table 2. Biomass determinations of the plants were performed at the harvest 21 days after exposure, as described below. Before the harvest of biomass a visual evaluation of the effect was performed.

### Semi-field studies with dose/response (927/05 & 928/05)

The study no. 927/05 contained loose silky-bent grass and annual meadow grass treated with Atlantis (1 N= 20 g/ha in respect of loose silky-bent grass and 125 g/ha in respect of annual meadow). Both loose silky-bent grass and perennial ryegrass were treated with Hussar (1 N= 100 g/ha and 50 g/ha, respectively). Both herbicides were squirt out in 6 doses in a mixture with 0.5 l/ha Renol (Bayer Crop Science). Renol is an additive that increases the penetration of the herbicide into the plant. The study no. 928/05 contained loose silky-bent grass and lobgrass treated with Monitor (1 N= 10 g/ha and 12 g/ha, respectively for the mentioned plant species). Furthermore, loose silky-bent grass and foxtail grass were treated with Lexus (1 N= 20 g/ha and 16 g/ha, respectively for the mentioned plant species). The herbicides were squirt out in 6 doses in a mixture with 0.1% Lissapol Bio (Syngenta). Lissapol Bio is an additive that decreases the surface tension and the contact between the herbicide and the plant is increased as the herbicide sticks better to the plant. Both studies included untreated study areas.

### Semi-field study with rain stability (972/04 & 946/06)

The study no. 972/04 contained perennial ryegrass (3-4 leaves) treated with 6 doses of Hussar (1 N= 200 g/ha) separately and in a mixture with 0.5 l/ha Renol. The rain treatments were performed in a rain simulator with an intensity of 20 mm/hour. A treatment with 5 mm rain, 1 and 4 hours after exposure were performed. The collected plant material was freeze-dried. In the study no. 946/06 perennial ryegrass (3-4 leaves) treatment with 6 doses of Atlantis and in the mixture with 0.5 l/ha Renol was performed. The used doses of Atlantis were 480 g/ha in treatment without rain and 1920 g/ha in treatments with rain. Corresponding in a mixture with Renol was used 120 and 960 g/ha, respectively. A treatment with 3 mm rain at the intensity of 10 mm/h respectively 1 and 3 hours after exposure were used.

### Semi-field study with mixture of herbicider (945/06)

The study was performed with perennial ryegrass and Hussar (1 N= 30 ml/ha) and in a mixture with 3 herbicides solely with effect on dicotyledon weed plants: (0.5 l/ha Oxitril (200 g/l ioxynil + 200 g/l bromoxynil), 0.6 l/ha Starane (180 g/l fluroxypyr) and 1 tablet/ha Express (3.75 g/tablet tribenuron).

### Field-study 2005 and 2006 - Cultivation and exposure

The goal of the field studies was to validate the test kit under natural conditions, where factors as soil humidity, nutrient supply and types of soil were variable. These factors could affect the content of natural compounds in the plants and hereby change the colour reaction on the used sticks. The studies were planned in two winter wheat fields close to the city Hobro and two winter wheat fields at Sealand (Moerkoev and Vejloe). Furthermore, two field experiments with perennial ryegrass were sown in pure stand.

In the studies with winter wheat 3 doses (1/4, 1/2 and 1N) of Hussar and Atlantis were used. Both herbicides were applied in a mixture with 0.5 l/ha Renol. The exposure was performed in the autumn 2005 and in the spring 2006. At Sealand a self-propelling sprayer equipped with LD-015-110 nozzle. A pressure of 3.2 bar and a solvent amount of 150 l/ha was used. In the study at Moerkoev perennial ryegrass was the dominating weed plant species. The plants was exposed to the herbicide on the 24^{th} of October 2005 (1 N=150 g/ha Hussar and 200 g/ha Atlantis), where the growth stage of perennial ryegrass were BBCH 11-12 (leaf development). In the spring the exposure was performed the 28th of April (1 N=200 g/ha Hussar and 300 g/ha Atlantis). At that time perennial ryegrass was at a BBCH growth stage of 30.2 (stretching). In the study at Vejloe loose silky-bent grass was the dominating weed specie. This study was sprayed the 14th of November (1 N=100 g/ha Hussar and 150 g/ha Atlantis), where loose silky-bent grass was at the BBCH growth stage 10-11 and the 11^{th} of Maj (1 N= 150 g/ha Hussar and 150 g/ha Atlantis), where loose silky-bent grass was at the BBCH growth stage 30-31. In Hobro at Koldkaergaard sprayer equipped with a flat spreading nozzle 4110-16 was used. A pressure of 2.5 bar and a solvent amount of 150 l /ha was used. In study no. 02 perennial ryegrass was the dominating weed plant specie. The herbicide in the study was sprayed the 19th of September (1 N=100 g / ha Hussar and 1 N=200g/ha Atlantis), where the perennial ryegrass was at BBCH growth stage 11-12 and the 4th of Maj (1 N=150 g/ha Hussar and 1 N=200 g/ha Atlantis), where perennial ryegrass was at the BBCH growth stage 30.

In study no. 03 perennial ryegrass was the domination weed plant specie. This study was sprayed the 20th of September (1N=150 g/ha Hussar and 200 g/ha Atlantis), where the annual meadow grass was at the BBCH growth stage 12, and the 15th of May (1N=200 g/ha Hussar and 300 g/ha Atlantis) where annual meadow grass was at the BBCH growth stage 33. The studies were conducted with 4 replicates for each treatment. In the study, plant material was removed for perennial ryegrass, annual meadow grass and loose silky-bent grass 4 and 7 days after exposure. The test-kit was tested directly in the field. At the same time samples were frozen with dry-ice for later testing in the laboratory.

Furthermore, two studies of perennial ryegrass in pure population were performed in the spring 2006. In the first study the importance of development stage for the final result of the method was investigated. In the study, the plants were exposed with 4 doses (1/8, 1/4, 1/2 and 1 N) of Hussar OD (1N=0,075 L/ha) in a mixture with 0.5 l/ha Renol at 3 different development stages (9th of June BBCH growth stage 12 (leaf development), 19^{th} of June BBCH growth stage 30 (stretching) and 29th of June BBCH growth stage 32 (stretching)). Visual evaluations and the final effect biomass reduction (fresh weight) were observed 42 days after exposure. In the second study the effect of Atlantis was investigated. The plants in the study were exposed the 28th of April, when perennial ryegrass was at the stage 29. Atlantis (1 N= 400 g/ha) was squirt out in 4 doses (1/8, 1/4, 1/2 and 1N) in mixture with 1 l/ha Biopower (Bayer Crop Science). Biopower is an additive.. Both studies were with 4 replicates of each treatment. Visual evaluations of effect were conducted 38 days after exposure.

### Physiological effects

Two different types of physiological effects were observed. An evaluation of the visual effects of the plants at each harvest was performed (see Visual effect evaluation). In the field studies a visual evaluation of the effect of autumn squirting was detected in the spring after the growth was started, while the effect of the spring squirting was evaluated ca. 6 weeks after squirting. Furthermore a final biomass determination was conducted 21 days after squirting (see biomass evaluation). In the field study, a visual evaluation of the effect of through sliding spikes was performed.

### Visual effect evaluation

The visual effect evaluation on the plants before sampling was evaluated according to table 2. The rating/scale 0 reflects no effect on the plants and 9 reflects dead plants (Hamil *et al.,* 1977; Boutin *et al.,* 1993). In the field studies the visual evaluations 5-6 weeks after squirting were based on a reduction of the weed plants cover of area in relation to untreated parcels.

### Biomass evaluation

In the semi-field studies the biomass determination was performed 21 days after squirting. The fresh weight was calculated with 3 replicates for each treatment. The plant material was harvested and air dried at 80° C for 18 hours, after which the dry-weight was calculated. For the field studies the biomass of the weed plants was calculated by cutting off the weed plants in 3 x 0.25 m² of each parcel. The number of plants was registered and weighted (fresh weight).

### Techniques used for method development

For method development different analytical chemical techniques has been used. Thin Layer Chromatography (TLC) to separate and select relevant biomarkers and biomarker groups was used. This TLC-technique forms the background for the development of the stick-method. TLC methods can be used to evaluate the effect of herbicide treatment, although the method is based on separation of the compositions of the plant material. The plant samples from the semi-field studies and field studies in 2005 were analyzed shortly after freezing. The plant samples from the field studies in 2006 were tested with fresh collected plant material directly in the field 4 and 7 days after exposure with the herbicides. Also analysis of frozen plant samples from the same spots was performed in the laboratory to calculate the high of the intensity (this feature is further described below) of the colours of the sticks.

### Collection of weed plants

Three samples (20-25 plants) for each field were collected 4 and 7 days after exposure with Hussar or Atlantis. The plants were immediately frozen with dry-ice or tested within 30 min..

### Colour, colour intensity and high of colour intensity

Colour and colour intensity of sticks from test of plants from semi-field studies were compared with a PANTONE^{®}formula guide uncoated scale and also analysed using advanced CAMAG picture analytical equipment. Here the colour intensity of each stick was measured in a 5 mm (diameter) cardboard shape in a closed box with white light (CAMAG Video-Store). The high of the area from the intensity curve of the electronic signal from the equipment was used as a value of the colour intensity. The intensity (high of area) is correlated with the concentration of the biomarkers in the extract (Lambert-Beer's Law).

The PANTONE-colours were evaluated visually and used since the colours observed by the CAMAG-equipment were not determined. Using the CAMAG-analysis the intensity between dark and light colours was used. However, the colour of e.g. blue and green to be seen for stick B was not possible using the CAMAG-equipment. The comparison between high in colour intensity for stick A followed the scale from light violet for the untreated plants to dark violet for the treated plants at high herbicide exposure (and effect). The colours for stick B were easy to evaluate using PANTONE-colours since light to dark green was detected in the extract of untreated plants and light to dark gray and light to dark blue for extracts of plants treated with herbicides. A value detected by the CAMAG for a dark green and dark blue could easily be detected similar if not the colour was identified using the PANTONE-colours.

The results from the CAMAG-analysis of the studies 927/05 and 928/05 were calculated as mean-values with standard deviation. These values are used as estimated of values the colour results of the field studies where laboratory analysis was not possible to conduct.

### Preparation of plant extract

In study no. 972/04 freeze-dried plant material was used. This plant material was crushed and 250 mg was extracted with 5.00 ml ELGA-water (deionized water) in 2 hours with ice in an ultra sonic bath. The samples were filtered through WHATMAN 0.45 urn GMF w/GMF filters. The extracts were treated as for the frozen and fresh plant extracts (see 2.7.3 Analysis with sticks A and B).

In the other studies fresh or frozen plant material was cut into small pieces with a scissor. 200 mg fresh plant material was weighed on a KERN 60-2N Pocket balance (max = 60g d = 0.01g) and crushed in a mortar (inner diameter = 5.2 cm) with 4.00 ml deionised water. The extract was filtered through a WHATMAN 0.45 µm GMF w/GMF filter. This filtered extract was used for stick A and stick B.

### Analysis performed with the sticks-method

This stick-method and its component described below is one embodiment of the method and test-kit. Two stick types denoted stick A and stick B are described. The two groups of biomarkers analysed by the method/sticks are amino acids and related compounds (stick A) and carbohydrates, carbohydrate derivatives and related compounds (stick B).

### Stick A

Plant material was harvested, an extract of the plant material was obtained, and the extract was filtered. Three drops of the filtered extract was placed in a plastic glass (plastic glass no. 1) together with 3 drops of reagens A (8% ninhydrin in 96% ethanol). A warm cap (plastic glass no. 2) was prepared by adding 0.5 ml deionized water with 15 drops of sulphuric acid dropped into the glass with ½ second between each drop (the reaction initiated a heating process at about 70°C). Glass no. 1 was immediately placed into glass no. 2 and both glasses were tilted to ensure heating of the material in glass no. 1. The heat from glass no. 2 will increase the chemical reaction in glass no. 1. After 10-15 minutes a stick of 1 cm x 4cm Adventec filter no. 526, where 3 cm was impregnated with paraffin, and thus 1 x1 cm without paraffin, were contacted with the solvent/plant extract such that the section without paraffin was entered into the solvent in glass no. 1 for about 5 seconds. (The paraffin makes the stick easier to handle and stops the penetration of the plant extract further into the stick, hereby the amount of absorbed plant extract is substantially equal in all tests as the volume/area of the stick into which the plant extract is absorbed is similar for each test). The colour of the stick was immediately (within 5-15 minutes) evaluated comparing the colour of the stick with a PANTONE^{®}-formula guide uncoated scale and the Pantone number was noted. For the analysis in the laboratory the sticks were placed in cardboard shape and analysed using CAMAG Video-Store box as described above.

### Stick B

Three drops of filtered extract were added into a plastic glass. 0.50 ml deionized water and 2 drop Reagent B (5% α-napthol in 96% ethanol) were added. Fifteen drops of concentrated sulphuric acid were added to the mixture with ½ second between the drops resulting in heat development and a colour reaction was created. The glass was tilted while cooling down for 10-15 minutes. A stick (1cm x 4cm Adventec filter no. 526, where 3 cm was impregnated with paraffin) with the part without paraffin was sticked into the solvent in glass no. 1 for about 5 seconds. The colour was evaluated (within 5-15 minutes) by comparing the colour of the stick with a PANTONE^{®}-formula guide uncoated scale and the Pantone number was noted. For the analysis in the laboratory the sticks were placed in cardboard shape and analysed using CAMAG Video-Store box as described above.

### Stick C

Three drops of filtered extract were added into a plastic glass and 3 drops of Reagent C (2.5% 2-aminoethyl-diphenylborinate in 96% ethanol) were added and the reaction was allowed to develop for 2 minutes. A stick (1cm x 4cm Adventec filter no. 526, where 3 cm was impregnated with paraffin) and with the part without paraffin was put into the solvent in the glass for about 5 seconds. The colour of the reaction was evaluated (within 5-15 minutes) by comparing the colour of the stick with a PANTONE⁶-formula guide uncoated scale and the Pantone number was noted. For the analysis in the laboratory the sticks were placed in cardboard shape and analysed using CAMAG Video-Store box as described above.

### Results

In figure 1-8 the connection between the PANTONE^{®}- colours and the effect on the biomass due to herbicide treatment (untreated = 0%) are presented. For each PANTONE^{®}- colour the number and the colour intensity was observed and the colour intensity high was calculated using the mean values +/- standard deviation of the curves from the semi-field studies no. 927/05 and 928/05. These values were used for calculation of the curves in the other studies and field studies 2005 & 2006.

The colours on both stick types (A and B) varies from light to dark colour in respect to increased doses and increased effect on the reduction in biomass. For stick-type A the colour and intensity varied from light beige /red violet via light blue violet to dark violet. For stick-type B the colours varied from green via gray to very dark blue for all plant species exposed to the herbicides. The most sensitive plant species to the herbicides, presented the colour pattern as short as 4 days after exposure, whereas the less sensitive plant species either did not shown as powerful colour changes or the pattern was not present before 7 days after exposure. In the appendix 2 to 8 all the detailed information concerning plant species, herbicide doses, visual effects or effect on biomass (fresh-and dry weight) are presented.

The PANTONE^{®}-colour for both stick-type A and B can be divided into three levels concerning different amount of reduced growth (biomass) of the weed grasses based on e.g. the results shown in table 7 to 10.

In the following the results of the different studies are presented in graphs. The doses are presented on the X-axis as parts of the normal doses (1 N) where full effect is seen under the cultivation conditions. The normal doses for each plant species and herbicide are presented elsewhere herein. The effect of the biomass is presented as graphs to be read on the right y-axis. The effect presents percent (%) reduction of fresh- and dry weight of plants in relation to untreated plants 21 days after exposure. In the figures from semi-field studies (figure 1-5) two columns are presenting different test results in respect of the plant species, the high of the columns indicate the colour intensity where the scale is presented in the left y-axis. The left column represents the results of stick A and the right column represents the results of stick B. The colours of the columns indicate the PANTONE^{®} - colour observed. The PANTONE^{®}- colour numbers also appears from the Tables. All parameters are analysed for 3 replicates and the standard deviation are presented on the columns. In the figures with results of the field studies 4 columns are presented for each dose - the first 2 columns shows the results of stick A after autumn- and spring treatment, respectively while the 3th and the 4th columns shows the results of stick B for autumn- and spring squirting.

### Semi-field study with dose/response (927/05 & 928/05)

In these studies the correlation between colour and biomass reduction were investigated. The results showed that for certain plant species and herbicides the correlation between colour and biomass reduction can be detected as early as 4 days after exposure. As presented in figure 1 for analysis 4 and 7 days after squirted with Atlantis and Hussar a significant response in relation to untreated plants for the colour intensity in treatments with more than 80% effect on the reduction in biomass for both stick A and B were seen. Figure 1 showed colour distinction, intensity and effect on biomass for treatments with Lexus and Monitor.

The results indicate that stick B in certain cases may be preferred. A combination of the results form the sticks A and B may also be preferred.

### Semi-field study with rain stability (972/04 & 946/06)

The semi-field study with rain stability (972/04 and 946/06) was conducted to investigate the connection between the colour of the sticks and the reduction in biomasss before and after rain with and without herbicide exposure. A large scatter for effect on the biomass reduction using herbicides without and with Additive (adhesive), and at rain treatment at different times after exposure were seen. In study no. 972/04 the plant material was freeze-dried and following analysed, while in study no. 946/06 fresh frozen plant material was analysed.

The results in figure 2 show after treatment with Hussar and rain on perennial ryegrass where a significant response on the colour intensity of stick A and B were observed. The PANTONE^{®} colour indicate a high reduced effect. Only in one case stick B indicate a false positive response on the colour intensity (no Additive, rain after 4 hours), but the PANTONE^{®} colour has a sure response. The stick test in this study was only performed 4 days after treating the plants with the herbicide. Thus the test can be performed also when the herbicide is added an additive or it becomes rain after the plants are treated with the herbicide.

The results in figure 3 show that a significant result was obtained for colour intensity down to 50% effect on the biomass reduction. For analysis 7 days after squirting for stick B a good correlation between the doses where the curv is flatten and where more than 80% effcct in biomass reduction is obtained.

### Semi-field study with mixtures of herbicides (945/06)

In these studies the goal was to investigate the mixture of Hussar with other herbicides and their effects both phytochemical and physiological. The results show a good correlation between the colour on sticks and final effect as a reduction in biomass for Hussar alone and in mixture with Starane and Express. A significant response was observed for colour intensity of the sticks indicating that the herbicide treatments had a final effect of more than 80% reduction in biomass. There was no false positive response with PANTONE^{®} colours. Mixture of Hussar with the herbicide Starane gives the same result as for Hussar alone, whereas mixtures of Hussar with the herbicide Express increased the final effect on the biomass and also the colour intensity on the sticks increased. The herbicide Express is like Hussar and Atlantis a sulfonylurea-herbicide. Express has an effect for dicotyledon weed plants but also some effect to perennial ryegrass. The result observed is therefore not a surprise. There is a good correlation between a colour reaction and the final effect 21 days after exposure.

### Field studies

### Field studies in winter crops at Hobro and Sealand 2005/2006 (including study no. 917/06)

The field study in autumn 2005 and spring 2006 support the results observed in the semi-field studies. The analyses were conducted for fresh harvest plant material directly in the fields. The results are presented in figure 5 to 7.

For loose silky-bent grass the doses were too high and therefore no differentiation between effect and colour on the sticks were detected. For these field studies, the results indicated that performing the test 4 days after exposure need not give a proper result, performing the test 5, 6 or 7 days after exposure may be needed. In certain cases an effect was detected according the colours of the sticks in the untreated parcels (perennial ryegrass, Seal and, autumn 2005). A pollution from lateral parcels as drift could explain the low effect values which are calculated relatively the control plants and sticks colour. Supplementary analyses from fields without herbicides (organic fields) were performed to ensure the field-values for the 0% effect colours in relation to the semi-field studies. These analyses showed the same results as for the semi-field studies.

At Sealand for perennial ryegrass a colour change in the PANTONE^{®} colour for the final effect of more than 80% effect on biomass reduction was in good agreement.

The development study with perennial ryegrass exposed to Hussar OD (948/06) was conducted to evaluate the sensitivity of the plant to the herbicide at different grow stages and correlate between sensitivity and colour reaction with the stick method. No large difference of the reaction (both colour of the sticks and the biomass reduction) were detected at the different growth stages 12, 30.2 and 32. The results were as seen before most distinct 7 days after exposure. In this study a tendency of the effect on the biomass reduction is underestimated according to the colour reaction of the stick. This means that the colour reaction on the sticks at the two highest herbicide doses of Hussar at the growth-stages 30.2 and 32 indicated an effect on the reduction of the biomass more than 80% effect.

### Summary for semi-field and field studies

In relation to the utility of the method in practice it is important that no false positive results are detected predicting the effect to be sufficient in cases where a supplementary squirting is needed. A false negative result is less important for the farmer but important for the environment.

**Table 7: Overview of the plant species, herbicides and PANTONE^{®}-colours for herbicide treatment with low final effect on the biomass reduction (0-39%).**

| **Plant species** | **Herbicide** | **Final effect (fresh weight)** | **Stick A 4 days PANTONE^{®} no.** | **Stick B 4 days PANTONE^{®} no.** | **Stick A 7 days PANTONE^{®} no.** | **Stick B 7 days PANTONE^{®} no.** | **Study no./ Comments** |
|---|---|---|---|---|---|---|---|
| Perennial ryegrass | Hussar | 0-12.8% | 663 | 5645 | - | - | 972/04 None/None |
| Perennial ryegrass | Hussar | 0-11.3% | 665, 666, 7446 | 5645. 416, 417, 418 | - | - | 972/04 None/1 hour |
| Perennial ryegrass | Hussar | 0-3.6% | 666, 667 | 5635 | - | - | 972/04 None/4 hours |
| Perennial ryegrass | Hussar | 0% | 664 | 5645 | - | - | 972/04 Additive/None |
| Perennial ryegrass | Hussar | 0% | 666 | 5645 | - | - | 972/04 Additive/1 hour |
| Perennial ryeqrass | Hussar | 0% | 665 | 5635 | - | - | 972/04 Additive/4 hours |
| Perennial ryegrass | Hussar | 0-16.2% | 7527, 664 | 5803, 7544 | - | - | 927/05 Dose/response |
| Perennial ryegrass | Hussar | 0-30.4% | 665, 666 | 535" | 665 | 535** | 945/06 No mixture |
| Perennial ryegrass | Hussar | 0-38.3%* | 5245 | 5655 | 664, 665 | 7544 | 945/06 0.5 l/ha Oxitril |
| Perennial ryegrass | Hussar | 0% | 665, 7445 | 535*,** | 665 | 7544 | 945/06 0.6 l/ha Starane |
| Perennial ryegrass | Hussar | 0* | 272* | 535* | 273* | 534* | 945/06 3.75 g a.i./ha Lexus |
| Perennial ryegrass | Hussar | 0-6.7% | 271, 272" | 534, 2758** | 7445 | 7544 | 948/06 Stadie 12 |
| Perennial ryegrass | Hussar | 0-25.7% | 7445, 665 | 7544 | 663, 665 | 7544, 7546 | 948/06 Stadie 30.2 |
| Perennial ryeqrass | Hussar | 0-21.7% | 665 | 7544, 7546 | 665 | 7544 | 948/06 Stadie 32 |
| Perennial ryegrass | Hussar | 0% | 664 | 7544 | 7444 | 7544 | Hobro 2005 |
| Perennial ryegrass | Hussar | 0% | 270 | 7545 | 270 | 534** | Sealand 2005 |
| Perennial ryegrass | Hussar | 0% | 7445 | 534** | 7445 | 7544 | Hobro 2006 |
| Perennial ryegrass | Hussar | 0% | 272** | 5793 | 272** | 7544 | Sealand 2006 |
| Perennial ryegrass | Atlantis | 0% | 664, 665 | 7543, 7544 | 7527, 664, 5235 | 5565, 5527, 7543 | 946/06 None/None |
| Perennial ryegrass | Atlantis | 0% | 664, 665 | 7543, 7544 | 7527 | 5565 | 946/06 None/1 hour |
| Perennial ryegrass | Atlantis | 0-23.5% | 664, 665 | 7543 | 7527, 664 | 5565(0%), 7543 (0%), 7544 (23.5%) | 946/06 None/3 timer |
| Perennial ryegrass | Atlantis | 0-29.0% | 665, 5235 | 7543 | 7527 | 7543 (0%) | 946/06 Additive/None |
| Perennial ryegrass | Atlantis | 0% | 664 | 7543 | 7527 | 5565 | 946/06 None/None |
| Perennial ryegrass | Atlantis | 0% | 664 | 7543 | 7527 | 5565 | 946/06 None/None |
| Perennial ryeqrass | Atlantis | 0% | 665 | 7544 | 665 | 7544 | Hobro 2005 |
| Perennial ryegrass | Atlantis | 0% | 270 | 7545 | 270 | 534** | Sealand 2005 |
| Perennial ryegrass | Atlantis | 0% | 7445 | 534" | 7445 | 7544 | Hobro 2006 |
| Perennial ryeqrass | Atlantis | 0% | 272** | 5793 | 272** | 7544 | Sealand 2006 |
| Perennial ryeqrass | Atlantis | 0% | 273** | 7544 | 273** | 7544 | 917/06 Field study |
| Loose silkybent grass | Hussar | 0% | 270 | 5517 | 7437-7439 | 7542 | 927/05 Dose/response |
| Loose silkybent grass | Hussar | 0% | 272** | 534** | 273** | 534** | Sealand 2005 |
| Loose silkybent grass | Hussar | 0% | 7446 | 535" | 7446 | 5517 | Sealand 2006 |
| Loose silkybent grass | Atlantis | 0% | 270 | 5517 | 7437-7439 | 7542 | 927/05 Dose/response |
| Loose silky-bent grass | Atlantis | 0% | 272** | 7545-533** | 2745** | 534** | Sealand 2005 |
| Loose silky-bent grass | Atlantis | 0% | 7446 | 535** | 7446 | 5517 | Sealand 2006 |
| Loose silkybent grass | Monitor | 0% | 664 | 5517 | 7527 | 5665 | 928/05 Dose/response |
| Loose silky-bent grass | Lexus | 0-39.4% | 664 (0%) | 7542 (0-39.6%) | 663, 7527 | 5803(0%) | 928/05 Dose/response |
| Annual meadow grass | Hussar | 0% | 664 | 7544 | 7444 | 7544 | Hobro 2005 |
| Annual meadow grass | Hussar | 0% | 7445 | 534" | 665 | 7543 | Hobro 2006 |
| Annual meadow grass | Atlantis | 0% | 7527 | 7544 | 7527 | 7544 | Hobro 2005 |
| Annual meadow grass | Atlantis | 0% | 7445 | 534** | 665 | 7543 | Hobro 2006 |
| Annual meadow grass | Atlantis | 0% | 7527 | 5783-5645 | - | - | 927/05 Dose/response |
| Lobgrass | Monitor | 0% | 7527 | 536 | 7527 | 5803 | 928/05 Dose/response |
| Agerraeve-hale | Lexus | 0% | 664 | 536 | 665 | 5665 | 928/05 Dose/response |

| | | | | | | | |
|---|---|---|---|---|---|---|---|
| *Oxitril, Starane and Lexus have an effect, which is seen in the "o-value", **Unexplainable high values | | | | | | | |

**Tabel 8a: Overview of plant species, herbicides, PANTONE^{®}-colour for herbicide treatments with medium final effekt on the biomass reduction (4085%).**

| **Plant species** | **Herbicide** | **Final effect (fresh weight)** | **Stick A 4 days PANTONE^{®} no.** | **Stick B 4 days PANTONE^{®} no.** | **Stick A 7 days PANTONE^{®} no.** | **Stick B 7 days PANTONE^{®} no.** | **Study no./ Comments** |
|---|---|---|---|---|---|---|---|
| Perennial ryegrass | Hussar | 33.6-61.8% | 665. 666 | 443 (33.6%) 7545, 7546 (61,884.3%) | - | - | 972/04 None/None |
| Perennial ryegrass | Hussar | 57.2% | 272 | 7545 | - | - | 972/04 None/1 hour |
| Perennial ryegrass | Hussar | 24-78.9% | 273 | 5487 (24%), 7545 (51.5%) , 534 (78.9%) | - | - | 972/04 None/4 hours |
| Perennial ryegrass | Hussar | 52.2% | 273 | 7545 (52.2-85.1%) | - | - | 972/04 Additive/None |
| Perennial ryegrass | Hussar | 36.1% | 7446 273 (59.5-88.0%) | 5625 (36.1%) 7545 (59.5%) | - | - | 972/04 Additive/1 hour |
| Perennial ryegrass | Hussar | 61.5-77.0% | 272 | 7545 | - | - | 972/04 Additive/4 hours |
| Peren-nial ryegrass | Hussar | 68.8% | 7446 272(>7 5.3%) | 535 (68.8-75.3%) | - | - | 927/05 Dose/response |
| Perennial ryegrass | Hussar | 64.7% | 7445 (64.7-94.9%) | 534 | 272 | 534 | 945/06 No mixture |
| Perennial ryegrass | Hussar | 79.0-90.4% | 663, 7445 | 5517 | 665, 666 | 5645 | 945/06 0,5 l/ha Oxitril |
| Perennial ryegrass | Hussar | 79.2% | 7445 | 534* | 666 | 2766* (>79.4%) | 945/06 0,6 l/ha Starane |
| Perennial ryegrass | Hussar | 83,7% | 272 | 535 | 273 | 2766* (>83.7%) | 945/06 3,75 g a.i./ha Lexus |
| Perennial ryegrass | Hussar | 40-86.7% | 273 (>40%) | 534,27 5b (>40%) | 7445 | 534 (40%) 2758 (86.7%) | 948/06 Stadie 12 |
| Perennial ryegrass | Hussar | 85% | 667 | 7546 | 7445 | 274 | 948/06 Stadie 30,2 |
| Perennial ryegrass | Hussar | 50-86.7% | 7445 (50%) | 7545 (>50%) | 272 (50%) | 7546 (86.7%) | 948/06 Stadie 32 |
| Perennial ryegrass | Hussar | - | - | - | - | - | Hobro 2005 |
| Perennial ryegrass | Hussar | 41,3-76.3% | 271 (>4 1.3%) | 7545 | 666, 272 | 534 (>41.3%) | Sealand 2005 |
| Perennial ryegrass | Hussar | - | - | - | - | - | Hobro 2006 |
| Perennial ryegrass | Hussar | 53.8% | 273 (>53.8 %) | 443 (53.8%) | 2745 | 7545 | Sealand 2006 |
| Perennial ryegrass | Atlantis | 31.6-78% | 666 | 535 | 664 (31.6%) 666 (78%) | 7544 (31.6%) 7545 (78%) | 946/06 None/None |
| Perennial ryegrass | Atlantis | 28.4-64.9% | 665 (>28.4 %) | 536(28. 4%) 535 (64.9%) | 5245 (28.4%) 665 (64.9%) | 7543 (28.4%) 535 (>64.9%) | 946/06 None/1 hour |
| Perennial ryeq rass | Atlantis | 72.8% | 666 | 534 | 5235 | 535 (>72.8%) | 946/06 None/3 timer |
| Perennial ryegrass | Atlantis | 55.7% | 665 (>55.7 %) | 535 | 5235 | 535 (>29.0%) | 946/06 Additive/None |
| Perennial ryeqrass | Atlantis | 56.2% | 665 | 535 (>56,2 %) | 5235 | 535 (>56.2%) | 946/06 None/1time |
| Perennial ryegrass | Atlantis | 71.9-89.3% | 665 | 535 (>71.9 %) | 5235 | 535 (>71.9%) | 946/06 None/3 timer |
| Perennial ryegrass | Atlantis | - | - | - | - | - | Hobro 2005 |
| Perennial ryegrass | Atlantis | 35-70% | 666 (35%) 7446 (43.8%) | 7545 (35%) | 271 | 535 | Sealand 2005 |
| Perennial ryegrass | Atlantis | - | - | - | - | - | Hobro 2006 |
| Perennial ryegrass | Atlantis | 48.3-62.5% | 273 | 443 (>48.3 %) | 2745 | 444 (48.3%) 7545 (62.5%)_{.} | Sealand 2006 |
| Perennial ryegrass | Atlantis | 7.5-75.0% | 667 (7.5-20%) 273 (75.0%) | 7545 | 667 (7.5%) 273 (20-75%) | 7544, 7545 | 917/06 Field study |
| Loose silkybent grass | Hussar | 61.9-81% | 272 | 443 (61,9%) 7545 (81%) | 7439 (61,9%) 7440(> 81%) | 7542 (61.9%) 7545,535 (>81%) | 927/05 Dose/response |
| Loose silky-bent grass | Hussar | >60% | 273 | 534 | 273 | 534 | Sealand 2005 |
| Loose silky-bent grass | Hussar | - | - | - | - | - | Sealand 2006 |
| Loose silkybent grass | Atlantis | 45.9-68% | 271, 272 (45,9-48.5%) 273 (>68%) | 443 (45.9%) 7544 | 7439, 7440, 260 | 7545, 535 | 927/05 Dose/response |
| Loose silky-bent grass | Atlantis | - | - | - | - | - | Sealand 2005 |
| Loose silky-bent grass | Atlantis | - | - | - | - | - | Sealand 2006 |
| Loose silky-bent grass | Monitor | 41,9-73.6% | 665, 7445. 7446 | 7544, 535 | 5245 | 536, 535 | 928/05 Dose/response |
| Loose silkybent grass | Lexus | 29.1-69.9% | 663 271(>6 9.9%) | 535 (>69.9 %) | 664, 665 | 5655 (29.1%) 7542 (39.4%) 535 (69.9%) | 928/05 Dose/response |
| Annual meadow grass | Hussar | 37.7% | 272 | 7544 (37,7-46.8%) | 272 | 535 | Hobro 2005 |
| Annual meadow grass | Hussar | 41.2-58.2% | 273 | 2766 | 273 | 2756 | Hobro 2006 |
| Annual meadow grass | Atlantis | 29-58.8% | 272 | 7545 (29-43.7%) | 273 | 534 (>29%) | Hobro 2005 |
| Annual meadow grass | Atlantis | 56.4-77.1% | 272, 273 | 534 | 2745, 2755 | 2756 | Hobro 2006 |
| Annual meadow grass | Atlantis | 61.9% | 665 | 443, 5645 | - | - | 927/05 Dose/response |
| Lobgrass | Monitor | 49.4-83.7% | 7527 (>0%) | 535 | 7527, 5245 | 535 | 928/05 Dose/response |
| Foxtail grass | Lexus | >83.8% | 270,27 1,272 | 535, 534 (>96.4 %) | 7444, 7445, 270, 271 | 534,2766 | 928/05 Dose/response |

| | | | | | | | |
|---|---|---|---|---|---|---|---|
| *Oxitril, Starane and Lexus have an effect, which is seen in the "o-value", **Unexplainable high values | | | | | | | |

**Table 8b: Overview of plant species, herbicides, PANTONE^{®}-colours for treatment with high final effect on the biomass (>85%)**

| **Plant species** | **Herbicide** | **Final effect (fresh weight)** | **Stick A 4 days PAN-TONE^{®} no.** | **Stick B 4 days PAN-TONE^{®} no.** | **Stick A 7 days PAN-TONE^{®} no.** | **Stick B 7 days PAN-TONE^{®} no.** | **Study no./ Comments** |
|---|---|---|---|---|---|---|---|
| Perennial ryegras s | Hussar | >89.3% | 273 | 534 (>88.8 %) | - | - | 972/04 None/None |
| Perennial ryegras s | Hussar | >79.9% | 273 | 534 | - | - | 972/04 None/1 hour |
| Perennial ryegras s | Hussar | 24-78.9% | 273 | 534 (>78.9 %) | - | - | 972/04 None/4 hours |
| Perennial ryegras s | Hussar | >89.5 % | 274 (>85.1 %) | 534 | | | 972/04 Additive/None |
| Perennial ryegras s | Hussar | > 86,8% | 2755 | 534 (>77.2 %) | | | 972/04 Additive/1 hour |
| Perennial ryegras s | Hussar | >82.6% | 273 | 534 | | | 972/04 Additive/4 hours |
| Perennial ryegras s | Hussar | >98.1% | 273 | 534 (>91.2 %) | | | 927/05 Dose/response |
| Perennial ryegras s | Hussar | >94.9% | 666 (>96.1 %) | 2766 | 668, 273 | 2766 | 945/06 No mixture |
| Perennial ryegras s | Hussar | >95.3% | 665 | 7443 | 667 | 444, 7545 | 945/06 0,5 l/ha Oxitril |
| Perennial ryeg ras s | Hussar | >96.1% | 667 (>79.2%) | 2766 | 7447, 668 | 2766 (>79.4%) | 945/06 0,6 l/ha Starane |
| Perennial ryegras s | Hussar | >92.5% | 273 | 534, 2766 | 274 | 2766 | 945/06 3,75 g a.i./ha Lexus |
| Perennial ryegras s | Hussar | >97% | 273 (>40%) | 2766 | 2755 | 2765 | 948/06 Stadie 12 |
| Perennial ryegras s | Hussar | >98.7% | 273 | 534 | 666 | 2766 | 948/06 Stadie 30,2 |
| Perennial ryegras s | Hussar | >86.7% | 666 | 7545 (>50%) | 667 | 7545, 7546 | 948/06 Stadie 32 |
| Perennial ryegras s | Hussar | - | - | - | - | - | Hobro 2005 |
| Perennial ryegras s | Hussar | >95.3% | 272 (>76.3%) | 7546 | 274 | 534 (>41.3%) | Sealand 2005 |
| Perennial ryegras s | Hussar | - | - | - | - | - | Hobro 2006 |
| Perennial ryegras s | Hussar | >96.0% | 2755 | 7545 | 2765 | 533 | Sealand 2006 |
| Perennial ryegras s | Atlantis | >95.4% | 665, 7445 | 534 | 667 | 534,2766 | 946/06 None/None |
| Perennial ryegras s | Atlantis | >96.8% | 665 (>28.4%) | 534 (>89.0 %) | 666 (>89.0 %) 273 | 535 (>64.9%) | 946/06 None/1 hour |
| Perennial ryegras s | Atlantis | >95.7% | 665, 5235 | 534 | 666, 667 | 534 | 946/06 None/3 timer |
| Perennial ryegras s | Atlantis | >94.2% | 665, 7445 | 534 | 666, 667 | 534 | 946/06 Additive/None |
| Perennial ryegras s | Atlantis | >93.3% | 665 (>56.2%) | 534 | 666 | 535 (>79.4%) | 946/06 None/1 hour |
| Perennial ryeg ras s | Atlantis | >96.4% | 666 | 534 | 666, 667 | 534 | 946/06 None/3 timer |
| Perennial ryegras s | Atlantis | - | - | - | - | - | Hobro 2005 |
| Perennial ryeg ras s | Atlantis | >43.8% | 7446 | 272 | 7545 | 534 | Sealand 2005 |
| Perennial ryegras s | Atlantis | - | - | - | - | - | Hobro 2006 |
| Perennial ryegras s | Atlantis | >91.5% | 273 (>48.1 %) | 443 (>48.1 %) | 2755 (>62.5 %) | 533 | Sealand 2006 |
| Perennial ryegras s | Atlantis | >97.3% | 274 | 534 | 274 | 534 | 917/06 Field study |
| Loose silky-bent qrass | Hussar | >88.8% | 273 | 534 | 260, 261, 262 | 655 | 927/05 Dose/response |
| Loose silkybent grass | Hussar | >60% | 273 | 534 | 273 | 534 | Sealand 2005 |
| Loose silkybent grass | Hussar | >93.3% | 272, 2745 | 2756, 2766 | 273 | 534 | Sealand 2006 |
| Loose silkybent grass | Atlantis | >83.9% | 2745, 2755 | 535 | 262 | 655 | 927/05 Dose/response |
| Loose silky-bent grass | Atlantis | >90% | 273 | 533 | 2745, 2755 | 534, 2768 | Sealand 2005 |
| Loose silkybent grass | Atlantis | >96% | 2745 | 534 | 2745 | 534 | Sealand 2006 |
| Loose silky-bent grass | Monitor | >88.4% | 272,273 | 534 | 664, 665 | 534, 2766 | 928/05 Dose/response |
| Loose silkybent grass | Lexus | >88.7% | 272 | 535 (>69.9 %) | 272 | 534, 2766 | 928/05 Dose/response |
| Annual meadow grass | Hussar | >46.8% | 273 | 7545 | 273 | 534 | Hobro 2005 |
| Annual meadow grass | Hussar | >74.7% | 273 (>58.2%) | 2766 (>58.2 %) | 2745 | 2756 (>58.2%) | Hobro 2006 |
| Annual meadow grass | Atlantis | >58.8% | 272 (>29%) | 535 | 273 (>29%) | 534 (>29%) | Hobro 2005 |
| Annual meadow grass | Atlantis | >56.4% | 272, 273 | 534 | 2745, 2755 | 2756 | Hobro 2006 |
| Annual meadow grass | Atlantis | >94.3% | 273 | 534 | - | - | 927/05 Dose/response |
| Lobgrass | Monitor | >90.5% | 7527 (>0%) | 534 | 5245 | 534, 2766 | 928/05 Dose/response |
| Foxtail grass | Lexus | >83.8% | 270, 271, 272 | 535, 534 | 7444, 7445, | 534, 2766 | 928/05 Dose/response |
| | | | | (>96.4 %) | 270, 271 | | |

| | | | | | | | |
|---|---|---|---|---|---|---|---|
| *Oxitril, Starane and Lexus have an effect, which is seen in the "o-value", **Unexplainable high values | | | | | | | |

**Table 9: Overview of plant species, herbicides, PANTONE^{®}-colours for treatment with high final effect on the biomass.**

| **Plant species** | **Herbicide** | **Final effect (fresh weight)** | **Stick A 4 days PANTONE^{®} no.** | **Stick B 4 days PANTONE^{®} no.** | **Stick A 7 days PANTONE^{®} no.** | **Stick B 7 days PANTONE^{®} no.** |
|---|---|---|---|---|---|---|
| Perennial ryegrass | - | 0% | 664, 665 | 7543, 7544 | 7527, 665 | 7543, 7544, 5565 |
| Perennial ryegrass | Hussar | 41-87% | 7445, 7446, 271, 272, 273 | 443, 7545, 535, 534 | 7445,2 72, 666 | 7545, 7546, 534 |
| Perennial ryegrass | Hussar | >96% | 2755 | 2766 | 2755 | 2766 |
| Perennial ryegrass | Atlantis | 32-78% | 666, 667, 7446, 273 | 443, 7545, 535 | 666, 271, 273 | 7545, 535 |
| Perennial ryegrass | Atlantis | >97% | 274 | 534 | 2755 | 2766 |
| Loose silky-bent grass | - | 0% | 7446, 664, 270 | 5517, 7542, 7545 | 7527, 7446, 7439 | 5517, 7542 |
| Loose silky-bent grass | Hussar | 60-81% | 272, 273 | 443, 7545, 534 | 7440,273 | 7545, 535, 534 |
| Loose silky-bent grass | Hussar | >93% | 2745 | 2766 | 273 | 655 |
| Loose silky-bent grass | Atlantis | 46-68% | 271, 272, 273 | 443, 7544 | 7440, 260 | 7545, 535 |
| Loose silky-bent grass | Atlantis | >90% | 2755 | 534 | 2755 | 2768 |
| Loose silky-bent grass | Monitor | 42-74% | 665, 7445, 7446 | 7544, 535 | 5245 | 536,535 |
| Loose silky-bent grass | Monitor | >88% | 273 | 534 | 665 | 2766 |
| Loose silky-bent grass | Lexus | 29-70% | 663, 271 | 535 | 664, 665 | 5655, 7542, 535 |
| Loose silky-bent grass | Lexus | >89% | 272 | 535 | 272 | 2766 |
| Annual meadow grass | - | 0% | 664, 7445, 7527 | 5783, 5645, 7544 | 7527, 665, 7444 | 7543, 7544 |
| Annual meadow grass | Hussar | 38-58% | 272, 273 | 7544 | 272, 273 | 535 |
| Annual meadow grass | Hussar | >58% | 273 | 2766 | 2745 | 2756 |
| Annual meadow grass | Atlantis | 29-77% | 665, 272, 273 | 443, 7545, 534 | 273 | 534 |
| Annual meadow grass | Atlantis | >56% | 273 | 534 | 2755 | 2756 |
| Lobgrass | - | 0% | 7527 | 536 | 7527 | 5803 |
| Lobgrass | Monitor | >91% | 7527 | 534 | 5245 | 2766 |
| Lobqrass | Monitor | 49-83% | 7527 | 535 | 7527, 5245 | 535 |
| Foxtail grass | - | 0% | 664 | 536 | 665 | 5665 |
| Foxtail grass | Lexus | >84% | 270, 271, 272 | 535, 534 | 7444, 7445, 270, 271 | 534.2766 |

For all the results of stick A the colours reactions are from light violet for the untreated plants to dark violet at high reduction in biomass effects due to the herbicide treatment. This result is correspondingly observed with stick B, where the colours vary from green/light gray for the untreated plants to dark gray and finally dark blue for high effects of the herbicide treatment. Margin colours can be used for the test kit to ensure proper responses for none and high effects on the biomass reduction. In very few cases a false positive result were observed. This was caused by pollution of herbicide from the neighbouring parcels since the parcels were very close to each other and thuys a drift of the herbicide was possible. The false positive tests were detected by the biomass results since these results were lower than expected. To support the colour reaction for the untreated plants, several extra tests with untreated plants were performed to ensure the colour reaction of 0%-values (non treated plants).

### The use of the test kit for other plants and herbicides

A Thin Layer Chromatography Screening performed with different plants (monotyle-dons and dicotyledons) indicated that at least the two phytochemical groups tested, represent general biomarkers in plants and thus can be used for a method of determining the effect of a herbicide treatment and for a test-kit based on these methods.

### Example 3

### Test-kit based on dicotyledon plants treated with herbicides

The two stick analysis (stick A and stick B) as described elsewhere herein, have also been tested on seed germinated plants and root germinated plants of dandelion, *Taraxacum vulgare* Weber exposed to the herbicide Roundup Bio.

### Plant and growth conditions

Seeds of dandelion were obtained from HerbiSeed U.K. Effects of the herbicide treatment were evaluated for sublethale doses. The plants were sown in 2L pots in a mixture of field soil, sand and sphagnum (2:1:1) (Weight per cent). The pots were placed in green-house. After germination the number of plants per pot was reduced to one. When the plants had 8-10 leaves, the above ground plant mass was cutted at the position where root germinated shots had developed. This was not dore for the seed germinated plants. The day before exposure, the pots were placed on an automatic watering table in the green-house. Here the weight of each pot was registered. The herbicide exposure was performed when the plants had 8 to 12 leaves.

### Herbicide

The herbicide used was: Roundup Bio, 360 g/L (Monsanto Crop Sciences Denmark A/S) (glyphosate 360 g/l).

The herbicides were applied in deionised water using a laboratory pot sprayer fitted with two ISO F-02-110 flat fan nozzles delivering a spray volume of 150 L per Ha with a pressure on 3 bar. Six doses of Roundup Bio (see table 6) were used. All treatments were repeated three times. The 15 replicates of each treatment were separated in 5 groups with three replicates per treatment. For the first 3 weeks after treatment, each week a plant group was harvested and fresh- and dry weight was measured in respect of each plant. A group of plants was used for seed production where mature seeds were selected weekly up to 4 months after treatment. The thousand-seed weight was calculated counting and weighing 100 seeds from each pot. One group of plants was used for stick A and B analysis 7 days after treatment.

Up to 3 weeks after treatment, weekly observations were performed of nondestructive measurements of visual effects (see table 2 for an overview of the score 0-9 indicating the visual effect of the treatment), water use and measurement of fluorescence were performed. The daily water use for each pot was registred, and the pots were automatically weighed several times a day and nutrients were added regulary. The fluorescence detactions (light-respons curves) were performed weekly with a HansaTech PAM. Before the measurements the plant were kept in a dark room for at least 1 hour to standadise the photosynthesis level in the plant in relation to sunshine- and overcast days. All measurements were performed on the same leaf of each plant.

### Results

In table 10 and 11 all the data of these investigations are presented. In Figure 8 and 9 the results of stick A and B are presented together with the reduction in biomasses 24 weeks after treatment. Compared with the monocotyledons, this is a very long term effekt 24 weeks = 168 days after treatment.

According to the reduction in biomasses three weeks after treatment, the prestressed plants (root germination stressed by cutting of the arial parts before treatment) are less sensitive to the herbicides. This was also confirmed testing the colour reaction for stick A and B.

The correlation of the colour of the two types of sticks A and B for the dicotyledon, *Taraxacum vulgare,* are in agreement with those seen for the monocotyledons. The colours are different for the two stick types when compared to the corresponding sticks obtained when testing herbicide treatment of monocotyledon plants. This is because there are different chemical compounds in dicotyledon plants and their reaction to stress is different than observed in monocotyledon plants. However, three different reaponse intervals due to herbicide treatment can be constructed as for the monocotyledons according to the level of the biomass reduction and the corresponding colour of the sticks.

For the root germinated plants (Figure 9) very little or no effect is seen for both biomass reduction 24 weeks after treatment and colour of stick A and B. This is in agreement with the results of the monocotyledons. Thus the figure and the results clearly show that the method and the stick can be used for testing the effect of the herbicide, as the colour obtained on the sticks indicated that there was no effect of the herbicide treatment which also was observed by the biomass evaluation.

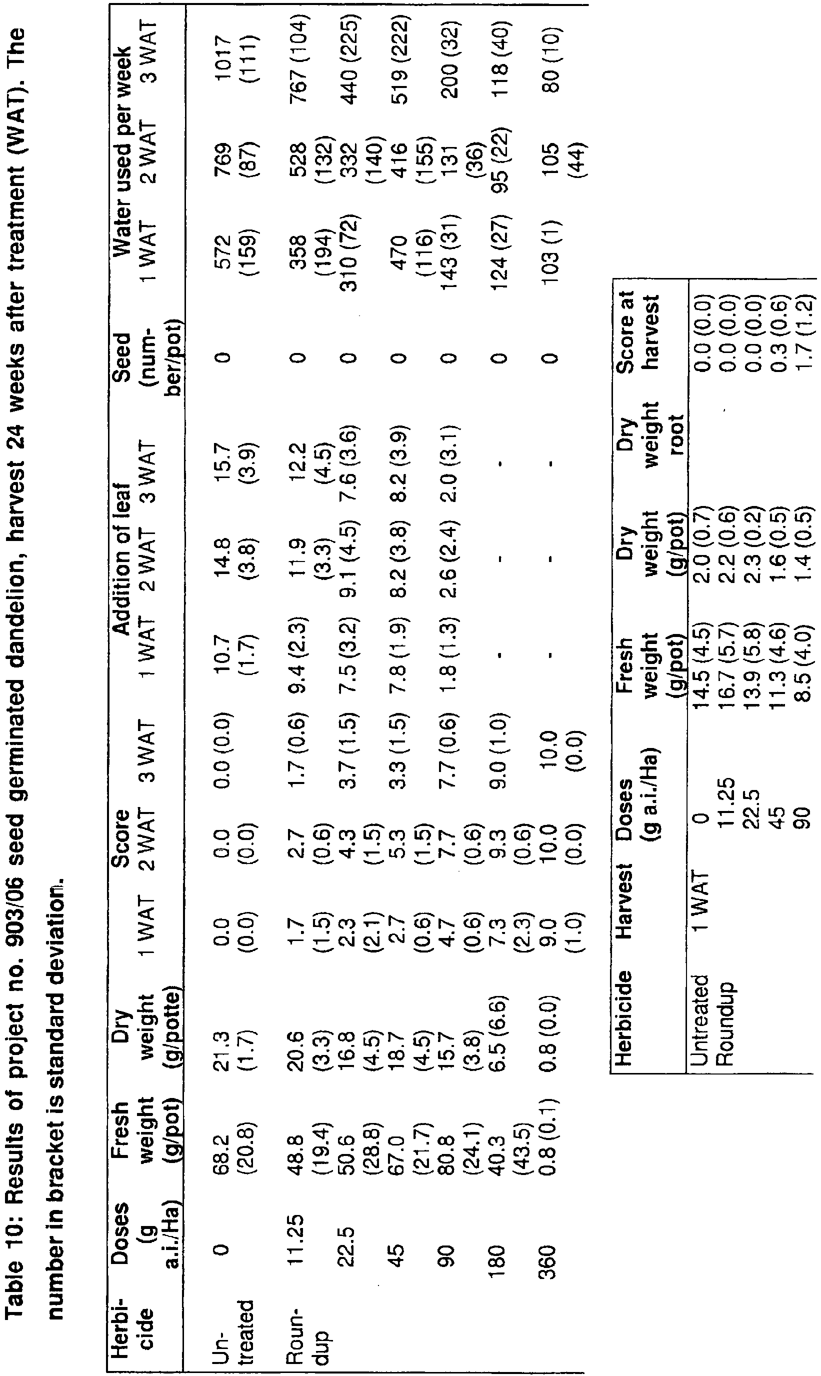

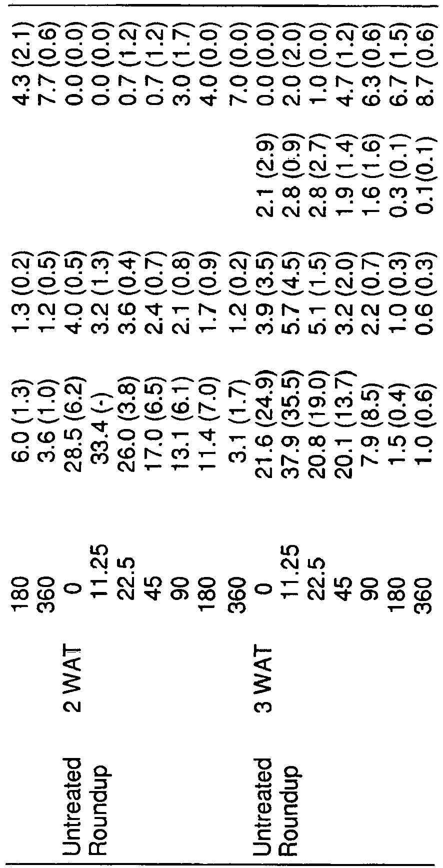

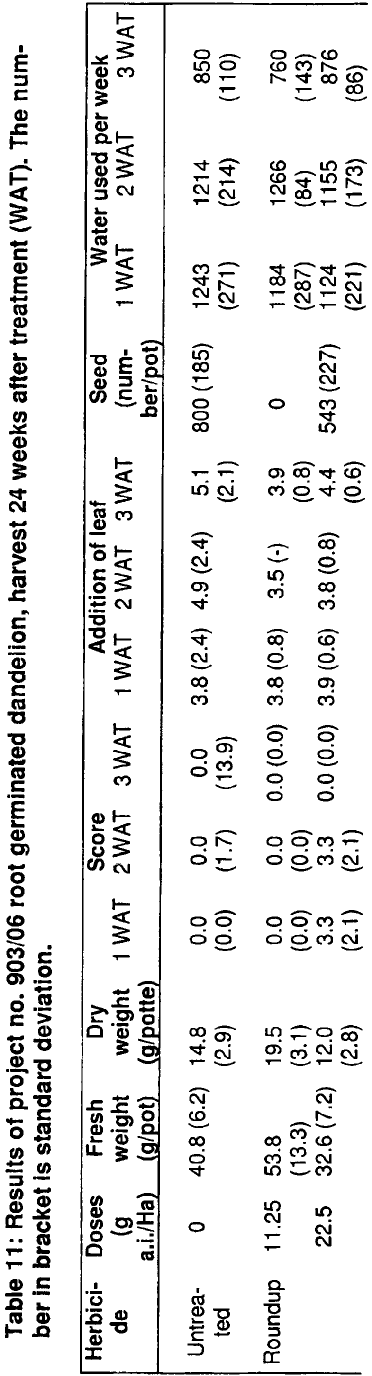

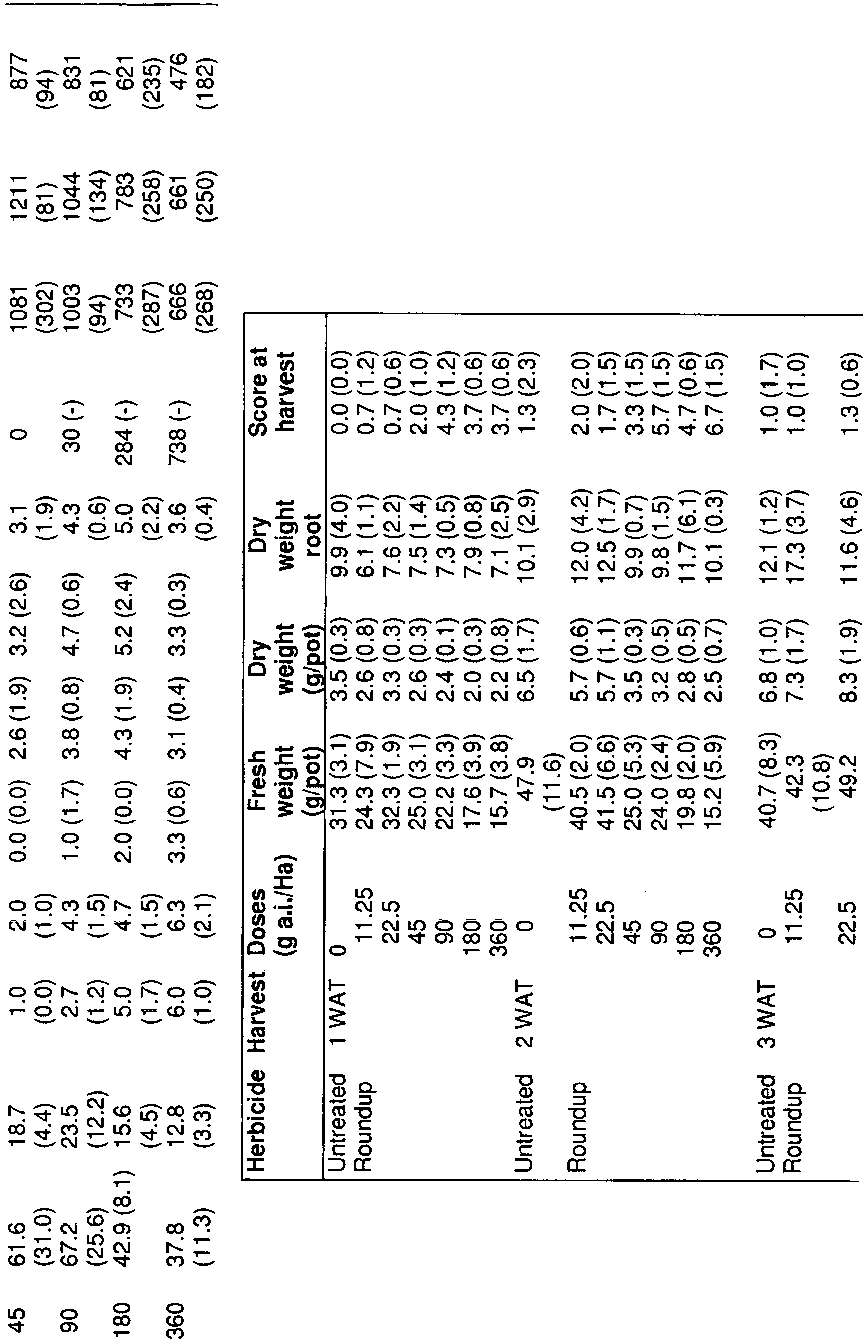

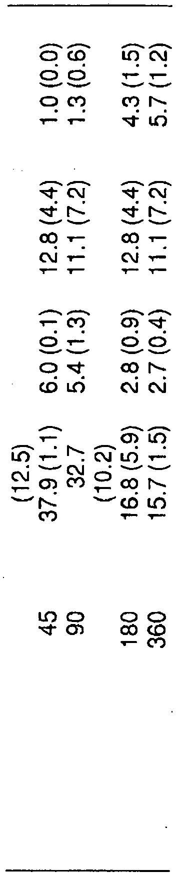

**Table 12 (A and B). 930/06 Semi-field study data to figure 8 and 9.**

| Table 12A. Semi-field study of seed germinated dandelion, *Taraxacum vulgare* | | | | | | | | | | | | | | | | | |
|---|---|---|---|---|---|---|---|---|---|---|---|---|---|---|---|---|---|
| Herbicide | Harvest | Doses | Dose | Fres h weig ht | % | 100-% | SDV | Dry weight | % | 100-% | SDV | Stick A | Stick A | Stick A | Stick B | Stick B | Stick B |
| | Days after exposure | Gram a.i./Ha | N= normal dose | After 24 week s FW g/pot | FW | FW | FW | After 24 weeks DW g/pot | DW | DW | DW | Pantone | Hight | SDW | Pantone | Hight | SDW |
| Roundup | 7 | 0 | 0 | 68.2 | 100 | 0 | 20.8 | 21.3 | 100 | 0 | 1.7 | No colour | 1786.4 | 181.6 | 5807 | 3277.0 | 416.4 |
| Roundup | 7 | 22.5 | jan-16 | 50.6 | 74.2 | 25.8 | 28.8 | 16.8 | 78.9 | 21.1 | 4.5 | 7500 | 1815.5 | 49.1 | 614 | 3156.0 | 496.1 |
| Roundup | 7 | 45 | 01-aug | 67 | 98.2 | 1.8 | 21.7 | 18.7 | 87.8 | 12.2 | 4.5 | No colour | 1727.0 | 208.0 | 454 | 3237.2 | 232.3 |
| Roundup | 7 | 90 | 01-apr | 80.8 | 118. 5 | 0 | 24.1 | 15.7 | 73.7 | 26.3 | 3.8 | 7500 | 2319.2 | 439.0 | 429 | 3779.3 | 1055.2 |
| Roundup | 7 | 180 | 01-feb | 40.3 | 59.1 | 40.9 | 43.5 | 6.5 | 30.5 | 69.5 | 6.6 | 7504 | 3747.2 | 630.8 | 429 | 4426.5 | 379.4 |
| Roundup | 7 | 360 | 1 | 0.8 | 1.2 | 98.8 | 0.1 | 0.8 | 3.8 | 96.2 | 0 | 4695 | 4012.0 | 638.7 | 415 | 3865.2 | 518.1 |

**Table 12B. Semi-field study of root germinated dandelion, Taraxacum vulgare**

| Herbicide | Harvest | Doses | Dose | Fresh weight | % | 100-% | SDV | Dry weight | % | 100-% | SDV | Stick A | Stick A | Stick A | Stick B | Stick B | Stick B |
|---|---|---|---|---|---|---|---|---|---|---|---|---|---|---|---|---|---|
| | Days after exposure | Gram a.i./Ha | N= normal dose | After 24 weeks FW g/pot | FW | FW | FW | After 24 weeks DW g/pot | DW | DW | DW | Pantone | Hight | SDW | Pantone | Hight | SDW |
| Roundup | 7 | 0 | 0 | 40,8 | 100 | 0 | 6,2 | 14,8 | 100 | 0 | 2,9 | No colour | 2139,7 | 101,3 | 5315 | 3050,7 | 285,6 |
| Roundup | 7 | 22.5 | jan-16 | 32,6 | 79,9 | 20,1 | 7,2 | 12 | 81,1 | 18,9 | 2,8 | No colour | 2308,5 | 208,9 | 5315 | 2945,5 | 228,8 |
| Roundup | 7 | 45 | 01-aug | 61,6 | 151 | 0 | 31 | 18,7 | 126,4 | 0 | 4,4 | No colour | 2240,2 | 102,5 | 5305 | 2955,7 | 145 |
| Roundup | 7 | 90 | 01-apr | 67,2 | 164,7 | 0 | 25,6 | 23,5 | 158,8 | 0 | 12,2 | No colour | 2315,9 | 85,8 | 5315 | 3027,7 | 282 |
| Roundup | 7 | 180 | 01-feb | 42,9 | 105,1 | 0 | 8,1 | 15,6 | 105,4 | 0 | 4,5 | No colour | 2324,7 | 217,1 | 5305 | 3058,7 | 359,6 |
| Roundup | 7 | 360 | 1 | 37,8 | 92,6 | 7,4 | 11,3 | 12,8 | 86,5 | 13,5 | 3,3 | No colour | 2698,5 | 782,9 | 5295 | 3450,9 | 142,3 |

The conclusion is that the same colour pattern for stick A and B for the monocotyledons was detected for the dicotyledons. This indicates that this method can be used for dicotyledon plant species and other herbicides as e.g. Roundup Bio.

### References

Boutin, C.; Freemark, K.E. & Keddy, C.J. (1993): Proposed guidelines for registration of chemical pesticides: Nontarget plant testing and evaluation. Technical Report Series. No. 145. 1-91. Ottawa. Canadian Wildlife Service. Environment Canada.
Hamil, A.I.; Marriage, P.B. & Friesen, G. (1977): A method for assessing herbicide performance in small plot experiments. Weed Sciences. 25. 386-389.
Snyder, L.R. (1974). Journal of Chromatography A, 92, 2, 233-230.

**Table 13. Overview over plant material tested.**

| **Herbicide** | **Active ingredient (a.i.)** | **Abbreviations** | **Additives** | **Dose** | **Plant species** | **Plant species (Latin)** | **Stage** | **Comments** | **Data** |
|---|---|---|---|---|---|---|---|---|---|
| Boxer | Prosulfocarb | PRO | | 2400 g/Ha | Loose silky-bent grass | *Apera spicaventi* | 2-3 leaves | Lethal dose harvested twice (7 & 14 days) | Visual evaluation (7 days) |
| Boxer | Prosulfocarb | PRO | | 2400 g/Ha | Annual meadow grass | *Poa annua* | 2-3 leaves | Lethal dose harvested twice (7 & 14 days) | Visual evaluation (7 days) |
| Stomp 400 EC | Pendimethalin | PEN | | 1600 g/Ha | Loose silky-bent grass | *Apera spicaventi* | 2-3 leaves | Lethal dose harvested twice (7 & 14 days) | Visual evaluation (7 days) |
| Stomp 400 EC | Pendimethalin | PEN | | 1600 g/Ha | Annual meadow grass | *Poa annua* | 2-3 leaves | Lethal dose harvested twice (7 & 14 days) | Visual evaluation (7 days) |
| Primera Super | Fenoxaprop-p-ethyl | FEN | 0.2% Isoblette | 69 g/Ha | Loose silky-bent grass | *Apera spicaventi* | 4 leaves 2 offshoot | Lethal dose harvested twice (7 & 14 days) | Visual evaluation (7 days) |
| Primera Super | Fenoxaprop-p-ethyl | FEN | 0.2% Isoblette | 55.2 g/Ha | Foxtail grass | *Alopecurus myosuroides* | 5 leaves 1 offshoot | Lethal dose harvested twice (7 & 14 days) | Visual evaluation (7 days) |
| Hussar | lodosulfuron | IOD | 0.5 l/Ha Renol | 5 g/Ha | Loose silky-bent grass | *Apera spicaventi* | 4 leaves 2 offshoot | Lethal dose harvested twice (7 & 14 days) | Visual evaluation (7 days) |
| Hussar | lodosulfuron | IOD | 0.5 l/Ha Renol | 5 g/Ha | Perennial ryegrass | *Lolium perenne* | 4 leaves 1 offshoot | Lethal dose harvested twice (7 & 14 days) | Visual evaluation (7 days) |
| Monitor | Sulfosulfuron | SUL | 0.1% Lissapol Bio | 3.2 g/Ha | Loose silky-bent grass | *Apera spicaventi* | 4 leaves 2 offshoot | Lethal dose harvested twice (7 & 14 days) | visual evaluation (7 days) |
| Monitor | Sulfosulfuron | SUL | 0.1% Lissapol Bio | 6.4 g/Ha | Lobgrass | *Bromus hordeaceus* | 4 leaves 1 offshoot | Lethal dose harvested twice (7 & 14 days) | Visual evaluation (7 days) |
| Lexus | Flupyrsulfuron | FLU | 0.1% Lissapol Bio | 5 g/Ha | Foxtail grass | *Alopecurus myosuroides* | 5 leaves 1 offshoot | Lethal dose harvested twice (7 & 14 days) | Visual evaluation (7 days) |
| Topik | Clodinafob-propargyl | CLO | 0.5 l/Ha Renol | 30 g/Ha | Foxtail grass | *Alopecurus myosuroides* | 5 leaves 1 offshoot | Lethal dose harvested twice (7 & 14 days) | Visual evaluation (7 days) |
| Topik | Clodinafob-propargyl | CLO | 0.5 l/Ha Renol | 40 g/Ha | Perennial ryegrass | *Lolium perenne* | 4 leaves 1 offshoot | Lethal dose harvested twice (7 & 14 days) | Visual evaluation (7 days) |
| Roundup Bio | Glyphosat | GLY | | 360 g/Ha | Loose silky-bent grass | *Apera spicaventi* | 4 leaves 2 offshoot | Lethal dose harvested twice (7 & 14 days) | Visual evaluation (7 days) |
| Roundup Bio | Glyphosat | GLY | | 360 g/Ha | Foxtail grass | *Alopecurus myosuroides* | 5 leaves 1 offshoot | dose harvested twice (7 & 14 days) | Visual evaluation (7 days) |
| Roundup Bio | Glyphosat | GLY | | 360 q/Ha | Perennial ryegrass | *Lolium perenne* | 4 leaves 1 offshoot | Lethal dose harvested twice (7 & 14 days) | Visual evaluation (7 days) |
| Roundup Bio | Glyphosat | GLY | | 360 q/Ha | Lobgrass | *Bromus hordeaceus* | 4 leaves 1 offshoot | Lethal dose harvested twice (7 & 14 days) | Visual evaluation (7 days) |
| Roundup Bio | Glyphosat | GLY | | 360 g/Ha | Annual meadow grass | *Poa annua* | 4 leaves 1 offshoot | Lethal dose harvested twice (7 & 14 days) | Visual evaluation (7 days) |
| Roundup Bio | Glyphosat | GLY | | 90 g as/Ha | Perennial ryegrass | *Lolium perenne* | st. 21 | Harvested after 4. 7 and 14 days (treated and untreated) | |
| Boxer | Prosulfocarb | PRO | | 600g as/Ha | Loose silky-bent grass | *Apera spicaventi* | st. 12 | Harvested after 4. 7 and 14 days (treated and untreated) | |
| Monitor | Sulfosulfuron | SUL | 0.1% Lissapol Bio | 4 q as/Ha | Lobgrass | *Bromus hordeaceus* | st.13 | Harvested after 4. 7 and 14 days (treated and untreated) | |
| Hussar | lodosulfuron | IOD | 0.5 l/Ha Renol | 3 q as/Ha | Loose silky-bent grass | *Apera spicaventi* | st. 12-13 | Harvested after 4. 7 and 14 days (treated and untreated) | |
| Hussar | lodosulfuron | IOD | 0.5 l/Ha Renol | 3 g as/Ha | Loose silky-bent grass | *Apera spicaventi* | st. 22 | Harvested after 4. 7 and 14 days (treated and untreated) | |
| Roundup Bio | Glyphosat | GLY | | 30 g as/Ha | Loose silky-bent grass | *Apera spicaventi* | st. 21-22 | Harvested after 7 and 14 days (treated and untreated) | |
| Roundup Bio | Glyphosat | GLY | | 45 g as/Ha | Perennial ryegrass | *Lolium perenne* | st. 21-22 | Harvested after 7 and 14 days (treated and untreated) | |
| Boxer | Prosulfocarb | PRO | | 1600 g as/Ha | Loose silky-bent grass | *Apera spicaventi* | st. 21 | 5 harvests (1.3.7.14.21 days) and 5 doses (1/16N. 1/8N. 1/4N. 1/2N. 1 N and control | Biomass; Visual evaluation |
| Hussar | Iodosulfuron | IOD | 0.5 l/ha Renol | 3 g as/Ha | Loose silky-bent grass | *Apera spicaventi* | St. 21-22 | 5 harvests (1.3.7.14.21 days) and 5 doses (1/16N. 1/8N. 1/4N. 1/2N. 1 N and control | Biomass; Visual evaluation |
| Roundup Bio | Glyphosat | GLY | | 120 g as/Ha | Loose sil-ky-bent grass | *Apera spica-venti* | St. 21-22 | 5 harvests (1.3.7.14.21 days) and 5 doses (1/16N. 1/8N. 1/4N. 1/2N. 1 N and control | Biomass; Visual evaluation |
| Monitor | Sulfosulfuron | SUL | 0.1% Lissapol Bio | 16g as/Ha | Lobgrass | *Bromus hordeaceus* | st. 22 | 5 harvests (1.3.7.14.21 days) and 5 doses (1/16N. 1/8N. 1/4N. 1/2N. 1 N and control | Biomass; Visual evaluation |
| Boxer | Prosulfocarb | PRO | | 3200 g as/Ha | Loose silky-bent grass | *Apera spicaventi* | st. 22 | Harvested after 1 and 2 weeks | |
| Monitor | Sulfosulfuron | SUL | 0.1% Lissapol Bio | 4 g as/Ha | Lobqrass | *Bromus hordeaceus* | st. 22 | Harvested after 1 and 2 weeks | |
| Roundup Bio | Glyphosat | GLY | as/Ha | 180 g as/Ha | Perennial ryegrass | *Lolium perenne* | St. 22 | Harvested after 1 and 2 weeks | |
| Hussar | Iodosulfuron | IOD | 0.5 l/haRenol | 1 g as/Ha | Loose silky-bent grass | *Apera spicaventi* | st. 22 | Harvested after 1 and 2 weeks | |
| Roundup Bio | Glyphosat | GLY | | 360 g as/Ha | Perennial ryegrass | *Lolium perenne* | st. 24 | Squirting d.5/11. Harvested ?? | |
| Hussar | Iodosulfuron | IOD | 0.5 l/ha Renol | 200 g/Ha = 10g as/Ha | Loose silky-bent grass | *Apera spicaventi* | st. 23-24 | Squirting d.5/11. Harvested ?? | |
| Monitor | Sulfosulfuron | SUL | 0.1% Lissapol Bio | 20 g as/Ha | Lobgrass | *Bromus hordeaceus* | st. 23 | Squirting d.5/11. Harvested ?? | |
| Boxer | Prosulfocarb | PRO | | 2400 g as/ha | Loose silky-bent grass | *Apera spicaventi* | 3-4 leaves | Squirting d.5/11. Harvested ?? | |
| Hussar | lodosulfuron | IOD | 0.5 l/Ha Renol | 1 N= 0.05 q as/ha | Fuglegræs | *Stellaria media* | 4-6 leaves | 2 biotypes (sensitive and resistent)3 doses ubeh. 1 N and 3N Udtagn. 1.4.6.8.14 days) | |
| Primera Super | Fenoxaprop-p-ethyl | FEN | 0.2% Isoblette | 1 N=55.2 g as/ha | Foxtail grass | *Alopecurus myosuroides* | 3 leaves | 4 biotypes (sensitive. 2 resistant and 1 metabolic resistant) 3 doses untreated.. 1 N and 3N harvest 1.4.6.8.14 days) | |
| Hussar | Iodosulfuron | IOD | 0.5 l/Ha Renol | 1N=2.5 and 5 g as/ha | Loose silky-bent qrass | *Apera spicaventi* | 1) 3-4leaves 2) 6-8 leaves | 3 harvests (4.7.14 and final harvest =21) 5 doses: 1/16 N. 1/8 N. 1/4 N. 1/2 N. 1 N (three replicates) | Biomass; Visual evaluation |
| Hussar | Iodosulfuron | IOD | 0.5 l/Ha Renol | 1N = 2.5 and 5 g as/ha | Perennial ryegrass | *Lolium perenne* | 1)3-4leaves 2) 6-8 leaves | 3 harvests (4.7.14 and final harvest =21)5 doses: 1/16 N. 1/8 N. 1/4 N. 1/2 N. 1 N (three replicates) | Biomass; Visual evaluation |
| Hussar | Iodosulfuron | IOD | 0.5 l/Ha Renol | 1N = 2.5 and 5 g as/ha | Italiensk rajgræs | *Lolium multiflorum* | 1)3-4leaves 2) 6-8 leaves | harvests (4.7.14 and final harvest =21)5 doses: 1/16 N. 1/8 N. 1/4 N. 1/2 N. 1 N (three replicates) | Biomass; Visual evaluation |
| Hussar | Iodosulfuron | IOD | 0.5 l/Ha Renol | 1N = 5 and 10 g as/ha | Annual meadow grass | *Poa annua* | 1)3-4leaves 2) 6-8 leaves | 3 harvests (4.7.14 and final harvest =21) 5 doses: 1/16 N. 1/8 N. 1/4 N. 1/2 N. 1 N (three replicates) | Biomass; Visual evaluation |
| Primera Super | Fenoxaprop-p-ethyl | FEN | 0.2% Isoblette | 1N = 48.3 and 69 g as/ha | Loose silky-bent grass | *Apera spicaventi* | 1)3-4leaves 2) 6-8 leaves | 3 harvests (4.7.14 and final harvest = 25 / 21) 5 doses: 1/16 N. 1/8 N. 1/4 N. 1/2 N. 1 N (three replicates) | Biomass; Visual evaluation |
| Primera Super | Fenoxaprop-p-ethyl | FEN | 0.2% Isoblette | 1N = 48.3 and 69 g as/ha | Foxtail grass | *Alopecurus myosuroides* | 1)3-4leaves 2) 6-8 leaves | 3 harvests (4.7.14 and final harvest =25/21)5 doses: 1/16 N. 1/8 N. 1/4 N. 1/2 N. 1N (three replicates) | Biomass; Visual evaluation |
| Primera Super | Fenoxaprop-p-ethyl | FEN | 0.2% Isoblette | 1N = 48.3 and 69 g as/ha | Wild oat-grass | *A vena fatua* | 1)3-4leaves 2) 6-8 leaves | 3 harvests (4.7.14 and final harvest = 25/21) 5 doses: 1/16 N. 1/8 N. 1/4 N. 1/2 N. 1 N (three replicates) | Biomass; Visual evaluation |
| Hussar Atlantis | Iodosulfuron + mesosulfuron | IOD IOD+ME S | None/0.5 l/ha Renol 0.5 l/ha Renol | 1 N=10 g as/ha 1 N=0.72 g as/ha | Perennial ryegrass Loose silky-bent qrass | *Lolium perenne Apera spicaventi* | 3-4 leaves (2 L pots) 3-4 leaves (2 L pots) | 3 harvests. 3 rain treatments. +/-Additives. 1/32 N. 1/16 N. 1/8 N. 1/4 N. 1/2 N. 1 N (three replicates) 4 harvests (4.7.14 and final harvest = 21*) 7 doses: 0N, 1/32N. 1/16N. 1/8N. 1/4N. 1/2N. 1 N (three replicates a 3 pots) | Biomass Final biomass. Visual evaluation |
| Atlantis | Iodosulfuron + mesosulfuron | IOD+ME S | 0.5 l/ha Renol | 1 N=4.5 g as/ha | Annual meadow grass | *Poa annua* | 3-4 leaves (2 L pots) | 4 harvests (4.7.14 and final harvest = 21 *) 7 doses: 0N. 1/32N. 1/16N. 1/8N. 1/4N. 1/2N. 1 N (three replicates a 3 pots) | Final biomass. Visual evaluation |
| Hussar | Iodosulfuron | IOD | 0.5 l/ha Renol | 1 N= 5 g as/ha | Loose silky-bent grass | *Apera spicaventi* | 3-4 leaves (2 L pots) | 4 harvests (4.7.14 and final harvest = 21 *) 7 doses: 0N. 1/32N. 1/16N. 1/8N. 1/4N. 1/2N. 1 N (three replicates a 3 pots) | Final biomass. Visual evaluation |
| Hussar | Iodosulfuron | IOD | 0.5 l/ha Renol | 1 N= 2.5 g as/ha | Perennial ryegrass | *Lolium perenne* | 3-4 leaves (2 L pots) | 4 harvests (4.7.14 and final harvest = 21 *) 7 doses: 0N. 1/32N. 1/16N. 1/8N. 1/4N. 1/2N. 1N (three replicates a 3 pots) | Final biomass. Visual evaluation |
| Monitor | Sulfosulfuron | SUL | 0.1% Lissapol Bio | 1 N=8g as/ha | Loose silky-bent grass | *Apera spicaventi* | 3-4 leaves (2 L pots) | 4 harvests (4.7.14 and final harvest =21*) 7 doses: 0N. 1/32N. 1/16N. 1/8N. 1/4N. 1/2N. 1N (three replicates a 3 pots) | Final biomass. Visual evaluation |
| Monitor | Sulfosulfuron | SUL | 0.1% Lissapol Bio | 1 N= 10 g as/ha | Lobgrass | *Bromus hordeaceus* | 3-4 leaves (2 L pots) | 4 harvests (4.7.14 and final harvest = 21 *) 7 doses: 0N. 1/32N. 1/16N. 1/8N. 1/4N. 1/2N. 1N (three replicates a 3 pots) | Final biomass. Visual evaluation |
| Lexus | Flupyrsulfuron | FLU | 0.1% Lissapol Bio | 1N=10g a.i./ha | Loose silky-bent grass | *Apera spicaventi* | 3-4 leaves (2 L pots) | 4 harvests (4.7.14 and final harvest = 21 *) 7 doses: 0N. 1/32N. 1/16N. 1/8N. 1/4N. 1/2N. 1N (three replicates a 3 pots) | Final biomass. Visual evaluation |
| Lexus | Flupyrsulfuron | FLU | 0.1% Lissapol Bio | 1 N=8g as/ha | Foxtail grass | *Alopecurus myosuroides* | 3-4 leaves (2 L pots) | 4 harvests (4.7.14 and final harvest = 21 *) 7 doses: 0N. 1/32N. 1/16N. 1/8N. 1/4N. 1/2N. 1N (three replicates a 3 pots) | Final biomass. Visual evaluation |
| Hussar | Iodosulfuron | IOD | 0.5 l/ha Renol | | Perennial ryegrass | *Lolium perenne* | | | |
| Atlantis | Iodosulfuron+mesosulfu ron | IOD+ME S | 0.5 l/ha Renol | | Perennial ryegrass | *Lolium perenne* | | | |
| Hussar Atlantis | Iodosulfuron +mesosulfuron | IOD+ME S | 0.5 l/ha Renol | | Annual meadow grass | *Poa annua* | | | |
| Hussar | Iodosulfuron | IOD | 0.5 l/ha Renol | 1N=150 g/ha afterår. 200 g/ha forår | Perennial ryegrass | *Lolium perenne* | 1-2 leaves | Selected plants 4 and 7 days after squirting. 4 doses (0. 1/4N. 1/2 N and 1 N) | Final biomass. Visual evaluation 2 weeks after. Squirting) |
| Atlantis | Iodosulfuron+mesosulfu ron | IOD+ME S | 0.5 l/ha Renol | 1 N=200 g/ha afterår. 300 g/ha forår | Perennial ryegrass | *Lolium perenne* | | Selected plants 4 and 7 days after squirting. 4 doses (0. 1/4N. 1/2 N and 1 N) | |
| Hussar | Iodosulfuron | IOD | 0.5 l/ha Renol | 1N=100 g/ha afterår. 150 g/ha forår | Loose silky-bent grass | *Apera spicaventi* | 1 leaf | Selected plants 4 and 7 days after squirting. 4 doses (0. 1/4N. 1/2 N and 1 N) | Final biomass. Visual evaluation forår) |
| Atlantis | Iodosulfuron+mesosulfu ron | IOD+ME S | 0.5 l/ha Renol | 1N=150 g/ha afterår. 150 g/ha forår | Loose silky-bent grass | *Apera spicaventi* | | Selected plants 4 and 7 days after squirting. 4 doses (0. 1/4N. 1/2 N and 1 N) | Final biomass |
| Atlantis | Iodosulfuron+mesosulfu ron | IOD+MES | 1 l/ha Bio-power | 1N = 400 g/ha forår | Alm raj-græs | *Lolium pe-renne* | st. 28 | Selected plants 4 and 7 days after squirting. 5 doses (0. 1/8N. 1/4N. 1/2 N and 1 N) | Final bio-mass, visual evaluation |
| Hussar OD | Iodosulfuron | IOD | 0.5 l/ha renol | 1 N= 0.075 l/ha | Perennial ryegrass | *Lolium perenne* | St. 12. 30.2. 32 | Selected plants 4 and 7 days after squirting. 5 doses (0. 1/8 N. 1/4N. 1/2 N and 1 N) | Final biomass. visual evaluation |
| Roundup Bio | Glyphosat | GLY | | 1 N=2 l/ha | Nettle | *Urtica dioeca* | Root germinated | Selected plants 8 DAT. 7 doses (0. 1/32N. 1/16N. 1/8N. 1/4N. 1/2N. 1N) | Final biomass |
| MaisTer | Foramsulfuron+iodosulfur on | FOR+IO D | Mais-oil | 1N=100 g/ha | Nettle | *Urtica dioeca* | Root germinated | Selected plants 8 DAT. 5 doses (0. 1/8N. 1/4N. 1/2n. 1N) | Final biomass |
| Roundup Bio | Glyphosat | GLY | | 1 N=1 l/ha | Dandelion | *Taraxacum vulgare* | Root germinated | Selected plants 7 DAT. 7 doses (0. 1/32N. 1/16N. 1/8N. 1/4N. 1/2N. 1N) | Final biomass |
| MaisTer | Foramsulfuron+iodosulfur on | FOR+IO D | Mais-oil | 1 N=50 g/ha | Dandelion | *Taraxacum vulgare* | Root germinated | Selected plants 7 DAT. 5 doses (0. 1/8N. 1/4N. 1/2n. 1 N) | Final biomass |
| Roundup Bio | Glyphosat | GLY | | 1 N=4 l/ha | Nettle | *Urtica dioeca* | Seed germinated | plants 8 DAT. 7 doses (0. 1/32N. 1/16N. 1/8N. 1/4N. 1/2N. 1 N) | Final biomass |
| MaisTer | Foramsulfuron+iodosulfur on | FOR+IO D | Mais-oil | 1 N=40 g/ha | Nettle | *Urtica dioeca* | Frofremspio ret | Selected plants 8 DAT. 5 doses (0. 1/8N. 1/4N. 1/2n. 1N) | Final biomass |
| Roundup Bio | Glyphosat | GLY | | 1 N=1 l/ha | Dandelion | *Taraxacum vulgare* | Seed germinated | Selected plants 7 DAT. 7 doses (0. 1/32N. 1/16N. 1/8N. 1/4N. 1/2N. 1N) | Final biomass |
| MaisTer | Foramsulfuron+iodosulfur on | FOR+IO D | Mais-oil | 1 N= 25 g/ha | Dandelion | *Taraxacum vulgare* | Seed germinated | Selected plants 7 DAT. 5 doses (0. 1/8N. 1/4N. 1/2n. 1N) | Final biomass |
| Hussar OD | Iodosulfuron | IOD | 0.5 l/ha Renol | 1 N=3g as/ha | Perennial ryegrass | *Lolium perenne* | 3-4 offshoot | Selected plants 4 DAT. 7 DAT. 14 DAT (0. 1/16 N. 1/8 N. 1/4 N. 1/2 N. 1 N) | Final biomass |
| Atlantis | Iodosulfuron + mesosulfuron | TOD+M ES | 0.5 l/ha Renol | 1 N= 120-1920 afh. Af regn | Perennial ryegrass | *Lolium perenne* | 3-4 offshoot | Selected plants 4 DAT. 7 DAT. 14 DAT (0. 1/32 N. 1/16 N. 1/8 N. 1/4 N. 1/2 N. 1 N) | Final biomass |
| Atlantis | Iodosulfuron+mesosulfu ron | IOD+ME S | 0.5 l/ha Renol | 1N= 20g/ha | Loose silky-bent grass | *Apera spicaventi* | 5-6 leaves. 2-3 offshoot | Selected plants 8 DAT (0. 1/16N. 1/8N. 1/4N. 1/2N and 1 N of biotype 1 and 2. from 1/4 N to 8 N on biotype 3-5 (resistant) | Visual evaluation 8 DAT. Final biomass after 3 weeks |

**Table 14. Study no. 972/04 Semi-field study (Dose/response) rain stability 1. Study (freeze dried)**

| Rain stability of Perennial ryegrass (*Lolium perenne*) exposed to Hussar. The plants were squirted the 30/9-2004 on 3-4 leaves stage and treated with 5 mm rain at intensity of 20 mm/hour. Harvested for biomarker the 4/10 (time 1). the 7/10 (time 2) and the 21/10 (time 3). At time 3 plants for biomass determination were harvested. No visual evaluations are present. All data are mean-values of three replicates. The numbers in brackets are standard deviations. | | | | | | |
|---|---|---|---|---|---|---|
| **Additive** | **Rain** | **Doses (g a.i./ha)** | **Reduction in fresh biomass (%)** | **Reduction in dry biomass (%)** | **PANTONE^{®}**-**colour Stick A 4 days after exposure** | **PANTONE^{®}**-**colour Stick B 4 days after exposure** |
| None | None | 0 | 0 (20.8) | 0 (18.1) | 663 | 5645 |
| | | 0.313 | 12.8 (18.9) | 6.2 (18.1) | 663 | 5645 |
| | | 0.625 | 33.6 (10.3) | 31.5 (11.1) | 665 | 443 |
| | | 1.25 | 61.8 (5.8) | 51.5 (6.4) | 666 | 7545 |
| | | 2.5 | 84.3 (3.6) | 72.0 (6.8) | 667 | 7546 |
| | | 5.0 | 88.8 (2.4) | 76.6 (5.5) | 667 | 534 |
| | | 10.0 | 89.3 (1.4) | 77.8 (3.4) | 273 | 534 |
| None | None | 0 | 0 (20.8) | 0 (18.1) | 666 | 5645 |
| | 1 hour | 0.313 | 8.3 (15.8) | 5.9 (15.6) | 665 | 416 |
| | | 0.625 | -13.9 (16.2) | -11.9 (16.7) | 665 | 416 |
| | | 1.25 | -11.6 (2.4) | -13.5 (3.2) | 666 | 418 |
| | | 2.5 | 11.3 (29.8) | 11.4 (28.4) | 7446 | 417 |
| | | 5.0 | 57.2 (2.9) | 49.2 (2.0) | 272 | 7545 |
| | | 10.0 | 79.9 (4.1) | 67.5 (3.4) | 273 | 534 |
| None | None | 0 | 0 (20.8) | 0 (18.1) | 666 | 5635 |
| | 4 hours | 0.313 | -13.6 (26.1) | -18.9 (24.2) | 667 | 5635 |
| | | 0.625 | 1.1 (13.1) | 5.4 (10.6) | 667 | 5635 |
| | | 1.25 | 3.6 (9.6) | 3.8 (11.5) | 667 | 5635 |
| | | 2.5 | 24 (18.2) | 18.8 (17.5) | 273 | 5487 |
| | | 5.0 | 51.5 (19.9) | 42.2 (17.7) | 273 | 7545 |
| | | 10.0 | 78.9 (0.6) | 64.3 (1.7) | 273 | 534 |
| None | None | 0 | 0 (20.8) | 0 (18.1) | 664 | 5645 |
| 0.5 l/ha Renol | | 0.313 | 52.2 (5.2) | -16.3 (0.9) | 273 | 7545 |
| | | 0.625 | 85.1 (8.1) | 40.3 (2.2) | 274 | 7545 |
| | | 1.25 | 89.5 (3.0) | 77.9 (6.1) | 274 | 534 |
| | | 2.5 | 87.0 (2.8) | 80.5 (4.5) | 274 | 534 |
| | | 5.0 | 91.1 (2.0) | 72.4 (1.0) | 274 | 534 |
| | | 10.0 | 90.8 (0.6) | 79.9 (4.3) | 274 | 534 |
| None | None | 0 | 0 (20.8) | 0 (18.1) | 666 | 5645 |
| 0.5 l/ha Renol | 1 hour | 0.313 | 36.1 (19.7) | 22.1 (5.8) | 7446 | 5625 |
| | | 0.625 | 59.5 (6.7) | 49.1 (8.2) | 273 | 7545 |
| | | 1.25 | 77.2 (1.7) | 65.8 (2.5) | 273 | 534 |
| | | 2.5 | 88.0 (2.4) | 78.7 (3.4) | 273 | 534 |
| | | 5.0 | 86.8 (2.0) | 73.1 (2.6) | 2755 | 534 |
| | | 10.0 | 89.8 (1.2) | 77.8 (2.2) | 2755 | 534 |
| None | None | 0 | 0 (20.8) | 0 (18.1) | 665 | 5635 |
| 0.5 l/ha Renol | 4 hours | 0.313 | 61.5 (6.3) | 53.8 (6.7) | 272 | 7545 |
| | | 0.625 | 77.0 (5.5) | 68.8 (3.2) | 272 | 7545 |
| | | 1.25 | 82.6 (2.5) | 69.7 (3.1) | 273 | 534 |
| | | 2.5 | 89.2 (1.6) | 78.8 (3.1) | 273 | 534 |
| | | 5.0 | 88.7 (0.3) | 76.7 (1.7) | 273 | 534 |
| | | 10.0 | 90.7 (2.2) | 83.0 (2.9) | 273 | 534 |

**Table 15: Study no. 945/06 Semi-field study (Hussar in mixture with other herbicides)**

| Semi-field study dose/response with Hussar and mixtures with other herbicides for dicotyledon weed plants on perennial ryegrass *(Lolium perenne*) at 3-4 leaves stage. All data are mean values of three replicates. The values in brackets are standard deviations. | | | | | | | | |
|---|---|---|---|---|---|---|---|---|
| **Hussar mixed with** | **Hussar Dose (g a.i. /ha)** | **Visual effect 4 days after exposure** | **Visual effect 7 days after exposure** | **Reduction in fresh biomass (%)** | **PANTONE^{®}**-**colour Stick A 4 days after exposure** | **PANTONE^{®}**-**colour Stick A 7 days after exposure** | **PANTONE^{®}**-**colour Stick B 4 days after exposure** | **PANTONE^{®}**-**colour Stick B 7 days after exposure** |
| None | 0 | 0 | 0 | 0 (5.3) | 665 | 665 | 535 | 535 |
| | 0.19 | 0 | 2.3 (0.6) | 30.4 (4.1) | 666 | 665 | 535 | 535 |
| | 0.38 | 0 | 2.3 (0.6) | 64.7 (4.2) | 7445 | 272 | 534 | 534 |
| | 0.75 | 0 | 2.7 (0.6) | 94.9 (0.4) | 7445 | 668 | 2766 | 2766 |
| | 1.5 | 0 | 4.0 (1.0) | 96.1 (0.7) | 666 | 273 | 2766 | 2766 |
| | 3 | 0 | 4.7 (1.2) | 97.4 (0.4) | 666 | 668 | 534 | 2766 |
| 0.5 l/ha Oxitril | 0 | 0 | 2.0 (0) | 0(10.1) | 5245 | 665 | 5655 | 7544 |
| | 0.19 | 0 | 1.7 | 38.3 | 5245 | 664 | 5655 | 7544 |
| | | | (0.6) | (10.1) | | | | |
| | 0.38 | 0 | 4.5 (2.1) | 79.0 (5) | 663 | 665 | 5517 | 5645 |
| | 0.75 | 0 | 3.3 (0.6) | 90.4 (0.4) | 7445 | 666 | 5655 | 5645 |
| | 1.5 | 0 | 3.3 (0.6) | 95.3 (0.4) | 665 | 667 | 7443 | 444 |
| | 3 | 0 | 3.0 (0) | 95.5 (0.1) | 665 | 667 | 5517 | 7545 |
| 0.6 l/ha Starane | 0 | 0 | 0 | 0 (6.6) | 665 | 665 | 535 | 7544 |
| | 0.19 | 0 | 0 | 0 (1.3) | 7445 | 665 | 535 | 7544 |
| | 0.38 | 0 | 2.3 (0.6) | 79.4 (2.6) | 7445 | 666 | 534 | 2766 |
| | 0.75 | 0 | 3.3 (1.5) | 79.2 (5.7) | 667 | 666 | 534 | 2766 |
| | 1.5 | 0 | 3.7 (0.6) | 96.1 (0.6) | 667 | 7447 | 2766 | 2766 |
| | 3 | 0 | 3.0 (1.0) | 96.2 (0.4) | 667 | 668 | 2766 | 2766 |
| 1 tablet/ha* Lexus | 0 | 0 | 3.0 (1.0) | 0 () | 272 | 273 | 535 | 534 |
| | 0.19 | 0 | 3.7 (0.6) | 83.7 (4.6) | 272 | 273 | 535 | 2766 |
| | 0.38 | 0 | 5.7 (0.6) | 92.5 (0.4) | 273 | 274 | 534 | 2766 |
| | 0.75 | 0 | 4.0 (1.7) | 97.0 (0.8) | 273 | 274 | 534 | 2766 |
| | 1.5 | 0 | 4.0 (1.4) | 98.1 (0.2) | 273 | 274 | 2766 | 2766 |
| | 3 | 0 | 4.3 (1.5) | 94.4 (0.3) | 667 | 274 | 2766 | 2766 |

| | | | | | | | | |
|---|---|---|---|---|---|---|---|---|
| * 1 tablet/ha corresponds to 3.75 g a.i./ha. | | | | | | | | |

**Table 16: Study no. 946/06 Semi-field study (Dose/response) 2. study with rain stability**

| Semi-field study dose/response with Atlantis on perennial ryegrass (*Lolium perenne*) at 3-4 leaves stage. All data are mean values for three replicates. The data in brackets are standard deviations. | | | | | | | | |
|---|---|---|---|---|---|---|---|---|
| **Additive** | **Rain (3 mm ved 10 mm/t)** | **Atlantis Dose (g a.i./ha )** | **Reduction in fresh biomass (%)** | **Reduction in dry biomass (%)** | **PANTONE^{®}**-**colour Stick A 4 days after exposure** | **PANTONE^{®}**-**colour Stick A 7 days after exposure** | **PANTONE^{®}**-**colour Stick B 4 days after exposure** | **PAN-TONE^{®} -colour Stick B 7 days after exposure** |
| None | None | 0 | 0 (3.8) | 0 (0.9) | 664 | 7527 | 7543 | 5565 |
| | | 15 | -17.1 (3.1) | -16.7 (0.8) | 665 | 664 | 7543 | 5527 |
| | | 30 | -16.8 (2.5) | -4.8 (0.8) | 665 | 5235 | 7544 | 7543 |
| | | 60 | 31.6 (5) | 36.9 (0.9) | 666 | 664 | 535 | 7544 |
| | | 120 | 78 (2.5) | 72.6(0.6) | 666 | 666 | 535 | 7545 |
| | | 240 | 95.4 (0.7) | 88.1 (0.2) | 7445 | 666 | 534 | 534 |
| | | 480 | 95.4 (0.3) | 86.9 (0.3) | 665 | 667 | 534 | 2766 |
| None | None | 0 | 0 (3.8) | 0 (0.9) | 664 | 7527 | 7543 | 5565 |
| | 1 hour | 60 | -2.3 (12.1) | -6.0 (3) | 665 | 7527 | 7544 | 5565 |
| | | 120 | 28.4 (0.5) | 27.4 (0.2) | 665 | 5245 | 536 | 7543 |
| | | 240 | 64.9 (3.2) | 60.7 (0.4) | 665 | 665 | 535 | 535 |
| | | 480 | 89.0 (1.8) | 82.1 (0.5) | 665 | 666 | 534 | 535 |
| | | 960 | 95.4 (0.3) | 88.1 (0.2) | 665 | 666 | 534 | 535 |
| | | 1920 | 96.8 (0.3) | 90.5 (0.2) | 665 | 273 | 534 | 535 |
| None | None | 0 | 0 (3.8) | 0 (0.9) | 664 | 7527 | 7543 | 5565 |
| | 3 hours | 60 | -4.1 (7.6) | 9.5 (0.7) | 665 | 664 | 7543 | 7543 |
| | | 120 | 23.5 (4.8) | 26.2 (0.6) | 665 | 7527 | 7543 | 7544 |
| | | 240 | 72.8 (1.6) | 69.0 (0.5) | 666 | 5235 | 534 | 535 |
| | | 480 | 90.4 (1.4) | 83.3 (0.4) | 665 | 5225 | 535 | 535 |
| | | 960 | 95.9 (0.4) | 89.3 (0.3) | 665 | 666 | 534 | 534 |
| | | 1920 | 95.7 (0.8) | 85.7 (0.4) | 5235 | 667 | 534 | 534 |
| None | None | 0 | 0 (3.8) | 0 (0.9) | 664 | 7527 | 7543 | 5565 |
| 0.5 l/ha Renol | None | 3.8 | 16.8(1.5) | 20.2 (1) | 665 | 7527 | 7543 | 7543 |
| | | 7.5 | 29.0 (4.1) | 31.0 (1) | 5235 | 7527 | 7543 | 535 |
| | | 15 | 55.7 (6.8) | 51.2 (1.4) | 665 | 5235 | 535 | 535 |
| | | 30 | 94.2 (0.9) | 86.9 (0.5) | 665 | 666 | 7544 | 535 |
| | | 60 | 93.9 (0.3) | 83.3 (0.2) | 7445 | 667 | 534 | 534 |
| | | 120 | 95.1 (0.3) | 86.9 (0.2) | 665 | 667 | 534 | 534 |
| None | None | 0 | 0 (3.8) | 0 (0.9) | 664 | 7527 | 7543 | 5565 |
| 0.5 l/ha Renol | 1 hour | 30 | 56.2 (9.1) | 56.0(1.8) | 665 | 5235 | 535 | 536 |
| | | 60 | 80.9 (3.2) | 77.4 (0.8) | 666 | 665 | 535 | 536 |
| | | 120 | 79.4 (2) | 72.6 (0.6) | 666 | 665 | 535 | 535 |
| | | 240 | 93.6 (0.8) | 91.7 (0.6) | 665 | 666 | 535 | 535 |
| | | 480 | 92.8 (0.6) | 82.1 (0.3) | 665 | 666 | 535 | 535 |
| | | 960 | 93.3 (1.3) | 82.1 (0.9) | 665 | 666 | 534 | 535 |
| None | None | 0 | 0 (3.8) | 0 (0.9) | 664 | 7527 | 7543 | 5565 |
| 0.5 l/ha Renol | 3 hours | 30 | 71.9 (7.2) | 69.0 (1.4) | 665 | 5235 | 535 | 535 |
| | | 60 | 89.3 (0.9) | 82.1 (0.2) | 665 | 5235 | 535 | 535 |
| | | 120 | 94.5 (0.4) | 86.9 (0.1) | 665 | 666 | 535 | 535 |
| | | 240 | 96.5 (0.2) | 89.3 (0.1) | 667 | 5225 | 535 | 534 |
| | | 480 | 95.4 (0.2) | 88.1 (0.1) | 666 | 666 | 534 | 534 |
| | | 960 | 96.5 (0.4) | 90.5 (0.2) | 666 | 667 | 536 | 534 |

**Table 17: Field study at Hobro/Sealand (964/05. 966/05 & 917/06) with Hussar and Atlantis and annual meadow grass. loose silky-bent grass and perennial ryegrass 2005 & 2006**

| Field study (dose/response) with Hussar and Atlantis on perennial ryegrass (*Lolium perenne*) loose silky-bent grass (*Apera spica-venti*) and annual meadow grass (*Poa annua*) at the autumn squirting 2005 and spring squirting 2006. All data are mean values of three replicates. Data in brackets are standard deviations. | | | | | | | | |
|---|---|---|---|---|---|---|---|---|
| **Herbicide/ plant species year/field area** | **Dose (g a.i./h a)** | **Reduction in fresh biomass (%)** | | **Visual effect** | **PANTONE^{®} colour Stick A 4 days after exposure** | **PANTONE^{®}**-**colour Stick A 7 days after exposure** | **PANTONE^{®}**-**colour Stick B 4 days after exposure** | **PANTONE^{®}**-**colour Stick P 7 days after exposure** |
| Atlantis/Annual meadow grass / 2005/Hobr o | 0 | 0 (0) | | - | 7527 | 7527 | 7544 | 7544 |
| | 50 | 29.0 (33.0) | | - | 272 | 273 | 7545 | 534 |
| | 100 | 43.7 (30.3) | | - | 272 | 273 | 7545 | 534 |
| | 200 | 58.8 (35.1) | | - | 272 | 273 | 535 | 534 |
| Atlantis/Annual meadow grass / 2006/Hobr o | 0 | 0 (0) | | - | 7445 | 665 | 534 | 7543 |
| | 50 | 56.9 (18.6) | | - | 272 | 2745 | 534 | 2756 |
| | 100 | 77.1 (6.7) | | - | 273 | 2755 | 534 | 2756 |
| | 200 | 56.4 (26.2) | | - | 273 | 2745 | 534 | 2756 |
| Hussar/Annual meadow grass/ 2005/Hobr o | 0 | 0 (0) | | - | 664 | 7444 | 7544 | 7544 |
| | 37.5 | 37.7 (23.6) | | - | 272 | 272 | 7544 | 535 |
| | 75 | 46.8 (20.9) | | - | 273 | 273 | 7544 | 534 |
| | 150 | 61.5 (13.1) | | - | 273 | 273 | 7545 | 534 |
| Hussar/Annual meadow grass/ 2006/Hobr o | 0 | 0 (0) | | - | 7445 | 665 | 534 | 7543 |
| | 50 | 58.2 (19.9) | | - | 273 | 273 | 2766 | 2756 |
| | 100 | 41.2 (22.9) | | - | 273 | 273 | 2766 | 2756 |
| | 200 | 74.7 (8.1) | | - | 273 | 2745 | 2766 | 2756 |
| Atlantis/Perenni al ryegrass/ 2005/Hobr o | 0 | 0 | | - | 665 | 665 | 7544 | 7544 |
| | 50 | - | | - | 272 | 666 | 535 | 534 |
| | 100 | - | | - | 271 | 665 | 535 | 535 |
| | 200 | - | | - | 273 | 273 | 535 | 534 |
| Atlantis/Perenni al ryegrass/ 2005/Seal and | 0 | 0 | | 0 (0) | 270 | 270 | 7545 | 534 |
| | 50 | 35 (26.5) | | 5.63 (0.75) | 666 | 271 | 7545 | 535 |
| | 100 | 43.8 (35.4) | | 7.50 (1.29) | 7446 | 272 | 7545 | 534 |
| | 200 | 70 (33.7) | | 9.17 (0.29) | 7446 | 272 | 7545 | 534 |
| Atlantis/Perenni al ryegrass/ 2006/Hobr o | 0 | 0 | | - | 7445 | 7445 | 534 | 7544 |
| | 50 | 25.1 | | - | 273 | 273 | 534 | 534 |
| | 100 | 48.1 | | - | 2746 | 2745 | 534 | 534 |
| | 200 | 65.6 | | - | 2746 | 2745 | 534 | 534 |
| Atlantis/Perenni al ryegrass/ 2006/Seal and | 0 | 0(0) | | 0 | 272 | 272 | 5793 | 7544 |
| | 50 | 48.3 (45.9) | | 1 (0) | 273 | 2745 | 443 | 444 |
| | 100 | 62.5 (23.6) | | 3.3 (1.0) | 273 | 2755 | 443 | 7545 |
| | 200 | 91.5 (11.1) | | 4.8 (1.7) | 273 | 2755 | 443 | 533 |
| Atlantis/Perenni al ryegrass/ 2006/Seal and | 0 | 0 | 0 | 0 | 273 | 273 | 7544 | 7544 |
| (Forsogs nr. 917/06) | 50 | 7.5 (9.6)* | 0 | 1 | 667 | 667 | 7545 | 7544 |
| | 100 | 20 | 0 | 1.4 | 667 | 273 | 7545 | 7545 |
| | | (8.2)* | | | | | | |
| | 200 | 75.0 (5.8)* | 50.0 (10.8)* * | 2.9 | 273 | 273 | 7545 | 7545 |
| | 400 | 97.3 (1.5)* | 90.0 (5.8)** | 3.1 | 274 | 274 | 534 | 534 |
| Hussar/Peren nial ryegrass/ 2005/Hobr o | 0 | 0 | | - | 664 | 7444 | 7544 | 7544 |
| | 37.5 | - | | - | 665 | 271 | 7544 | 7544 |
| | 75 | - | | - | 7446 | 272 | 535 | 534 |
| | 150 | - | | - | 272 | 272 | 535 | 534 |
| Hussar/Peren nial ryegrass/ 2005/Seal and | 0 | 0 | | 0 | 270 | 270 | 7545 | 534 |
| | 37.5 | 41.3 (32.8) | | 7.1 (0.5) | 271 | 666 | 7545 | 534 |
| | 75 | 76.3 (12.5) | | 7.4 (1.0) | 272 | 272 | 7545 | 534 |
| | 150 | 95.3 (3.8) | | 9.1 (0.3) | 272 | 274 | 7546 | 534 |
| Hussar/Peren nial ryegrass/ 2006/Hobr o | 0 | 0 | | - | 7445 | 7445 | 534 | 7544 |
| | 50 | - | | - | 2745 | 2745 | 534 | 534 |
| | 100 | - | | - | 273 | 2745 | 534 | 534 |
| | 200 | - | | - | 2745 | 273 | 534 | 2757 |
| Hussar/Peren nial ryegrass/ 2006/Seal and | 0 | 0 | | 0 | 272 | 272 | 5793 | 7544 |
| | 50 | 53.8 (34.0) | | 3.1 (1.3) | 273 | 2745 | 443 | 7545 |
| | 100 | 95.3 (3.8) | | 6.0 (1.4) | 273 | 2745 | 7545 | 534 |
| | 200 | 96.0 (4.0) | | 5.5 (1.7) | 2755 | 2765 | 7545 | 533 |
| Atlantis/ Loose silky-bent grass/200 | 0 | 0 | | 0 | 272 | 2745 | 7545-533 | 534 |
| 5/Sealand | | | | | | | | |
| | 37.5 | 95 | | 6.5 (2.2) | 273 | 2755 | 533 | 534 |
| | 75 | 90 (0) | | 6.8 (2.2) | 273 | 2745 | 533 | 2768 |
| | 150 | 92.5 (3.5) | | 9.1 (0.9) | 273 | 2755 | 533 | 2768 |
| Atlantis/ Loose silky-bent grass/200 6/Sealand | 0 | 0 | | 0 | 7446 | 7446 | 535 | 5517 |
| | 37.5 | 96.0 (1.7) | | 7.7 (0.6) | 2745 | 2745 | 534 | 535 |
| | 75 | 98.7 (0.6) | | 9.2 (0.6) | 2745 | 2745 | 534 | 534 |
| | 150 | 98.0 (0) | | 9.5 (0) | 2745 | 2745 | 534 | 534 |
| Hussar/ Loose silky-bent grass/200 5/Sealand | 0 | 0 | | 0 | 272 | 273 | 534 | 534 |
| | 25 | 60.0 (28.3) | | 6.3 (2.4) | 273 | 273 | 534 | 534 |
| | 50 | 96.5 (2.1) | | 9.0 (0.8) | 273 | 273 | 534 | 534 |
| | 100 | 99.0(1.4) | | 9.7 (0.2) | 273 | 273 | 534 | 534 |
| Hussar/ Loose silky-bent grass/200 6/Sealand | 0 | 0 | | 0 | 7446 | 7446 | 535 | 5517 |
| | 37.5 | 93.3 (2.9) | | 7.7 (0.6) | 272 | 273 | 2756 | 534 |
| | 75 | 97.0 (1.7) | | 9.2 (0.6) | 2745 | 273 | 2766 | 534 |
| | 150 | 97.0 (1.7) | | 9.5 (0) | 2745 | 273 | 2766 | 534 |

| | | | | | | | | |
|---|---|---|---|---|---|---|---|---|
| *With crop; **Without crop | | | | | | | | |

**Table 18: Study no. 948/06 Semi-field study (dose/response) investigations with Hussar OD and different development stages for perennial ryegrass**

| Semi-field dose/response study with Hussar OD on perennial ryegrass (*Lolium perenne*) at three different stages. All data are mean values of three replicates. The values in bracket are standard deviation. | | | | | | |
|---|---|---|---|---|---|---|
| Development stage | Hussar OD Dose (g a.i./ha) | Reduction in fresh biomass (%) | PANTONE^{®}-colour Stick A 4 days after exposure | PANTONE^{®}-colour Stick A 7 days after exposure | PANTONE^{®}-colour Stick B 4 days after exposure | PANTONE^{®}-colour Stick B 7 days after exposure |
| Stage 12 | 0 | 0 (0) | 272 | 7445 | 534 | 7544 |
| | 0.0094 | 6.7 (5.8) | 271 | 7445 | 2758 | 7544 |
| | 0.0188 | 40 (10) | 273 | 7445 | 2758/534 | 534 |
| | 0.0375 | 86.7 (5.8) | 273 | 7445 | 2758 | 2758 |
| | 0.075 | 97 (1.7) | 273 | 2755 | 2766 | 2765 |
| Stage 30.2 | 0 | 0 (0) | 7445 | 663 | 7544 | 7544 |
| | 0.0094 | 25.7 (7.6) | 665 | 665 | 7544 | 7546 |
| | 0.0188 | 85 (5) | 667 | 7445 | 7546 | 274 |
| | 0.0375 | 98.7 (0.6) | 273 | 666 | 534 | 2766 |
| | 0.075 | 100 (0) | 273 | 666 | 7546 | 2766 |
| Stage 32 | 0 | 0 (0) | 665 | 665 | 7544 | 7544 |
| | 0.0094 | 21.7 (12.8) | 665 | 665 | 7546 | 7544 |
| | 0.0188 | 50 (0) | 7445 | 272 | 7545 | 7544 |
| | 0.0375 | 86.7 (2.9) | 666 | 667 | 7545 | 7546 |
| | 0.075 | 97 (1.7) | 666 | 667 | 7545 | 7545 |

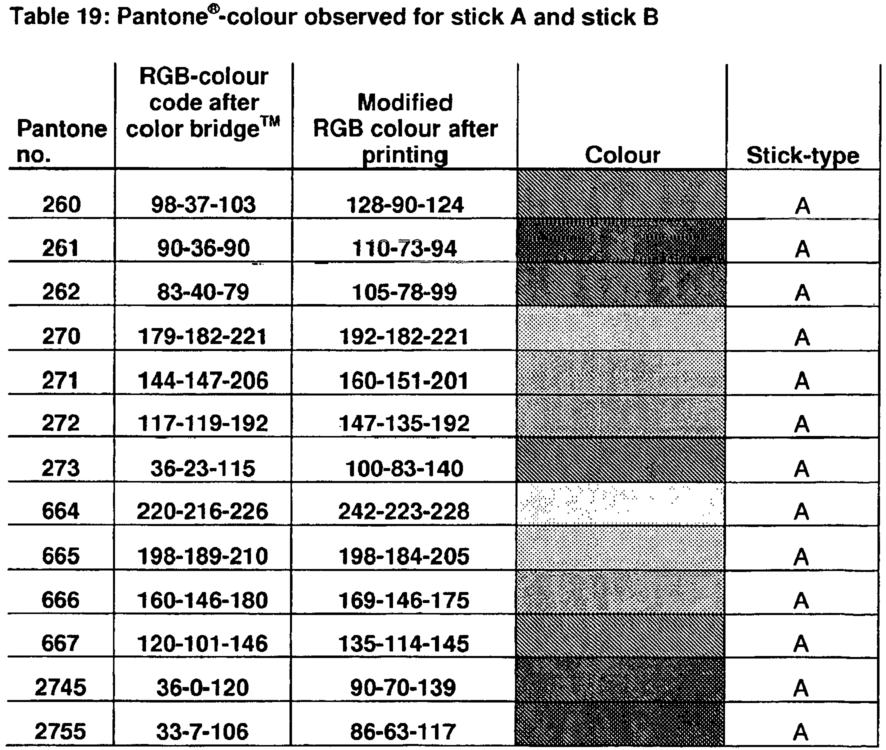

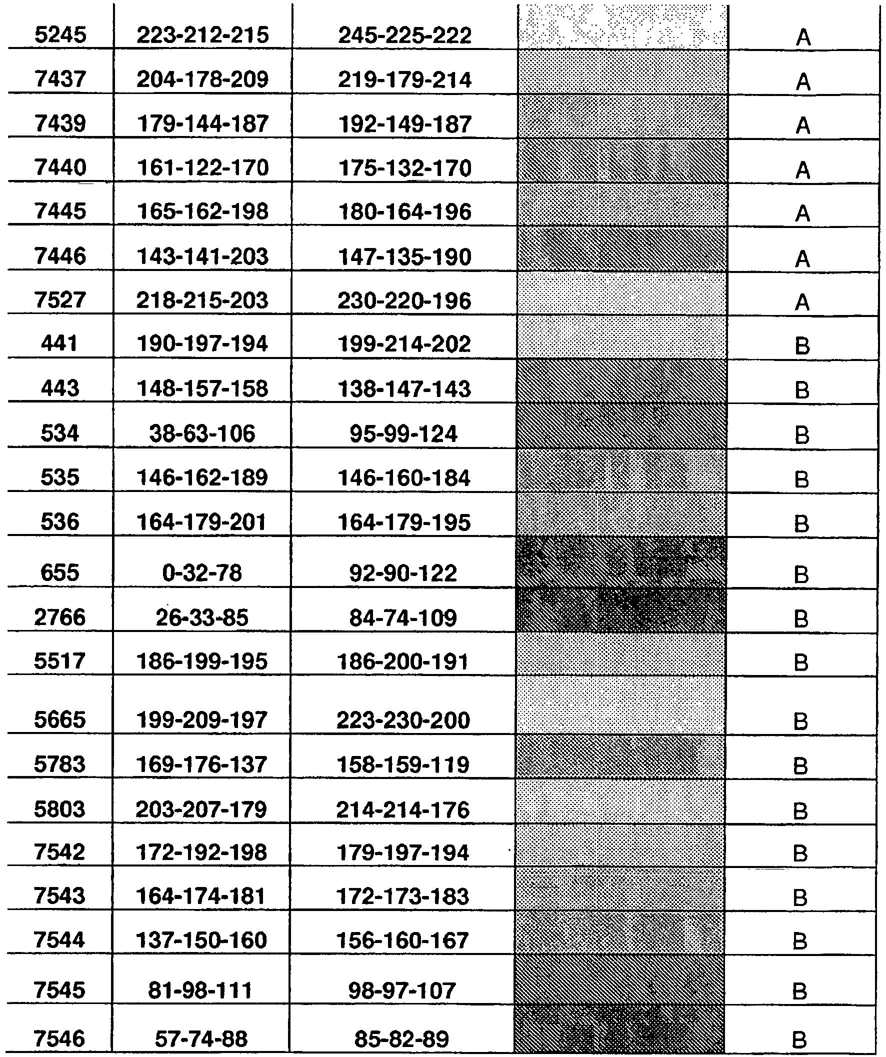

### Example 4

### A test-kit to be used for testing effect of Hussar or Atlantis on Perennial Rye grass, Silky bent-grass or Annual meadow grass

The kit is described with respect to stick B as an Herbicide Weed Response Test. The reagent is 5% α-naphthol (1-naphthol) in 96% ethanol. The disk to be added is the filter Adventec 590.

### Herbicide Weed Response-Test for Sulfonylurea-herbicides

This Herbicide Weed Response Test is used to evaluate the final effect of the herbicides Hussar or Atlantis, with extracts of fresh weed plant species: Rye grass, Silky bent-grass or Annual meadow grass 6-8 days after exposure.

The test is used to control the effect of the applied herbicide dose. In case of rain shortly after spraying, the test can also be used to control whether the herbicide has had enough time to have an effect in the used dose.

The test is developed to weed plants at BBCH growth stage 12 (2 leaves) to BBCH growth stage 23 (tillering or bush-stage). The stages are described in Danish in "Vejledning i Planteværn", Landbrugsforlaget, Dansk Landbrugsrädgivning Landscenteret, Planteavl, Ministeriet for Fodevare, Landbrug og Fiskeri, Danmarks Jordbrugs-Forskning (ISSN: 0907-4066; ISBN: 87 7470 926 7, page 303 (1-323). Although the stages are described in respect of cereals, the description can also be used in respect of other plants e.g. weed plants, and especially grass weed plants. The test is performed within 15 minutes after the weed plants are collected.

### Collection of weed plants

1. Start by identifying which plant species of the 3 different plant species Rye grass, Silky-bent grass and Annual meadow grass is the widely distributed or most problematic in the field.
2. Three samples of this weed species need to be collected from 3 different places in each field (without regard to field size). Choose the areas in the field where the spraying has been most evenly distributed.
3. The plants must be collected 6 to 8 days after exposure.
4. From each area a small bunch of 20-25 weed plants is placed in the plastic bags. Avoid soil and water on the plants.
5. Place the plastic bags in a cool area protected against light.
6. The plants must be tested between half an hour and one hour after collection.

### Description of weed plants

### Rye grass, Lolium perenne

### Family: Grass (Poaceae)

**Early stage:** The vernation is valvated. The leaves appear soft and slack. The ventral side of the leaves is very shiny. Small leaf teeth. The ligule is short approx. 1 mm. The lower ligules are often pink purple.

**Risk of confusion:** Italian rye grass has larger and more comprehensive leaf teeth and curled vernation.

**Adult plants:** Rye grass is a medium-sized grass species with 20-50 cm tall, vertical stems. The leaves sheaths are compressed and the ventral sides are strongly shining. The awnless spikelets are collected in an open oblonged spike. The edge of the spikelets is turned against the straw in contrast to ryegrass where the surface of the spikelets is turned against the straw.

**Seed production:** Up to 150 seeds per spike.

### Annual meadow grass, Poa annua

### Family: Grass (Poaceae)

**Early stage:** The vernation is valvated and the leaves sheaths are compressed. The leaves are thin and often transversal wrinkled. The leaf apex is boat shaped. When the leaves are held against a source of light two light, stribes along the middle of the leaf appear. The ligule is 2-4 mm long. The whole plant often has a crumpled look.

**Risk of confusion:** 1. Rye grass which is larger and without transverse leaves. 2. Spiky-bent grass which does not have boat shaped leaf apexes.

**Adult plants:** Annual meadow grass is a small plant with 5-20 cm long ascending stems. The awnless spikelets are placed in small open tops. Annual meadow grass is easily distinguished from other meadow grasses by the size.

**Seed production:** Up to 500 seeds per spike.

### Silky-bent grass, Apera spica-venti

### Family: Grass (Poaceae)

**Early stage:** The vernation is rolled. The leaves are dull, scabrous and narrow. The leaf teeth are missing. The ligule is frayed and often very long, up to approx. 7 mm.

**Risk of confusion:** Spiky-bent grass which has boat shaped leaf apexes.

**Adult plants:** Silky-bent grass is a large plant with 40-80 cm tall ascending straws. The small single flower spikelets (length approx. 2.5-3 mm) are colleted in a pyramidal top. Each spikelet is supplied with a 5-10 mm long awn.

**Seed production:** Up to 5.000 seeds per spike.

### Collection of weed plants

Regardless of the size of the field, 3 samples are collected from 3 different places in the field. Collection and testing must take place 6-8 days after exposure to the herbicides. 20-25 weed plants are collected and the roots cut off. The plants are then placed in the enclosed plastic bags and stored in a cool place protected from light. The weed plants must be tested between ½ hour and 1 hour after collection.

### Preparation of plant extract

0.20 g plant material is used for each test.

Remove the protection cover of the scale and make sure that the scale is placed in a horizontal position. Press the on/off button to turn on the scale and make sure that the scale is in level.

Unscrew the lid of the yellow container. Remove the foam and place the glass balls in the lid. Place the container without the lid on the scale. Press the reset button so that 0.00 appears on the display.

Weigh the needed plant material. Use only plants with no water and soil. Use the scissors to cut pieces of approx. 1/4 cm from a bunch of the plants from the plastic bag directly into the plastic container. Move the glass balls back into the plastic cup and carefully add 3.5 ml of the solvent from the syringe (the plunger is placed at the 10 ml point) into the plastic container.

Put the lid on the plastic cup and make sure it is closed tightly. Shake the plant material vigorously for 2 minutes until the extract appears green and muddy and only fibres remain in the container. Carefully add the remaining solvent in the syringe to the suspension in the container and shake the container closed with the lid for further ½ minute.

Suck up the extract with the empty syringe. Place a syringe filter on the filled syringe and filter the extract into the small plastic cup found in the left side of the test-kit box. Slowly press the plunge entirely down. The filtered extract is now ready for use. The plastic bag is to be used as disposal bag for the syringe, syringe filters, plastic cup, glass balls etc.

### The test

The test must be performed in a well-ventilated room or outdoors. Avoid inhalation of vapours. Use the reagents with care.

Hold the pipette close to the plastic cup containing the filtered extract and suck up the extract a couple of times until the pipette is full.

Hold the pipette in vertical position and drip 13 drops (approximately 0.67g) into an empty plastic cup. With the cup tilted, slowly add 2 drops of reagent (about 0.04 g) followed by 24 drops (about 0.95g) of conc. sulphuric acid to the mixture. The mixture develops heat at 70oC which starts the chemical reaction (it fizzes and develops vapours - do not inhale these vapours). (The density of the plant extract and each of the chemicals may be different, thus 1 drop of one liquid need not have the same weight as 1 drop of another liquid).

Leave the plastic cup to cool for 10-15 minutes.

### Reading of the results

Gently shake the plastic cup and place a filter disc at the bottom of the cup. The colour is hereby distributed evenly in the disc. After 15 seconds, compare the colour to the colour chart.

Place the plastic cup with the disc on the colour chart to the right, compare the colours and read the result of the final effect indicated by a red, yellow or green colour. If at least two out of three samples from one field are within the same colour zone, the result is unambiguous.

After testing, place the stacked plastic cups, syringe, syringe filter, and glass balls in the plastic disposable bag.

Only the scale, the scissors and the pincer can be reused.

### Colour scale - Effect assessment

Effect zones are separated into three zones indicated by red, yellow and green. These colours are not the colours obtained on the stick, but a group of colours which can be obtianed on the stick correspond to one of the three indicated colours.

Red zone (PANTONE^{®} No. 5517u, 443u, 7543u, 7544u (u = uncoated) on the stick) - Stop! No effect of the herbicide treatment

Yellow zone (PANTONE^{®} No. 7545u, 535u, 534u (u = uncoated) on the stick) - Concider the result!

| | | |
|---|---|---|
| Rye grass | - Hussar/Atlantis | 40-90% reduction in growth |
| Silky bent-grass | - Hussar/Atlantis | 45-80% reduction in growth |
| Annual meadow grass | - Hussar/Atlantis | 30-80% reduction in growth |

Green zone (PANTONE^{®} No. 2766u (u = uncoated) on the stick) - OK! The effect of the herbicide treatment is reducing the growth of the plant in a sufficient amount

| | | |
|---|---|---|
| Rye grass | - Hussar/Atlantis | > 96% reduction in growth |
| Silky bent-grass | - Hussar/Atlantis | > 90% reduction in growth |
| Annual meadow grass | - Hussar/Atlantis | > 90% reduction in growth |

### Red zone

Pantone No. 5517u, 443u, 7543u, 7544u (u = uncoated) is observed on the stick. Spraying effect is entirely insufficient

### Yellow zone

Pantone No. 7545u, 535u, 534u (u = uncoated) is observed on the stick. The effect of the herbicide treatment is insufficient. Consider further treatment.

### Green zone

Pantone No. 2766u (u = uncoated) is observed on the stick. The effect of the herbicide treatment is expected to have full effect. No further action is necessary.

## Claims

1. A method for testing whether material from a specific living plant has been exposed to a specific pesticide, the method comprising the steps of:
a) obtaining material from a specific plant,
b) preparing a liquid suspension of the specific plant of a) wherein the liquid suspension of the specific plant comprises at least one phytochemical compound,
c) detecting the phytochemical compounds in the liquid suspension of b) by their visual and/or UV-light colour, wherein the detection is performed without separation of said phytochemical compounds,
d) correlating said colour of the detected phytochemical compounds with a standard scale of unique visual and/or UV-light colours of unseparated phytochemical compounds for specific plant and specific pesticide combinations,
e) assessing whether the specific plant has been exposed to the specific pesticide.

2. The method according to claim 1, wherein before assessing whether the specific plant has been exposed to the specific pesticide
(a) at least one chemical reagent is selected from one or more of the following compounds:
Vanillin, sulphuric acid, naphtoresorcinol, methylene blue, β-naphtol, thymol, fluorescein, ammonia, bromocresol green, bromophenol blue, potassium permanganate, 2,7-dichlorofluorescein, Rhodamin 6G, diphenyl boric acid 2-aminoethylester, phosphoric acid, iod, potassium iodide, ammoniummolybdattin(II) chloride, cobalt(II) chloride, palladium(II) chloride, ninhydrin, 1-naphthol, bismuth(III) nitrate potassium iodide, molybdat phosphor acid, rodamin B, anise aldehyde, silver nitrate, ferri(III) chloride, zinc chloride, chlorofenolred, Methylred, Ethylred, bromothymol blue, 2,6-dichlorophenolindophenole sodium salt, Bromocresolpurpur, potassium hydroxide, glucose, 4-chloro-7-nitrobenzofurazan, 2,4-dinitrophenylhydrazine, 9-fluorenylmethylchloroformate, Tetrabutylammoniumhydroxide, lode, ammonium ferri(III)sulphate, 2-methoxy-2.4-diphenyl-3(2H)furanon (MDPF), 2-aminoethyl-diphenylborinate, aluminium chloride, berberine chloride dehydrate, 1,2-naphthochinon-4-sulfonsodium salt, Anthrone, 8-hydroxychinolin, 2-aminodiphenyl(biphenyl-2-amine), Orcinol, Urea, 4-hydroxybenzoic acid, 4-aminobenzoic acid, molybdatophosphoric acid, 2',7'-dichlorofluoresceine, 8-anilinonaphthaline-1-sulfonic acid-ammonium salt, Rhodamine, bismuth(III) nitrate, potassium iodide and chemicals or mixtures hereof, with at least one group of said chemical compounds which may or may not be present in said plant material, and
(b) provoking a chemical reaction between said at least one chemical reagent and the unseparated phytochemical compounds of the plant material,
(c) detecting the result of the chemical reaction of step (b) by visual and/or UV-light, without performing a separation of the phytochemical compounds,
(d) correlating the results of step (c) to a standard scale result developed in respect of the specific plant.

3. The method according to any one of the preceding claims further comprising
i) contacting the liquid suspension of the plant material with a chemical reagent, thus provoking a chemical reaction between the liquid suspension of the plant material and the chemical reagent, and
ii) contacting a solid support with the plant material chemically reacted with the chemicals of i), thus obtaining a solid support with a colour and and colour intensity.

4. A method of preparing a standard scale for assessing if a specific living plant has been exposed to a specific pesticide, the method comprising the steps of:
a) subjecting a specific living plant to a specific pesticide,
b) obtaining material from the exposed plant of a),
c) determining chemical responses of the specific plant by a visual and/or UV-light colour detection without performing a separation of the phytochemical compounds of the plant,
d) obtaining a standard scale of unique visual and/or UV-light colours of unseparated phytochemical compounds for the specific plant and specific pesticide combination.

5. An assay kit for use in the method according to any one of claims 1 to 4, said kit comprising:
- at least one filter paper,
- at least one solvent,
- at least one squeezing means,
- at least one standard visualising scale of unique visual and/or UV-light colours of unseparated phytochemical compounds for detection and/or prediction of exposure of a specific living plant to a specific pesticide,
- at least one container.

6. The assay kit according to claim 5, further comprising one or more of the components selected from the group consisting of:
- at least one chemical reagent, wherein said chemical reagent is selected from on one or more of the compounds:
Vanillin, sulphuric acid, naphtoresorcinol, methylene blue, β-naphtol, thymol, fluorescein, ammonia, bromocresol green, bromophenol blue, potassium permanganate, 2,7-dichlorofluorescein, Rhodamin 6G, diphenyl boric acid 2-aminoethylester, phosphoric acid, iod, potassium iodide, ammoniummolybdattin(II) chloride, cobalt(II) chloride, palladium(II) chloride, ninhydrin, 1-naphthol, bismuth(III) nitrate potassium iodide, molybdat phosphor acid, rodamin B, anise aldehyde, silver nitrate, ferri(III) chloride, zinkchloride, chlorofenolred, Methylred, Ethylred, bromothymol blue, 2,6-dichlorophenolindophenole sodium salt, Bromocresolpurpur, potassium hydroxide, glucose, 4-chloro-7-nitrobenzofurazan, 2,4-dinitrophenylhydrazine, 9-fluorenylmethylchloroformate, Tetrabutylammoniumhydroxide, Iode, ammonium ferri(III)sulphate, 2-methoxy-2.4-diphenyl-3(2H)furanon (MDPF), 2-aminoethyl-diphenylborinate, aluminium chloride, berberine chloride dehydrate, 1,2-naphthochinon-4-sulfonsodium salt, Anthrone, 8-hydroxychinolin, 2-aminodiphenyl(biphenyl-2-amine), Orcinol, Urea, 4-hydroxybenzoic acid, 4-aminobenzoic acid, molybdatophosphoric acid, 2',7'-dichlorofluoresceine, 8-anilinonaphthaline-1-sulfonic acid-ammonium salt, Rhodamine, bismuth(III) nitrate, potassium iodide and chemicals or mixtures hereof,
- at least one mortar with pistil and/or at least one box with balls to shake and/or at least one hand-press
- at least one pipette,
- at least one UV-lamp,
- at least one heater and/or at least one warm cap made of chemical reagents and solvents,
- at least one balance,
- at least one scissor,
- at least one pair of forceps,
- at least one plastic bag,
- at least one identification information to identify plant species,
- at least one instruction describing how to use the assay kit,
- at least one syringe,
- at least one filter.

7. The assay kit according to any one of claims 5 or 6, wherein said kit comprises
• 3 pieces of filter paper,
• 3 containers with lids and each with 2, 3 or 4 glass balls
• 6 glasses,
• 3 syringes each with e.g. 13.5 mL solvent,
• 1-3 containers with chemical reagents,
• 3 pipettes,
• 1 balance,
• 1 scissor,
• 1 pair of forceps,
• 3 plastic bags,
• 1 identification information to identify plant species,
• 1 instruction describing how to use the assay kit including a standard colour scale, and
• 3 filters.

8. Use of a method as defined in any one of claims 1 to 4 for determining whether a specific living crop plant or living weed plant has been subjected to a specific pesticide.

9. The method according to any one of claims 1 to 4, or the use according to claim 8, wherein the specific pesticide is a herbicide.

10. The method or the use according to claim 9, wherein the herbicide is a sulfonylurea herbicide.

11. The method according to any one of claims 1 to 4, or the use according to claim 8, wherein the specific plant is selected from the group consisting *of Apera spica venti, Lolium perenne, Poa annua and Bromus hordeaceus.*

## Patentansprüche

1. Verfahren zum Testen, ob Material von einer spezifischen lebenden Pflanze einem spezifischen Pestizid ausgesetzt wurde, wobei das Verfahren die Schritte umfasst:
a) Gewinnen von Material aus einer spezifischen Pflanze,
b) Zubereiten einer flüssigen Suspension der spezifischen Pflanze aus a), wobei die flüssige Suspension der spezifischen Pflanze wenigstens eine phytochemische Verbindung umfasst,
c) Detektieren der phytochemischen Verbindungen in der flüssigen Suspension von b) mittels ihrer sichtbaren und/oder UV-Lichtfarbe, wobei die Detektion ohne Auftrennung der phytochemischen Verbindungen durchgeführt wird,
d) Korrelieren der Farbe der detektierten phytochemischen Verbindungen mit einer Standardskala der einzigartigen sichtbaren und/oder UV-Lichtfarben der nicht-aufgetrennten phytochemischen Verbindungen für Kombinationen der spezifischen Pflanze und des spezifischen Pestizids,
e) Bewerten, ob die spezifische Pflanze dem spezifischen Pestizid ausgesetzt wurde.

2. Verfahren nach Anspruch 1, wobei vor dem Bewerten, ob die spezifische Pflanze dem spezifischen Pestizid ausgesetzt wurde,
(a) wenigstens ein chemisches Reagenz aus den folgenden Stoffen ausgewählt wird:
Vanillin, Schwefelsäure, Naphtoresorcin, Methylenblau, β-Naphthol, Thymol, Fluorescein, Ammoniak, Bromcresolgrün, Bromphenolblau, Kaliumpermanganat, 2,7-Dichlorfluorescein, Rhodamin 6G, Diphenylborsäure-2-aminoethylester, Phosphorsäure, Iod, Kaliumiodid, Ammoniummolybdat-Zinn-(II)-chlorid, Kobalt-(II)-chlorid, Palladium-(II)-chlorid, Ninhydrin, 1-Naphthol, Bismut-(III)-nitrat-Kaliumiodid, Molybdatphosphorsäure, Rhodamin B, Anisaldehyd, Silbernitrat, Eisen-(III)-chlorid, Zinkchlorid, Chlorphenolrot, Methylrot, Ethylrot, Bromthymolblau, 2,6-Dichlorphenolindophenol-Natriumsalz, Bromcresolpurpur, Kaliumhydroxid, Glucose, 4-Chlor-7-nitrobenzofurazan, 2,4-Dinitrophenylhydrazin, 9-Fluorenylmethylchlorformat, Tetrabutylammoniumhydroxid, Iod, Ammoniumeisen-(III)-sulfat, 2-Methoxy-2,4-diphenyl-3(2H)-furanon (MDPF), 2-Aminoethyldiphenylborinat, Aluminiumchlorid, Berberinchloriddehydrat, 1,2-Naphthochinon-4-sulfon-Natriumsalz, Anthron, 8-Hydroxychinolin, 2-Aminodiphenyl(biphenyl-2-amin), Orcinol, Urea, 4-Hydroxybenzoesäure, 4-Aminobenzoesäure, Molybdatphosphorsäure, 2',7'-Dichlorfluorescein, 8-Anilinnaphthalin-1-sulfonsäure-Ammoniumsalz, Rhodamin, Bismut-(III)-nitrat, Kaliumiodid und Chemikalien und Mischungen davon, wobei wenigstens eine Gruppe der chemischen Stoffe in dem Pflanzenmaterial vorhanden sein kann oder nicht, und
(b) eine chemische Reaktion zwischen dem wenigstens einen chemischen Reagenz und dem nicht-aufgetrennten phytochemischen Verbindungen des Pflanzenmaterials provoziert wird,
(c) das Ergebnis der chemischen Reaktion des Schrittes (b) mittels sichtbarem und/oder UV-Licht durchgeführt wird, ohne dass eine Auftrennung der phytochemischen Verbindungen erfolgt,
(d) die Ergebnisse des Schrittes (c) mit einem Standardskalaergebnis, das in Bezug zu der spezifischen Pflanze entwickelt worden ist, korreliert werden.

3. Verfahren nach einem der vorhergehenden Ansprüche, ferner umfassend:
i) in Kontakt bringen der flüssigen Suspension des Pflanzenmaterials mit einem chemischen Reagenz, um so eine chemische Reaktion zwischen der flüssigen Suspension des Pflanzenmaterials und dem chemischen Reagenz zu provozieren, und
ii) in Kontakt bringen eines festen Trägerstoffes mit dem Pflanzenmaterial, das mit den Chemikalien aus i) chemisch reagiert hat, um so einen festen Trägerstoff mit einer Farbe und einer Farbintensität zu gewinnen.

4. Verfahren zum Anfertigen einer Standardskala zum Bewerten, ob eine spezifische lebende Pflanze einem spezifischen Pestizid ausgesetzt wurde, wobei das Verfahren die Schritte umfasst:
a) Aussetzen einer spezifischen lebenden Pflanze einem spezifischen Pestizid,
b) Gewinnen von Material aus der in a) ausgesetzten Pflanze,
c) Bestimmen der chemischen Reaktionen der spezifischen Pflanze mittels sichtbarer und/oder UV-Lichtfarbdetektion, ohne dass eine Auftrennung der phytochemischen Verbindungen der Pflanze durchgeführt wird,
d) Erhalten einer Standardskala der einzigartigen sichtbaren und/oder UV-Lichtfarben der nicht-aufgetrennten phytochemischen Verbindungen für die Kombination der spezifischen Pflanze und des spezifischen Pestizids.

5. Testkit zum Verwenden in dem Verfahren nach einem der Ansprüche 1 bis 4, wobei das Testkit umfasst:
- wenigstens ein Filterpapier,
- wenigstens ein Lösungsmittel,
- wenigstens ein Quetschmittel,
- wenigstens eine Standardvisualisierungsskala der einzigartigen sichtbaren und/oder UV-Lichtfarben der nicht-aufgetrennten phytochemischen Verbindungen einer spezifischen lebenden Pflanze zum Detektieren und/oder zum Vorhersagen des Aussetzens einem spezifischen Pestizid,
- wenigstens einen Behälter.

6. Testkit nach Anspruch 5, ferner umfassend eine oder mehrere Komponente/n, die aus der folgenden Gruppe ausgewählt ist/sind:
- wenigstens ein chemisches Reagenz, wobei das chemische Reagenz aus einer oder mehreren der folgenden Stoffe ausgewählt ist:
Vanillin, Schwefelsäure, Naphtoresorcin, Methylenblau, β-Naphtol, Thymol, Fluorescein, Ammoniak, Bromcresolgrün, Bromphenolblau, Kaliumpermanganat, 2,7-Dichlorfluorescein, Rhodamin 6G, Diphenylborsäure-2-aminoethylester, Phosphorsäure, Iod, Kaliumiodid, Ammoniummolybdat-Zinn-(II)-chlorid, Kobalt-(II)-chlorid, Palladium-(II)-chlorid, Ninhydrin, 1-Naphthol, Bismut-(III)-nitrat-Kaliumiodid, Molybdatphosphorsäure, Rhodamin B, Anisaldehyd, Silbernitrat, Eisen-(III)-chlorid, Zinkchlorid, Chlorphenolrot, Methylrot, Ethylrot, Bromthymolblau, 2,6-Dichlorphenolindophenolnatriumsalz, Bromcresolpurpur, Kaliumhydroxid, Glucose, 4-Chlor-7-nitrobenzofurazan, 2,4-Dinitrophenylhydrazin, 9-Fluorenylmethylchlorformat, Tetrabutylammoniumhydroxid, Iod, Ammoniumeisen-(III)-sulfat, 2-Methoxy-2,4-diphenyl-3(2H)-furanon (MDPF), 2-Aminoethyldiphenylborinat, Aluminiumchlorid, Berberinchloriddehydrat, 1,2-Naphthochinon-4-sulfon-Natriumsalz, Anthron, 8-Hydroxychinolin, 2-Aminodiphenyl(biphenyl-2-amin), Orcinol, Urea, 4-Hydroxybenzoesäure, 4-Aminobenzoesäure, Molybdatphosphorsäure, 2',7'-Dichlorfluorescein, 8-Anilinnaphthalin-1-sulfonsäure-Ammoniumsalz, Rhodamin, Bismut-(III)-nitrat, Kaliumiodid und Chemikalien oder Mischungen davon,
- wenigstens einen Mörser mit Stößel und/oder wenigstens eine Box mit Kugeln zum Schütteln und/oder wenigstens eine Handpresse,
- wenigstens eine Pipette,
- wenigstens eine UV-Lampe,
- wenigstens ein Heizgerät und/oder wenigstens einen aus chemischen Reagenzien und Lösungsmitteln hergestellten Wärmedeckel,
- wenigstens eine Waage,
- wenigstens eine Schere,
- wenigstens ein Zange,
- wenigstens eine Plastiktüte,
- wenigstens eine Identifizierungsinformation, um Pflanzenarten zu identifizieren,
- wenigstens eine Anleitung, die beschreibt wie das Testkit anzuwenden ist,
- wenigstens eine Injektionsspritze,
- wenigstens einen Filter.

7. Testkit nach Anspruch 5 oder 6, wobei das Kit umfasst:
- 3 Stück Filterpapier,
- 3 Boxen mit Deckel und jeweils 2, 3 oder 4 Glaskugeln,
- 6 Gläser,
- 3 Injektionsspritzen mit z.B. jeweils 13,5 ml Lösungsmittel,
- 1-3 Behälter mit chemischen Reagenzien,
- 3 Pipetten,
- 1 Waage,
- 1 Schere,
- 1 Zange,
- 3 Plastiktüten,
- 1 Identifizierungsinformation, um Pflanzenarten zu identifizieren,
- 1 Anleitung, die beschreibt wie das Testkit anzuwenden ist, inklusive einer Standardfarbskala,
- 3 Filter.

8. Verwendung eines Verfahrens nach einem der Ansprüche 1 bis 4 zum Bestimmen, ob eine spezifische lebende Nutzpflanze oder lebende Unkrautpflanze einem spezifischen Pestizid ausgesetzt wurde.

9. Verfahren nach einem der Ansprüche 1 bis 4 oder Verwendung nach Anspruch 8, wobei das spezifische Pestizid ein Herbizid ist.

10. Verfahren oder Verwendung nach Anspruch 9, wobei das Herbizid ein Sulfonylureaherbizid ist,

11. Verfahren nach einem der Ansprüche 1 bis 4 oder Verwendung nach Anspruch 8, wobei die spezifische Pflanze aus der aus *Apera spica venti, Lolium perenne, Poa annua* und *Bromus hordeaceus* bestehenden Gruppe ausgewählt ist.

## Revendications

1. Procédé permettant de tester si un matériel provenant d'une plante vivante spécifique a été exposé à un pesticide spécifique, le procédé comprenant les étapes suivantes :
a) l'obtention d'un matériel provenant d'une plante spécifique,
b) la préparation d'une suspension liquide de la plante spécifique de a), dans laquelle la suspension liquide de la plante spécifique comprend au moins un composé phytochimique,
c) la détection des composés phytochimiques se trouvant dans la suspension liquide de b) par leur couleur observable sous lumière visible et/ou UV, dans laquelle la détection est réalisée sans séparation desdits composés phytochimiques,
d) la corrélation de ladite couleur des composés phytochimiques détectés avec une échelle normalisée de couleurs uniques observables sous lumière visible et/ou UV de composés phytochimiques non séparés pour des combinaisons de la plante spécifique et du pesticide spécifique,
e) le fait de déterminer si la plante spécifique a été exposée au pesticide spécifique.

2. Procédé selon la revendication 1, dans lequel, avant de déterminer si la plante spécifique a été exposée au pesticide spécifique,
(a) au moins un réactif chimique est choisi parmi un ou plusieurs des composés suivants :
la vanilline, l'acide sulfurique, le naphtorésorcinol, le bleu de méthylène, le β-naphtol, le thymol, la fluorescéine, l'ammoniac, le vert de bromocrésol, le bleu de bromophénol, le permanganate de potassium, la 2,7-dichlorofluorescéine, la rhodamine 6G, le diphénylborate de 2-aminoéthyle, l'acide phosphorique, l'iode, l'iodure de potassium, le molybdate d'ammonium, le chlorure d'étain (II), le chlorure de cobalt (II), le chlorure de palladium (II), la ninhydrine, le 1-naphtol, le nitrate de bismuth (III), l'iodure de potassium, le molybdate/ acide phosphorique, la rhodamine B, l'anisaldéhyde, le nitrate d'argent, le chlorure de fer (III), le chlorure de zinc, le rouge de chlorophénol, le rouge de méthyle, le rouge d'éthyle, le bleu de bromothymol, le sel de sodium du 2,6-dichlorophénolindophénol, le pourpre de bromocrésol, l'hydroxyde de potassium, le glucose, le 4-chloro-7-nitrobenzofurazane, la 2,4-dinitrophényl-hydrazine, le chloroformiate de 9-fluorénylméthyle, l'hydroxyde de tétrabutylammonium, l'iode, le sulfate d'ammonium et de fer (III), la 2-méthoxy-2,4-diphényl-3(2H)furanone (MDPF), le borinate de 2-aminoéthyldiphényle, le chlorure d'aluminium, le chlorure de berbérine déshydraté, le sel de sodium de la 1,2-naphtochinon-4-sulfone, l'anthrone, la 8-hydroxyquinoléine, la 2-aminodiphényl(biphényl-2-amine), l'orcinol, l'urée, l'acide 4-hydroxybenzoïque, l'acide 4-aminobenzoïque, l'acide molybdatophosphorique, la 2',7'-dichlorofluorescéine, le 8-anilinonaphtaline-1-sulfonate d'ammonium, la rhodamine, le nitrate de bismuth (III), l'iodure de potassium et leurs produits chimiques ou leurs mélanges, au moins un groupe desdits composés chimiques pouvant être présent ou non dans ledit matériel de la plante, et
(b) la provocation d'une réaction chimique entre lesdit réactif chimique et les composés phytochimiques non séparés du matériel de la plante,
(c) la détection du résultat de la réaction chimique de l'étape (b) sous une lumière visible et/ou UV, sans effectuer une séparation des composés phytochimiques,
(d) la corrélation des résultats de l'étape (c) avec un résultat d'échelle normalisée développée par rapport à la plante spécifique.

3. Procédé selon l'une quelconque des revendications précédentes, comprenant en outre :
i) la mise en contact de la suspension liquide du matériel de la plante avec un réactif chimique, pour ainsi provoquer une réaction chimique entre la suspension liquide du matériel de la plante et le réactif chimique, et
ii) la mise en contact d'un support solide avec le matériel de la plante ayant réagi par voie chimique avec les produits chimiques de i), pour ainsi obtenir un support solide présentant une couleur et une intensité de couleur.

4. Procédé de préparation d'une échelle normalisée permettant de déterminer si une plante vivante spécifique a été exposée à un pesticide spécifique, le procédé comprenant les étapes suivantes :
a) la soumission d'une plante vivante spécifique à un pesticide spécifique,
b) l'obtention d'un matériel provenant de la plante exposée de a),
c) la détermination de réponses chimiques de la plante spécifique par la détection d'une couleur observable sous lumière visible et/ou UV sans effectuer une séparation des composés phytochimiques de la plante,
d) l'obtention d'une échelle normalisée de couleurs uniques observables sous lumière visible et/ou UV de composés phytochimiques non séparés pour une combinaison de la plante spécifique et de pesticides spécifiques.

5. Kit d'essai destiné à être utilisé dans le procédé selon l'une quelconque des revendications 1 à 4, ledit kit comprenant :
- au moins un papier-filtre,
- au moins un solvant,
- au moins un moyen d'écrasement,
- au moins une échelle normalisée de visualisation de couleurs uniques observables sous lumière visible et/ou UV de composés phytochimiques non séparés permettant de détecter et/ou de prédire l'exposition d'une plante vivante spécifique à un pesticide spécifique,
- au moins un récipient.

6. Kit d'essai selon la revendication 5, comprenant en outre un ou plusieurs des composants choisis dans le groupe constitué par :
- au moins un réactif chimique, dans lequel ledit réactif chimique est choisi parmi un ou plusieurs des composés :
la vanilline, l'acide sulfurique, le naphtorésorcinol, le bleu de méthylène, le β-naphtol, le thymol, la fluorescéine, l'ammoniac, le vert de bromocrésol, le bleu de bromophénol, le permanganate de potassium, la 2,7-dichlorofluorescéine, la rhodamine 6G, le diphénylborate de 2-aminoéthyle, l'acide phosphorique, l'iode, l'iodure de potassium, le molybdate d'ammonium, le chlorure d'étain (II), le chlorure de cobalt (II), le chlorure de palladium (II), la ninhydrine, le 1-naphtol, le nitrate de bismuth (III), l'iodure de potassium, le molybdate/ acide phosphorique, la rhodamine B, l'anisaldéhyde, le nitrate d'argent, le chlorure de fer (III), le chlorure de zinc, le rouge de chlorophénol, le rouge de méthyle, le rouge d'éthyle, le bleu de bromothymol, le sel de sodium du 2,6-dichlorophénolindophénol, le pourpre de bromocrésol, l'hydroxyde de potassium, le glucose, le 4-chloro-7-nitrobenzofurazane, la 2,4-dinitrophénylhydrazine, le chloroformiate de 9-fluorénylméthyle, l'hydroxyde de tétrabutylammonium, l'iode, le sulfate d'ammonium et de fer (III), la 2-méthoxy-2,4-diphényl-3(2H)furanone (MDPF), le borinate de 2-aminoéthyldiphényle, le chlorure d'aluminium, le chlorure de berbérine déshydraté, le sel de sodium de la 1,2-naphtochinon-4-sulfone, l'anthrone, la 8-hydroxyquinoléine, la 2-aminodiphényl(biphényl-2-amine), l'orcinol, l'urée, l'acide 4-hydroxybenzoïque, l'acide 4-aminobenzoïque, l'acide molybdatophosphorique, la 2',7'-dichlorofluorescéine, le 8-anilinonaphtaline-1-sulfonate d'ammonium, la rhodamine, le nitrate de bismuth (III), l'iodure de potassium et leurs produits chimique ou leurs mélanges,
- au moins un mortier et pilon et/ou au moins une boîte à secouer contenant des billes et/ou au moins une presse à main,
- au moins une pipette,
- au moins une lampe UV,
- au moins un dispositif de chauffage et/ou au moins une coiffe chauffante faite de réactifs chimiques et de solvants,
- au moins une balance,
- au moins une paire de ciseaux,
- au moins une paire de pinces,
- au moins un sac en plastique,
- au moins une information d'identification servant à identifier une espèce végétale,
- au moins une instruction décrivant comment utiliser le kit d'essai,
- au moins une seringue,
- au moins un filtre.

7. Kit d'essai selon l'une quelconque des revendications 5 et 6, dans lequel ledit kit comprend :
- 3 morceaux de papier-filtre,
- 3 récipients avec des bouchons, contenant chacun 2, 3 ou 4 billes de verre,
- 6 verres,
- 3 seringues, contenant chacune par exemple 13,5 ml de solvant,
- 1 à 3 récipients contenant des réactifs chimiques,
- 3 pipettes,
- 1 balance,
- 1 paire de ciseaux,
- 1 paire de pinces,
- 3 sacs en plastique,
- 1 information d'identification servant à identifier une espèce végétale,
- 1 instruction décrivant comment utiliser le kit d'essai comprenant une échelle normalisée de couleurs, et
- 3 filtres.

8. Utilisation du procédé tel que défini dans l'une quelconque des revendications 1 à 4, pour déterminer si une plante vivante spécifique en culture ou une mauvaise herbe vivante spécifique a été soumise à un pesticide spécifique.

9. Procédé selon l'une quelconque des revendications 1 à 4 ou utilisation selon la revendication 8, dans lequel le pesticide spécifique est un herbicide.

10. Procédé ou utilisation selon la revendication 9, dans lequel l'herbicide est un herbicide de type sulfonylurée.

11. Procédé selon l'une quelconque des revendications 1 à 4 ou utilisation selon la revendication 8, dans lequel la plante spécifique est choisie dans le groupe constitué par *Apera spica venti, Lolium perenne, Poa annua* et *Bromus hordeaceus.*
